# FASCICULE DE BREVET EUROPEEN

(11) **EP 1 831 209 B1**
(45) Date de publication et mention de la délivrance du brevet: **12.08.2009**
(21) Numéro de dépôt: 05826562.0
(22) Date de dépôt: 16.12.2005
(51) Int. Cl.: C07D 417/14, C07D 417/12, C07D 413/12, A61K 31/428, A61P 35/00, A61P 37/00, A61P 29/00

(54) **INHIBITEURS DE PHOSPHATASES CDC25**
INHIBITOREN DER CDC25 PHOSPHATASE
CDC25 PHOSPHATASE INHIBITORS

(30) Priorité: 17.12.2004 FR 0413456
(43) Date de publication de la demande: 12.09.2007
(73) Titulaire: IPSEN PHARMA, 92100 Boulogne-Billancourt (FR)
(72) Inventeur: SIDHU, Alban, F-91070 Bondoufle (FR); PREVOST, Grégoire, 92160 ANTONY (FR); BIGG, Dennis, F-91190 GIF SUR YVETTE (FR); GALCERA CONTOUR, Marie-Odile, F-91070 BONDOUFLE (FR)
(74) Mandataire: Bourgouin, André
(86) Numéro de dépôt international: PCT/FR2005/003161
(87) Numéro de publication internationale: WO 2006/067311

(56) Documents cités:
- WO-A-03/055868
- ECKSTEIN, J.W.: "Cdc25 as a potential target of anticancer agents" INVESTIGATIONAL NEW DRUGS, vol. 18, 2000, pages 149-156, XP002340121
- KRISTJANSDOTTIR, K. ET AL.: "Cdc25 Phospatases and cancer" CHEMISTRY & BIOLOGY, vol. 11, août 2004 (2004-08), pages 1043-1051, XP002340122
- MCCAINT, D.F. ET AL.: "Suramin Derivatives as Inhibitors and Activators of Protein-tyrosine Phospatases" JOURNAL OF BIOLOGICAL CHEMISTRY, vol. 279, no. 15, janvier 2004 (2004-01), pages 14713-14725, XP002340123

## Description

La présente invention a pour objet de nouveaux inhibiteurs de phosphatases cdc25.

Le contrôle de la transition entre les différentes phases du cycle cellulaire durant la mitose ou de la méiose est assurée par un ensemble de protéines dont les activités enzymatiques sont associées à des états différents de phosphorylation. Ces états sont contrôlés par deux grandes classes d'enzymes : les kinases et les phosphatases.

La synchronisation des différentes phases du cycle cellulaire permet ainsi la réorganisation de l'architecture cellulaire à chaque cycle dans l'ensemble du monde vivant (microorganismes, levure, vertébrés, plantes). Parmi les kinases, les kinases dépendantes des cyclines (CDKs) jouent un rôle majeur dans ce contrôle du cycle cellulaire. L'activité enzymatique de ces différentes CDKs est contrôlée par deux autres familles d'enzymes qui travaillent en opposition (Jessus et Ozon, Prog. Cell Cycle Res. (1995), 1, 215-228). La première regroupe des kinases telles que Weel et Mik1 qui désactivent les CDKs en phosphorylant certains acides aminés (Den Haese et coll., Mol. Biol. Cell (1995), 6, 371-385). La seconde regroupe des phosphatases telle que Cdc25 qui activent les CDKs en déphosphorylant des résidus tyrosine et thréonine de CDKs (Gould et coll., Science (1990), 250, 1573-1576).

Les phosphatases sont classifiées en 3 groupes : les sérines/thréonines phosphatases (PPases), les tyrosines phosphatases (PTPases) et les phosphatases à double spécificité (DSPases). Ces phosphatases jouent un rôle important dans la régulation de nombreuses fonctions cellulaires.

En ce qui concerne les phosphatases cdc25 humaines, 3 gènes (cdc25-A, cdc25-B et cdc25-C) codent pour les protéines cdc25. De plus, des variants issus de splicing alternatif du gène cdc25B ont été identifiés : il s'agit de cdc25B1, cdc25B2 et cdc25B3 (Baldin et coll., Oncogene (1997), 14 , 2485-2495).

Le rôle des phosphatases Cdc25 dans l'oncogénèse est maintenant mieux connu et les mécanismes d'action de ces phosphatases sont illustrés en particulier dans les références suivantes : Galaktionov et coll., Science (1995), 269, 1575-1577 ; Galaktionov et coll., Nature (1996), 382, 511-517 ; et Mailand et coll., Science (2000), 288, 1425-1429.

La surexpression des différentes formes de cdc25 est maintenant rapportée dans de nombreuses séries de tumeurs humaines, par exemple :
- Cancer du sein : cf. Cangi et coll., Résumé 2984, AACR meeting San Francisco, 2000) ;
- Lymphomes : cf. Hernandez et coll., Int. J Cancer (2000), 89, 148-152 et Hernandez et coll., Cancer Res. (1998), 58, 1762-1767 ;
- Cancers du cou et de la tête : cf. Gasparotto et coll., Cancer Res. (1997), 57, 2366-2368 ;
- Cancers du pancréas: cf. Junchao Guo et coll., Oncogene (2004), 23, 71-81.

Par ailleurs, le groupe de E. Sausville rapporte une corrélation inverse entre le niveau d'expression de cdc25-B dans un panel de 60 lignées et leurs sensibilités aux inhibiteurs de CDK, suggérant que la présence de cdc25 puisse apporter une résistance à certains agents antitumoraux et plus particulièrement aux inhibiteurs de CDK (Hose et coll., Proceedings of AACR, Abstract 3571, San Francisco, 2000).

Parmi d'autres cibles, on recherche donc à présent des composés capables d'inhiber les phosphatases Cdc25 afin de les utiliser notamment comme agents anti-cancéreux.

Les phosphatases Cdc25 jouent également un rôle dans les maladies neurodégénératives telles que la maladie d'Alzheimer (cf. Zhou et. coll., Cell Mol. Life Sci. (1999), 56(9-10), 788-806 ; Ding et coll., Am. J. Pathol. (2000), 157(6), 1983-90 ; Vincent et coll., Neuroscience (2001), 105(3), 639-50) de sorte que l'on peut aussi envisager d'utiliser des composés possédant une activité d'inhibition de ces phosphatases pour traiter ces maladies.

Un autre problème auquel s'adresse l'invention est la recherche de médicaments destinés à prévenir ou traiter le rejet de greffes d'organes ou encore à traiter des maladies auto-immunes. Dans ces désordres / maladies, l'activation non appropriée des lymphocytes et des monocytes/macrophages est impliquée. Or les médicaments immunosuppresseurs connus à ce jour ont des effets secondaires qui pourraient être diminués ou modifiés par des produits ciblant spécifiquement les voies de signalisation dans les cellules hématopoïétiques qui initient et maintiennent l'inflammation.

L'invention a tout d'abord pour objet de nouveaux inhibiteurs de phosphatases cdc25 (en particulier de la phosphatase cdc25-C), lesquels sont des dérivés de type dimère de benzothiazole-4,7-diones et de benzoxazole-4,7-diones et répondent à la formule générale **(I)** définie ci-après. Compte tenu de ce qui précède, ces composés sont susceptibles d'être utilisés comme médicaments, en particulier dans le traitement et / ou la prévention des maladies ou désordres suivants :
- l'inhibition de la prolifération tumorale seule ou en combinaison avec d'autres traitements ;
- l'inhibition de la prolifération des cellules normales seule ou en combinaison avec d'autres traitements ;
- les maladies neurodégénératives comme la maladie d'Alzheimer ;
- la prévention de l'alopécie spontanée ;
- la prévention de l'alopécie induite par des produits exogènes
- la prévention de l'alopécie radio-induite ;
- la prévention de l'apoptose spontanée ou induite des cellules normales ;
- la prévention de la méiose et / ou la fécondation ;
- la prévention de la maturation des oocytes ;
- toutes les maladies / tous les désordres correspondant à des utilisations rapportées pour les inhibiteurs de CDKs, et notamment les maladies prolifératives non tumorales (par exemple : angiogénèse, psoriasis ou resténose), maladies prolifératives tumorales, parasitologie (prolifération de protozoaires), infections virales, maladies neurodégénératives, myopathies ; et / ou
- toutes les maladies / tous les désordres correspondant à des applications cliniques de la vitamine K et de ses dérivés.

Par ailleurs, les composés de la présente invention sont également, du fait de leurs propriétés d'inhibition des phosphatases cdc25, susceptibles d'être utilisés pour inhiber ou prévenir la prolifération des microorganismes, notamment des levures. L'un des avantages de ces composés consiste en leur faible toxicité sur les cellules saines.

A présent, la Demanderesse a découvert de façon surprenante que les composés répondant à la formule générale **(I)** 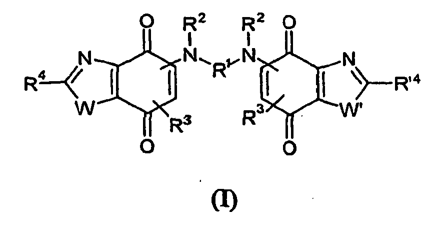 sous forme racémique, d'énantiomère ou toute combinaison de ces formes, dans laquelle :
chacun de W et W' représente indépendamment O ou S ;
R¹ représente l'un des radicaux -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ- et -(CH₂)ₛ-CO-NR⁸-(CH₂)ₜ- dans lesquels m et n sont chacun indépendamment un entier de 2 à 6 (de préférence un entier de 2 à 4 et plus préférentiellement un entier de 2 à 3), p et t sont chacun indépendamment un entier de 2 à 12 (de préférence un entier de 2 à 8 et plus préférentiellement un entier de 2 à 6), q est un entier de 2 à 4 (de préférence un entier de 2 à 3), r est un entier de 0 à 4 (de préférence un entier de 0 à 2), s est un entier de 1 à 12 (de préférence un entier de 1 à 8 et plus préférentiellement un entier de 1 à 6), X est choisi parmi les radicaux NR⁵-, -S, -CO-, -CR⁶R⁷-, cycloalkyle et aryle, étant entendu que lorsque X représente -S-, -CO-, - CR⁶R⁷-, cycloalkyle ou aryle, m et n sont égaux,
R⁵ représentant un atome d'hydrogène ou un radical alkyle ou arylalkyl éventuellement substitué de 1 à 3 fois par des substituants choisis indépendamment parmi un atome halogène et un radical alkyle ou alkoxy, R⁶ et R⁷ représentant chacun indépendamment un atome d'hydrogène ou un radical alkyle et R⁸ représentant un atome d'hydrogène ou un radical alkyle ou arylalkyl ,
ou R¹ représente 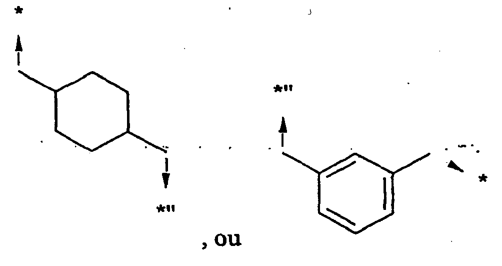 étant entendu que 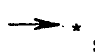 signifie le point d'attachement à la formule générale (I) ;
   ou encore R¹ représente le radical -(CH₂)_{w}-N(Y)-(CH₂)_{w}- dans lequel Y est le radical 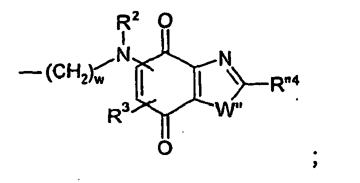
W" représente O ou S et w est un entier de 2 à 3 ;
R² représente un atome d'hydrogène ou un radical alkyle ou arylalkyl ;
R³ représente un atome d'hydrogène ou un atome halogène ;
chacun de R⁴, R'⁴ et R"⁴ représente indépendamment un atome d'hydrogène, un radical alkyle, un radical alkoxyalkyle, un radical aryloxyalkyle, un radical phényle possédant deux substituants qui forment ensemble un radical méthylènedioxy ou éthylène dioxy, un radical -CH₂-NR⁹R¹⁰, un radical -CO-NR¹⁴R¹⁵, ou encore un radical aryle carbocyclique ou arylalkyl carbocyclique éventuellement substitué de 1 à 4 fois par des substituants choisis indépendamment parmi un atome halogène et un radical alkyle, haloalkyle, alkoxy, haloalkoxy ou aryle,
ou R⁴ représente le radical 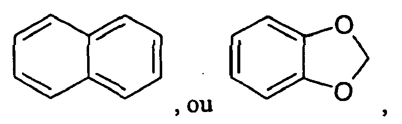
R⁹ représentant indépendamment à chaque fois qu'il intervient un radical alkyle et R¹⁰ représentant indépendamment à chaque fois qu'il intervient un atome d'hydrogène ou un radical alkyle, ou encore R⁹ et R¹⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹¹R¹²-, -O-, -S-, et -NR¹³-, R¹¹ et R¹² représentant indépendamment à chaque fois qu'ils interviennent un atome d'hydrogène ou un radical allyle et R¹³ représentant un radical alkyle ou arylalkyl , ou encore R¹³ représentant un radical phényle éventuellement substitué de 1 à 3 fois par des substituants choisis indépendamment parmi un atome halogène et un radical alkyle ou alkoxy,
R¹⁴ représentant indépendamment à chaque fois qu'il intervient un radical alkyle, un radical haloalkyle, un radical cycloalkyle, un radical cycloalkylalkyle, un radical alkoxyalkyle, l'un des radicaux aryle carbocyclique ou hétérocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂NH₂,
ou R¹⁴ représente un radical 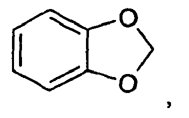 ou encore R¹⁴ représentant l'un des radicaux -(CH₂)ₐ-[O-(CH₂)_{b}]_{c}-O-Alk, -(CH₂)_{d}-[O-(CH₂)ₑ]_{f}-NR¹⁶R¹⁷ ou -(CH₂)_{g}-A dans lesquels a, b et e sont chacun indépendamment un entier de 2 à 4, c est un entier de 1 à 4, f est un entier de 0 à 4, d est un entier de 2 à 12 (et de préférence un entier de 2 à 8 et notamment un entier de 2 à 6) et g est un entier de 1 à 12 (et de préférence un entier de 1 à 8 et notamment un entier de 1 à 6), Alk est un radical alkyle, R¹⁶ est un atome d'hydrogène ou un radical alkyle, alkoxycarbonyle ou aralkoxycarbonyle, R¹⁷ est un atome d'hydrogène ou un radical alkyle et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical alkyle et R¹⁹ représentant un atome d'hydrogène, un radical alkyle ou un groupement alkoxycarbonyle ou aralkoxycarbonyle,
et R¹⁵ représentant indépendamment à chaque fois qu'il intervient un atome d'hydrogène ou un radical alkyle ou arylalkyl , R¹⁵ pouvant également représenter un radical identique à R¹⁴ lorsque R¹⁴ représente un radical alkyle, haloalkyle, alkoxyalkyle
   ou arylalkyl carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂
   ou encore R¹⁴ et R¹⁵ formant ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et -NR²²-, R²⁰ et R²¹ représentant un atome d'hydrogène
   ou un radical alkyle ou arylalkyl et R²² représentant -COR²³ ou -SO₂R²⁴,
R²³ représentant un radical alkyl, un radical aryle carbocyclique éventuellement: substitué de 1 à 3 fois par des substituants choisis indépendamment parmi le groupe constitué par un atome halogène, un radical alkyle et un radical alkoxy, ou encore R²³ représentant un radical aryle hétérocyclique ou un hétérocycle saturé comptant de 5 à 7 chaînons et de 1 à 2 hétéroatomes choisis indépendamment parmi O, N et S (et notamment l'un des radicaux pipéridino, pipérazino, morpholino, thiomorpholino ou 2-tétrahydrofuryle),
R²⁴ représentant un atome d'hydrogène ou un radical alkyle (et de préférence un radical alkyle),
ou enfin R¹⁴ et R¹⁵ formant ensemble avec l'atome d'azote qui les porte un radical aryle hétérocyclique choisi parmi les radicaux 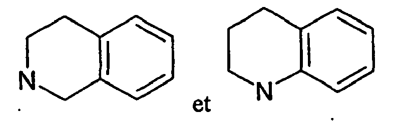 dont le noyau aromatique peut être substitué de 1 à 3 fois par des substituants choisis indépendamment parmi le groupe constitué par un radical alkyle et un radical alkoxy ;
étant entendu que dans le cas où R¹ représente le radical -(CH₂)_{w}-N(Y)-(CH₂)_{w}-, W, W' et W" sont identiques (autrement dit représentent soit tous O soit tous S), R⁴, R'⁴ et R"⁴ sont identiques et les atomes d'azote adjacents aux noyaux 1,3-benzothiazole-4,7-dione ou 1,3-benzoxazole-4,7-dione sont soit chacun attaché à la position 5 du noyau 1,3-benzothiazole-4,7-dione ou 1,3-benzoxazole-4,7-dione correspondant soit chacun attaché à la position 6 du noyau 1,3-benzothiazole-4,7-dione ou 1,3-benzoxazole-4,7-dione correspondant ;
et leurs sels sont de puissants inhibiteurs des phosphatases Cdc25 (et notamment de la phosphatase Cdc25C), ce qui les rend aptes à une utilisation en tant qu'agents anti-cancéreux.

L'invention concerne donc en premier lieu les composés de formule générale **(I)** définie précédemment et les sels de tels composés.

Par cycloalkyle, lorsqu'il n'est pas donné plus de précision, on entend un radical cycloalkyle comptant de 3 à 7 atomes de carbone.

Par aryle carbocyclique ou hétérocyclique, on entend un système carbocyclique ou hétérocyclique de 1 à 3 cycles condensés comprenant au moins un cycle aromatique, un système étant dit hétérocyclique lorsque l'un au moins des cycles qui le composent comporte au moins un hétéroatome (O, N ou S) ; lorsqu'un radical aryle carbocyclique ou hétérocyclique est dit substitué sans qu'il soit donné plus de précision, on entend que ledit radical aryle carbocyclique ou hétérocyclique est substitué de 1 à 3 fois, et de préférence de 1 à 2 fois par des radicaux différents d'un atome d'hydrogène qui, s'ils ne sont pas précisés, sont choisis parmi un atome halogène et les radicaux alkyle ou alkoxy; par ailleurs, lorsqu'il n'est pas donne plus de précision, on entendu par aryle un aryle carbocyclique exclusivement.

Par arylalkyl, on entend au sens de la présente invention un radical -alkyl-aryl.

Par alkyle, lorsqu'il n'est pas donné plus de précision, on entend un radical alkyle linéaire ou ramifié comptant de 1 à 12 atomes de carbone, de préférence de 1 à 10 atomes de carbone et plus préférentiellement 1 à 8 atomes de carbone (et notamment de 1 à 6 atomes de carbone).

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par haloalkyl, on entend notamment le radical trifluorométhyle. Par haloalkoxy, on entend notamment le radical trifluorométhoxy. Par aryle carbocyclique, on entend en particulier les radicaux phényle et naphtyle. Par arylalkyle, on entend en particulier les radicaux phénylalkyle, et notamment le radical benzyle. Par système cyclique carboné saturé comptant de 1 à 3 cycles condensés choisis indépendamment parmi des cycles de 3 à 7 chaînons, on entend en particulier les radicaux cyclopropyle, cyclobutyle, cyclohexyle et adamantyle. Par aryle hétérocyclique ou hétéroaryle, on entend en particulier les radicaux thiényle, furannyle, pyrrolyle, imidazolyle, thiazolyle, oxazolyle et pyridyle. Enfin, par halogène, on entend les atomes de fluor, de chlore, de brome ou d'iode.

Par halogène ou atome halogène on entend un atome de chlore, de brome, de fluor ou d'iode.

Par alkoxy, lorsqu'il n'est pas donné plus de précision, on entend un radical alkoxy, linéaire ou ramifié comptant de 1 à 6 atomes de carbone (et notamment de 1 à 4 atomes de carbone).

Par haloalkyl, on entend un radical alkyle dont au moins l'un des atomes d'hydrogène (et éventuellement tous) est remplacé par un atome halogène.

Par radicaux cycloalkylalkyle, alkoxyalkyle, haloalkyle et haloalkoxy, on entend respectivement les radicaux cycloalkylalkyle, alkoxyalkyle, haloalkyle, haloalkoxy et arylalkyl dont les radicaux alkyle, cycloalkyle et aryle ont les significations indiquées précédemment.

Lorsqu'il est indiqué qu'un radical est éventuellement substitué de 1 à 4 fois, il est de préférence éventuellement substitué de 1 à 3 fois, plus préférentiellement éventuellement substitué de 1 à 2 fois et encore plus préférentiellement éventuellement substitué une fois.

Par sel d'un composé, on entend les sels d'addition dudit composé avec un acide organique ou inorganique ou, le cas échéant, avec une base, et notamment les sels pharmaceutiquement acceptables dudit composé.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33**, 201-217.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Selon une variante générale de l'invention, les composés de formule générale **(I)** ou leurs sels seront tels que R¹ ne représente pas un radical -(CH₂)_{w}-N(Y)-(CH₂)_{w}-, W et W' soient identiques et R⁴ et R'⁴ soient identiques ; pour la suite de cet exposé, les composés de formule générale **(I)** tels que R¹ ne représente pas un radical -(CH₂)_{w}-N(Y)-(CH₂)_{w}-, W et W' soient identiques et R⁴ et R'⁴ soient identiques seront appelés « composés de formule générale **(I)_{D}** ».

Un aspect particulier de cette variante générale de l'invention concerne les composés de formule générale **(I)_{D}** dans laquelle chacun de W et W' représente S, lesquels seront désignés dans la suite de cet exposé « composés de formule générale **(I)_{DS}** », ainsi que les sels de ces composés. L'invention concerne en particulier les composés de formule générale **(I)_{D5}** dans laquelle la chaîne de liaison -N(R²)-R¹-N(R²)- sera positionnée telle qu'elle connecte les positions 5 des motifs 1,3-benzothiazole-4,7-dione, à savoir les composés de sous-formule générale **(I)_{DS5}** 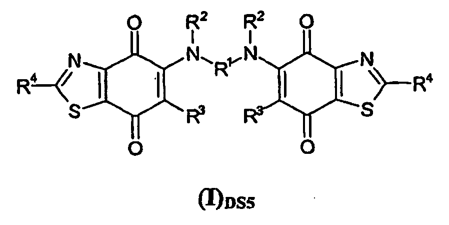 dans laquelle R¹ a la même signification que dans la formule générale **(I)_{D}** et R², R³ et R⁴ ont la même signification que dans la formule générale **(I),** ainsi que les sels de ces composés. Elle concerne également les composés de formule générale **(I)_{DS}** dans laquelle la chaîne de liaison -N(R²)-R¹-N(R²)- sera positionnée telle qu'elle connecte les positions 6 des motifs 1,3-benzothiazole-4,7-dione, à savoir les composés de sous-formule générale **(I)D_{S6}** 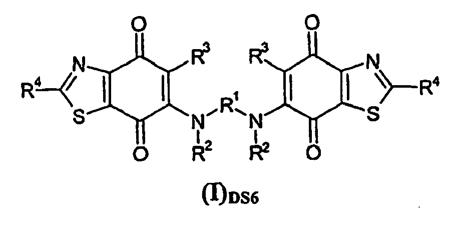 dans laquelle R¹ a la même signification que dans la formule générale **(I)_{D}** et R², R³ et R⁴ ont la même signification que dans la formule générale **(I),** ainsi que les sels de ces composés.

Un autre aspect particulier de cette variante générale de l'invention concerne les composés de formule générale **(I)_{D}** dans laquelle chacun de W et W' représente O, lesquels seront désignés dans la suite de cet exposé « composés de formule générale **(I)_{DO}** », ainsi que les sels de ces composés. L'invention concerne en particulier les composés de formule générale **(I)_{DO}** dans laquelle la chaîne de liaison -N(R²)-R¹-N(R²)-sera positionnée telle qu'elle connecte les positions 5 des motifs 1,3-benzoxazole-4,7-dione, à savoir les composés de sous-formule générale **(I)_{DO5}** 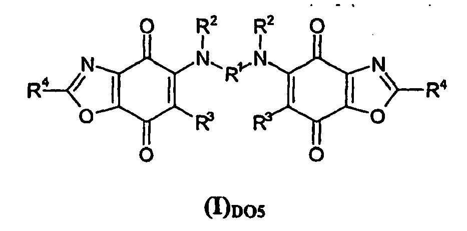 dans laquelle R¹ a la même signification que dans la formule générale **(I)_{D}** et R², R³ et R⁴ ont la même signification que dans la formule générale **(I),** ainsi que les sels de ces composés. Elle concerne également les composés de formule générale **(I)_{DO}** dans laquelle la chaîne de liaison -N(R²)-R¹-N(R²)- sera positionnée telle qu'elle connecte les positions 6 des motifs 1,3-benzoxazole-4,7-dione, à savoir les composés de sous-formule générale **(I)_{DO6}** 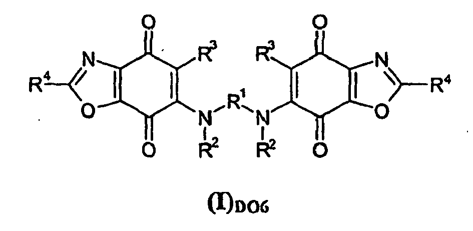 dans laquelle R¹ a la même signification que dans la formule générale **(I)_{D}** et R², R³ et R⁴ ont la même signification que dans la formule générale **(I),** ainsi que les sels de ces composés.

Selon une autre variante générale de l'invention, les composés de formule générale **(I)**
ou leurs sels seront tels que **R¹** représente un radical -(CH₂)_{w}-N(Y)-(CH₂)_{w}- ; pour la suite de cet exposé, les composés de formule générale **(I)** tels que R¹ représente un radical -(CH₂)_{w}-N(Y)-(CH₂)_{w}- seront appelés « composés de formule générale **(I)_{T}** ».

Un aspect particulier de cette variante générale de l'invention concerne les composés de formule générale **(I)_{T}** dans laquelle chacun de W, W' et W" représente S, lesquels seront désignés dans la suite de cet exposé « composés de formule générale **(I)_{TS}** », ainsi que les sels de ces composés. L'invention concerne en particulier les composés de formule générale **(I)_{TS}** dans laquelle la chaîne de liaison -N(R²)-R¹-N(R²)- sera positionnée telle qu'elle connecte les positions 5 des motifs 1,3-benzothiazole-4,7-dione, à savoir les composés de sous-formule générale **(I)_{TS5}** 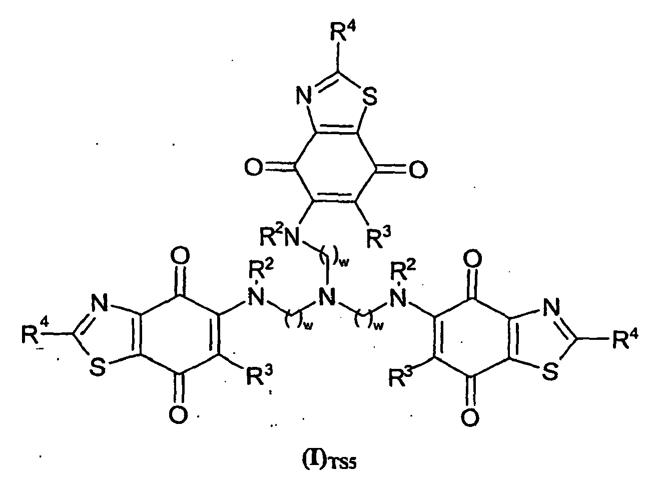 dans laquelle w, R², R³ et R⁴ ont la même signification que dans la formule générale **(I),** ainsi que les sels de ces composés. Elle concerne également les composés de formule générale **(I)_{TS}** dans laquelle la chaîne de liaison -N(R²)-R¹-N(R²)- sera positionnée telle qu'elle connecte les positions 6 des motifs 1,3-benzothiazole-4,7-dione, à savoir les composés de sous-formule générale **(I)_{TS6}** 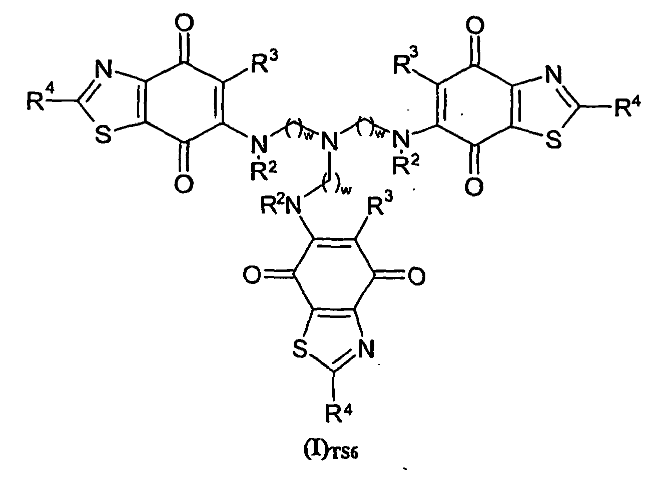 dans laquelle R¹ a la même signification que dans la formule générale **(I)_{T}** et R², R³ et R⁴ ont la même signification que dans la formule générale **(I),** ainsi que les sels de ces composés.

Un autre aspect particulier de cette variante générale de l'invention concerne les composés de formule générale **(I)_{T}** dans laquelle chacun de W, W' et W" représente O, lesquels seront désignés dans la suite de cet exposé « composés de formule générale **(I)_{TO}** », ainsi que les sels de ces composés. L'invention concerne en particulier les composés de formule générale **(I)_{TO}** dans laquelle la chaîne de liaison -N(R²)-R¹-N(R²)- sera positionnée telle qu'elle connecte les positions 5 des motifs 1,3-benzoxazole-4,7-dione, à savoir les composés de sous-formule générale **(I)_{TO5}** 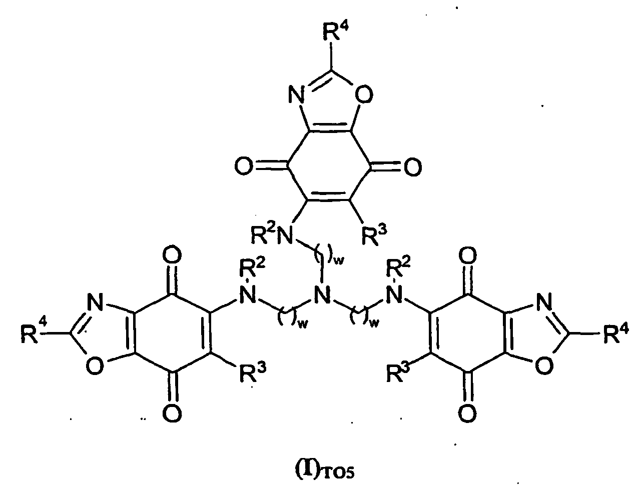 dans laquelle w, R², R³ et R⁴ ont la même signification que dans la formule générale **(I),** ainsi que les sels de ces composés. Elle concerne également les composés de formule générale **(I)_{DO}** dans laquelle la chaîne de liaison -N(R²)-R¹-N(R²)- sera positionnée telle qu'elle connecte les positions 6 des motifs 1,3-benzoxazole-4,7-dione, à savoir les composés de sous-formule générale **(I)_{TO6}** 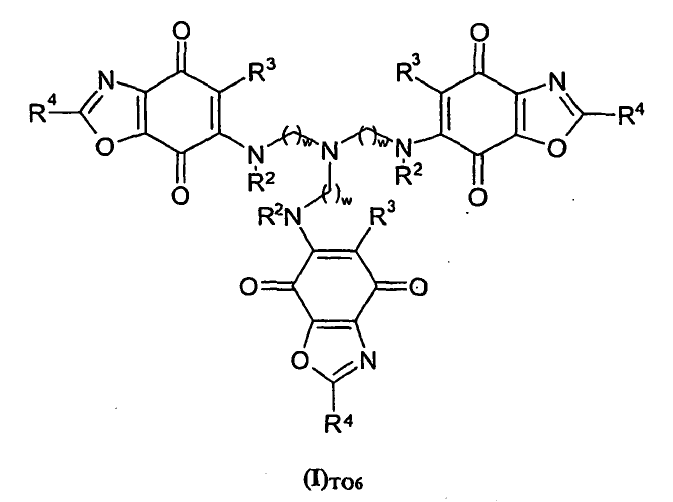 dans laquelle w, R², R³ et R⁴ ont la même signification que dans la formule générale **(I)**, ainsi que les sels de ces composés.

Selon encore une autre variante générale de l'invention, les composés de formule générale **(I)** ou leurs sels seront tels que R¹ ne représente pas un radical -(CH₂)_{w}-N(Y)-(CH₂)_{w}- et l'un de W et W' représente O tandis que l'autre représente S et/ou R⁴ et R'⁴ sont différents ; pour la suite de cet exposé, les composés de formule générale **(I)** tels que R¹ ne représente pas un radical -(CH₂)_{w}-N(Y)-(CH₂)_{w}- et l'un de W et W' représente O tandis que l'autre représente S et/ou R⁴ et R'⁴ sont différents seront appelés « composés de formule générale **(I)_{DM}** »·

De préférence, les composés de formule générale **(I)** ou leurs sels seront tels qu'ils possèdent au moins l'une des caractéristiques suivantes :
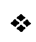 R¹ représentant l'un des radicaux -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ-, -(CH₂)ₛ-CO-NR⁸-(CH₂)ₜ- ou bien R¹ représentant le radical -(CH₂)_{w}-N(Y)-(CH₂)_{w}- ;
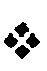 R² représentant un atome d'hydrogène ou le radical méthyle, éthyle ou benzyle ;
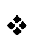 R³ représentant un atome d'hydrogène ou un atome halogène ;
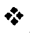 chacun de R⁴, R'⁴ et R"⁴ représentant indépendamment un atome d'hydrogène, un radical alkyle, -CO-NR¹⁴R¹⁵ ou encore un radical aryle carbocyclique ou arylalkyl carbocyclique éventuellement substitué de 1 à 4 fois par des substituants choisis indépendamment parmi un atome halogène et un radical alkyle, haloalkyle, alkoxy, haloalkoxy ou aryle.

Plus préférentiellement, les composés de formule générale **(I)** ou leurs sels seront tels qu'ils possèdent au moins l'une des caractéristiques suivantes :
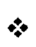 R¹ représentant l'un des radicaux -(CH₂)ₘ-X-(CH₂)ₙ-, ou bien R¹ représentant le radical -(CH₂)_{w}-N(Y)-(CH₂)_{w}- (w représentant de préférence 2) ;
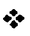 R² représentant un atome d'hydrogène ou le radical méthyle ;
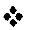 chacun de R⁴, R'⁴ et R"⁴ représentant indépendamment un atome d'hydrogène, un radical alkyle, -CO-NR¹⁴R¹⁵ ou encore un radical aryle carbocyclique ou arylalkyl carbocyclique éventuellement substitué de 1 à 4 fois par des substituants choisis indépendamment parmi un atome halogène (les atomes de fluor étant préférés parmi les substituants halogènes) et un radical alkyle, trifluorométhyle, alkoxy, trifluorométhoxy ou phényle.

Encore plus préférentiellement, les composés de formule générale **(I)** ou leurs sels seront tels qu'ils possèdent au moins l'une des caractéristiques suivantes :
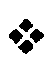 R¹ représentant -(CH₂)ₘ-X-(CH₂)ₙ- ;
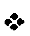 R² représentant un atome d'hydrogène.

Encore plus préférentiellement, les composés de formule générale **(I)** ou leurs sels seront tels que :
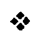 R¹ représentant l'un des radicaux -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ-, -(CH₂)ₛ-CO-NR⁸-(CH₂)ₜ- ou bien R¹ représentant le radical -(CH₂)_{w}-N(Y)-(CH₂)_{w}- ;

Encore plus préférentiellement, les composés de formule générale **(I)** ou leurs sels seront tels que :
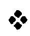 R¹ représentant -(CH₂)ₘ-X-(CH₂)ₙ-

Par ailleurs, on préférera d'une façon générale les composés de formule générale **(I)** (ou leurs sels) tels qu'ils possèdent au moins l'une des caractéristiques suivantes :
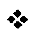 X représente NR⁵- ou -CR⁶R⁷ - ;
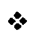 R⁸ représente un atome d'hydrogène ou un radical méthyle ;
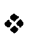 R¹⁴ représente indépendamment à chaque fois qu'il intervient un radical alkyle, un radical cycloalkyle, un radical cycloalkylalkyle, un radical alkoxyalkyle, l'un des radicaux aryle carbocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ représente l'un des radicaux -(CH₂)ₐ-[O-(CH₂)_{b}]_{c}-O-Alk, -(CH₂)_{d}-[O-(CH₂)ₑ]_{f}-NR¹⁶R¹⁷ ou -(CH₂)_{g}-A dans lesquels a, b et e sont chacun indépendamment 2 ou 3, c est un entier de 1 à 3, f est un entier de 0 à 3, d est un entier de 2 à 6 et g est un entier de 1 à 6, Alk est un radical alkyle, R¹⁶ est un atome d'hydrogène ou un radical alkoxycarbonyle, R¹⁷ est un atome d'hydrogène ou un radical méthyle et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical méthyle et R¹⁹ représentant un atome d'hydrogène, un radical alkyle ou un groupement alkoxycarbonyle, ou encore R¹⁴ représente un radical 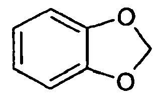 et R¹⁵ représente indépendamment à chaque fois qu'il intervient un atome d'hydrogène ou un radical alkyle ou arylalkyl , R¹⁵ pouvant également représenter un radical identique à R¹⁴ lorsque R¹⁴ représente un radical alkyle, alkoxyalkyle ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ et R¹⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et -NR²²-, R²⁰ et R²¹ représentant un atome d'hydrogène ou un radical alkyle ou arylalkyl et R²² représentant -COR²³ ou -SO₂R²⁴,
R²³ représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R²³ représentant un radical aryle hétérocyclique ou un hétérocycle saturé choisi parmi les radicaux pipéridino, pipérazino, morpholino, thiomorpholino et 2-tétrahydrofuryle,
R²⁴ représentant un atome d'hydrogène ou un radical alkyle;
ou encore R¹⁴ et R¹⁵ formant ensemble avec l'atome d'azote qui les porte un radical aryle hétérocyclique du type 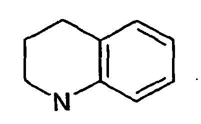 dont le noyau aromatique peut être substitué de 1 à 3 fois par des substituants choisis indépendamment parmi le groupe constitué par un radical alkyle et un radical alkoxy.

D'une façon générale, on préférera encore plus les composés de formule générale **(I)** (ou leurs sels) tels qu'ils possèdent au moins l'une des caractéristiques suivantes :
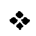 X représente -NR⁵- dans lequel R⁵ représente un radical méthyle ou arylalkyl événtuellement substitué par un groupement alkoxy (et en particulier méthoxy);
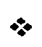 R⁸ représente un atome d'hydrogène ;
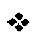 R¹⁴ représente indépendamment à chaque fois qu'il intervient un radical alkyle, un radical cycloalkyle, un radical cycloalkylalkyle, un radical alkoxyalkyle, l'un des radicaux aryle carbocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ représente l'un des radicaux -(CH₂)_{d}-(O-(CH₂)ₑ]_{f}-NR¹⁶R¹⁷ ou -(CH₂)_{g}-A dans lesquels e est 2 ou 3, f est un entier de 0 à 3, d est un entier de 2 à 6 et g est un entier de 1 à 6, R¹⁶ est un atome d'hydrogène ou un radical alkoxycarbonyle (et notamment *tert*-butoxycarbonyle), R¹⁷ est un atome d'hydrogène et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical méthyle et R¹⁹ représentant un atome d'hydrogène, un radical alkyle ou un groupement alkoxycarbonyle (et notamment *tert*-butoxycarbonyle), ou encore R¹⁴ représente un radical 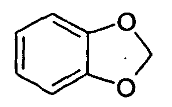 et R¹⁵ représente indépendamment à chaque fois qu'il intervient un atome d'hydrogène, R¹⁵ pouvant également représenter un radical identique à R¹⁴ lorsque R¹⁴ représente un radical alkyle, alkoxyalkyle ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ et R¹⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et -NR²²-, R²⁰ et R²¹ représentant un atome d'hydrogène ou un radical alkyle et R²² représentant -COR²³ ou -SO₂R²⁴,
R²³ représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore
R²³ représentant un radical aryle hétérocyclique ou un hétérocycle saturé choisi parmi les radicaux pipéridino, pipérazino, morpholino, thiomorpholino et 2-tétrahydrofuryle,
R²⁴ représentant un atome d'hydrogène ou un radical alkyle ;
ou encore R¹⁴ et R¹⁵ formant ensemble avec l'atome d'azote qui les porte un radical aryle hétérocyclique du type 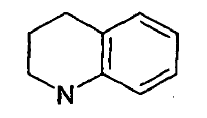 dont le noyau aromatique peut être substitué de 1 à 3 fois par des substituants choisis indépendamment parmi le groupe constitué par un radical alkyle et un radical alkoxy, en particulier methoxy.

D'une façon générale, on préférera encore plus les composés de formule générale **(I)** (ou leurs sels) tels qu'ils possèdent au moins l'une des caractéristiques suivantes :
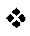 X représente NR⁵- dans lequel R⁵ représente un radical méthyle ;
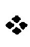 R¹⁴ représente indépendamment à chaque fois qu'il intervient un radical alkyle, un radical cycloalkyle, un radical cycloalkylalkyle, un radical alkoxyalkyle, l'un des radicaux aryle carbocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ représente l'un des radicaux -(CH₂)_{d}-[O-(CH₂)ₑ]_{f}-NR¹⁶R¹⁷ ou -(CH₂)_{g}-A dans lesquels e est 2 ou 3, f est un entier de 0 à 3, d est un entier de 2 à 6 et g est un entier de 1 à 6, R¹⁶ est un atome d'hydrogène ou un radical alkoxycarbonyle (et notamment *tert*-butoxycarbonyle), R¹⁷ est un atome d'hydrogène et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical méthyle et R¹⁹ représentant un atome d'hydrogène, un radical alkyle ou un groupement alkoxycarbonyle (et notamment *tert*-butoxycarbonyle),
ou encore R¹⁴ représente un radical 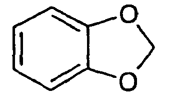 et R¹⁵ représente indépendamment à chaque fois qu'il intervient un atome d'hydrogène,
ou encore R¹⁴ et R¹⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et NR²²-, R²⁰ et R²¹ représentant un atome d'hydrogène ou un radical méthyle et R²² représentant -COR²³ ou -SO₂R²⁴,
R²³ représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore
R²³ représentant un radical aryle hétérocyclique ou un hétérocycle saturé choisi parmi les radicaux pipéridino, pipérazino, morpholino et 2-tétrahydrofuryle,
R²⁴ représentant un atome d'hydrogène ou un radical alkyle (et en particulier un radical alkyle, notamment méthyle) ;
ou encore R¹⁴ et R¹⁵ formant ensemble avec l'atome d'azote qui les porte un radical aryle hétérocyclique du type 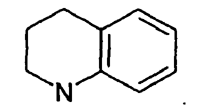 dont le noyau aromatique peut être substitué un radical methoxy.

Parmi les composés de formule générale **(I)**, on préférera en particulier les composés suivants décrits dans les exemples :
- 5,5'-[(méthylimino)*bis*(propane-3,1-diylimino)]*bis*(2-méthyl-1,3-benzothiazole-4,7-dione) ;
- 5,5'-[(méthylimino)*bis*(éthane-2,1-diylimino)]*bis*(2-méthyl-1,3-benzothiazole-4,7-dione) ;
- 5,5'-[oxy*bis*(éthane-2,1-diylimino)]*bis*(2-méthyl-1,3-benzothiazole-4,7-dione) ;
- 5,5'-(pentane-1,5-diyldiimino)*bis*(2-méthyl-1,3-benzothiazole-4,7-dione) ;
- 6,6'-[(méthylimino)bis(propane-3,1-diylimino)]bis(2-méthyl-1,3-benzothiazole-4,7-dione) ;
- 6,6'-[(méthylimino)bis(éthane-2,1-diylimino)]bis(2-méthyl-1,3-benzothiazole-4,7-dione) ;
- 5,5'-[(méthylimino)bis(propane-3,1-diylimino)]bis{4,7-dioxo-*N-*[3-(2-oxopyrrolidin-1-yl)propyl]-4,7-dihydro-1,3-benzothiazole-2-carboxamide} ;
- 6,6'-[(méthylimino)bis(propane-3,1-diylimmo)]bis[2-(2,5-difluorophényl)-1,3-benzoxazole-4,7-dione] ;
- 6,6'-[(méthylimino)bis(propane-3,1-diylimino)]bis[2-(3,5-dibromophényl)-1,3-benzoxazole-4,7-dione] ;
- 6,6'-[(méthylimino)bis(propane-3,1-diylimino)]bis[2-(2-chloro-6-fluorobenzyl)-1,3-benzoxazole-4,7-dione] ;
- 6,6'-[(méthylimino)bis(propane-3,1-diylimino)]bis[2-(3-bromophényl)-1,3-benzoxazole-4,7-dione] ;
- 6,6'-[(méthylimino)bis(propane-3,1-diylimino)]bis[2-(4-bromophényl)-1,3-benzoxazole-4,7-dione] ;
- 6,6'-[(méthylimino)bis(propane-3,1-diylimino)]bis[2-(3,5-difluorophényl)-1,3-benzoxazole-4,7-dione] ;
- 6,6'-[(méthylimino)bis(propane-3,1-diylimino)]bis[2-(3-chlorophényl)-1,3-benzoxazole-4,7-dione];
- 6,6'-[(méthylimino)bis(propane-3,1-diylimino)]bis[2-(4-fluorophényl)-1,3-benzoxazole-4,7-dione] ;
- 6,6'-[(méthylimino)bis(propane-3,1-diylimino)]bis[2-(4-bromo-3-méthylphényl)-1,3-benzoxazole-4,7-dione] ;
- 5,5'-[(méthylimino)bis(propane-3,1-diylimino)]bis(6-bromo-2-méthyl-1,3-benzothiazole-4,7-dione) ;
- 5,5',5"-[nitrilotris(propane-3,1-diylimino)]tris(2-méthyl-1,3-benzothiazole-4,7-dione) ;
- 5,5'-(2,2-diméthylpropane-1,3-diyldiimino)bis(2-méthyl-1,3-benzothiazole-4,7-dione) ;
- 5,5'-[cyclohexane-1,4-diylbis(méthyleneimino)]bis(2-méthyl-1,3-benzothiazole-4,7-dione) ;
- 5,5'-[1,3-phénylènebis(méthylèneimino)]bis(2-méthyl-1,3-benzothiazole-4,7-dione) ;
- 5,5'-[éthane-1,2-diylbis(oxypropane-3,1-diylimino)]bis(2-méthyl-1,3-benzothiazole-4,7-dione) ;
- 6,6'-{(méthylimino)bis[propane-3,1-diyl(méthylimino)]}bis[2-(2,5-difluorophényl)-1,3-benzoxazole-4,7-dione] ;
- *N*³-[2-(2,5-difluorophényl)-4,7-dioxo-4,7-dihydro-1,3-benzoxazol-6-yl]-*N*¹-(3-{[2-(2,5-difluorophényl)-4,7-dioxo-4,7-dihydro-1,3-benzoxazol-6-yl]amino}propyl)-β-alaninamide ;
- 5,5'-[(méthylimino)bis(propane-3,1-diylimino)]bis(1,3-benzothiazole-4,7-dione) ;
- 5,5'-[(méthylimino)bis(propane-3,1-diylimino)]bis[2-(morpholin-4-ylcarbonyl)-1,3-benzothiazole-4,7-dione] ;
- 5,5'-[(méthylimino)bis(propane-3,1-diylimino)]bis(2-{[4-(morpholin-4-ylcarbonyl)pipérazin-1-yl]carbonyl}-1,3-benzothiazole-4,7-dione) ;
- 5,5'-[(méthylimino)bis(propane-3,1-diylimino)]bis(2-{([4-(tétrahydrofuran-2-ylcarbonyl)pipérazin-1-yl]carbonyl}-1,3-benzothiazole-4,7-dione) ;
- 5,5'-[(méthylimino)bis(propane-3,1-diylimino)]bis(2-{[4-(méthylsulfonyl)pipérazin-1-yl]carbonyl}-1,3-benzothiazole-4,7-dione) ; ,
- 5,5'-[(méthylimino)bis(propane-3,1-diylimino)]bis[*N*-(4-méthoxyphenyl)-4,7-dioxo-4,7-dihydro-1,3-benzothiazole-2-carboxamide] ;
- 6,6'-[(méthylimino)bis(propane-3,1-diylimino)]bis[2-(pyrrolidin-1-ylcarbonyl)-1,3-benzothiazole-4,7-dione] ;
- 5,5'-[(méthylimino)bis(propane-3,1-diylimino)]bis[*N*-(4-fluorophényl)-4,7-dioxo-4,7-dihydro-1,3-benzothiazole-2-carboxamide] ;
- 5,5'-[(méthylimino)bis(propane-3,1-diylimino)]bis[*N*-(4-méthoxybenzyl)-4,7-dioxo-4,7-dihydro-1,3-benzothiazole-2-carboxamide] ;
- 5,5'-[(méthylimino)bis(propane-3,1-diylimino)]bis{2-[(6-méthoxy-3,4-dihydroquinolin-1(2*H*)-yl)carbonyl]-1,3-benzothiazole-4,7-dione} ;
- 5,5'-[(méthylimino)bis(propane-3,1-diylimino)]bis(*N*-cyclohexyl-4,7-dioxo-4,7-dihydro-1,3-benzothiazole-2-carboxamide) ;
- 5,5'-[(méthylimino)bis(propane-3,1-diylimino)]bis(2-{[4-(4-méthoxybenzoyl)pipérazin-1-yl]carbonyl}-1,3-benzothiazole-4,7-dione) ;
- 5,5'-[(méthylimino)bis(propane-3,1-diylimino)]bis(2-{[4-(2-furoyl)pipérazin-1-yl]carbonyl}-1,3-benzothiazole-4,7-dione) ;
- 6,6'-[(méthylimino)bis(propane-3,1-diylimino)]bis[2-(2-naphthyl)-1,3-benzothiazole-4,7-dione] ;
- 6,6'-[(méthylimino)bis(propane-3,1-diylimino)]bis[2-(1,3-benzodioxol-5-yl)-1,3 benzothiazole-4,7-dione] ;
- 6,6'-[(méthylimino)bis(propane-3,1-diylimino)]bis[2-(4-éthylphényl)-1,3-benzoxazole-4,7-dione] ;
- *N*-(4-méthoxyphényl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- 5-{[3-(méthyl{3-[(2-méthyl-4,7-dioxo-4,7-dihydro-1,3-benzothiazol-6-yl)amino]propyl}amino)propyl]amino}-2-{[4-(tétrahydrofuran-2-ylcarbonyl)pipérazin-1-yl]carbonyl}-1,3-benzothiazole-4,7-dione ;
- *N*-éthyl-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- 5-({3-[(3-{[4,7-dioxo-2-(pyrrolidin-1-ylcarbonyl)-4,7-dihydro-1,3-benzothiazol-6-yl]amino}propyl)(méthyl)amino]propyl}amino)-2-méthyl-1,3-benzothiazole-4,7-dione ;
- *N*-(4-méthoxybenzyl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- *N*-1,3-benzodioxol-5-yl-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- 2-[(6-méthoxy-3,4-dihydroquinolin-1(2*H*)-yl)carbonyl]-5-{[3-(méthyl{3-[(2-méthyl-4,7-dioxo-4,7-dihydro-1,3-benzothiazol-5-yl)amino]propyl}amino)propyl]amino}-1,3-benzothiazole-4,7-dione ;
- 2-{[4-(4-méthoxybenzoyl)pipérazin-1-yl]carbonyl}-5-{[3-(méthyl{3-[(2-méthyl-4,7-dioxo-4,7-dihydro-1,3-benzothiazol-5-yl)amino]propyl}amino)propyl]amino}-1,3-benzothiazole-4,7-dione ;
- 5-({3-[(3-{[2-(4-méthoxyphenyl)-4,7-dioxo-4,7-dihydro-1,3-benzothiazol-6-yl]amino}propyl)(méthyl)amino]propyl}amino)-2-méthyl-1,3-benzothiazole-4,7-dione ;
- 5-({3-[{3-[(4,7-dioxo-4,7-dihydro-1,3-benzothiazol-5-yl)amino]propyl}(méthyl)amino]propyl}amino)-2-méthyl-1,3-benzothiazole-4,7-dione ;
- 2-(2,5-difluorophényl)-6-{[3-(méthyl{3-[(2-méthyl-4,7-dioxo-4,7-dihydro-1,3-benzothiazol-5-yl)amino]propyl}amino)propyl]amino}-1,3-benzoxazole-4,7-dione ;
- 2-(4-éthylphényl)-6-{[3-(méthyl{3-[(2-méthyl-4,7-dioxo-4,7-dihydro-1,3-benzothiazol-5-yl)amino]propyl}amino)propyl]amino}-1,3-benzoxazole-4,7-dione ;
- 2-(2,5-difluorophényl)-6-{[3-(méthyl{3-[(2-méthyl-4,7-dioxo-4,7-dihydro-1,3-benzothiazol-6-yl)amino]propyl}amino)propyl]amino}-1,3-benzoxazole-4,7-dione ;
- 5,5'-[[(4-méthoxybenzyl)imino]bis(propane-3,1-diylimino)]bis(2-méthyl-1,3-benzothiazole-4,7-dione) ;
- 5,5'-[(méthylimino)bis(butane-4,1-diylimino)]bis(2-méthyl-1,3-benzothiazole-4,7-dione) ;
- 2-méthyl-5-{[3-(méthyl{4-[(2-méthyl-4,7-dioxo-4,7-dihydro-1,3-benzothiazol-5-yl)amino]butyl}amino)propyl]amino}-1,3-benzothiazole-4,7-dione ;
et les sels de ces composés.

L'invention concerne également, à titre de médicaments, les composés de formule générale **(I)** mentionnés ci-dessus, ou leurs sels pharmaceutiquement acceptables.

L'invention a encore pour objet les compositions pharmaceutiques comprenant, à titre de principe actif, un composé de formule générale **(I)** ou un sel pharmaceutiquement acceptable d'un tel composé, avec au moins un excipient pharmaceutiquement acceptable.

Un autre objet de l'invention est l'utilisation des composés de formule générale **(I)** ou de leurs sels pharmaceutiquement acceptables pour préparer un médicament destiné à traiter une maladie / un désordre choisi parmi les maladies suivantes / les désordres suivants : les maladies prolifératives tumorales (et en particulier le cancer), les maladies prolifératives non tumorales, les maladies neurodégénératives, les maladies parasitaires, les infections virales, l'alopécie spontanée, l'alopécie induite par des produits exogènes, l'alopécie radio-induite, les maladies auto-immunes, les rejets de greffes, les maladies inflammatoires et les allergies.

Tout particulièrement, les composés de formule générale **(I)** ou leurs sels pharmaceutiquement acceptables pourront être utilisés pour préparer un médicament destiné à traiter le cancer, et notamment le cancer du sein, les lymphomes, les cancers du cou et de la tête, le cancer du poumon, le cancer du colon, le cancer de la prostate et le cancer du pancréas.

L'invention concerne de plus une méthode de traitement de l'une des maladies / l'un des désordres mentionnés, ladite méthode comprenant l'administration au patient atteint de ladite maladie / dudit désordre d'un quantité thérapeutiquement efficace d'un composé de formule générale **(I)** ou d'un sel pharmaceutiquement acceptable d'un tel composé.

Les préférences mentionnées pour les composés de formule générale **(I)** et leurs sels sont bien entendu applicables *mutatis mutandis* aux composés de formules générales **(I)_{D}, (I)_{DS}, (I)_{DS5}, (I)_{DS6}, (I)_{DO}, (I)_{DO5}, (I)_{DO6}, (I)_{T}, (I)_{TS}, (I)_{TS5}, (I)_{TS6}, (I)_{TO}, (I)_{TO5}, (I)_{TO6}** ou **(I)_{DM},** ainsi qu'aux médicaments, compositions pharmaceutiques et utilisations selon l'invention concernant lesdits composés et leurs sels pharmaceutiquement acceptables.

Les compositions pharmaceutiques contenant un composé de l'invention peuvent se présenter sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de formule générale **(I)** par les procédés décrits ci-après.

### Préparation des composés de formule générale (I)

Les procédés de préparation ci-après sont donnés à titre illustratif et non limitatif.

### Méthodes générales:

### i) Composés de formule générale (I)_{D} dans laquelle R5 est différent de arylalkyl :

Les composés de formule générale **(I)_{D}** dans lesquels R³ représente un atome d'hydrogène, W, R² et R⁴ sont tels que définis précédemment et R¹ est tel que défini précédemment, mais où R⁵ ne représente pas un radical arylalkyl, peuvent être préparés selon la procédure résumée dans le schéma 1 ci-après.

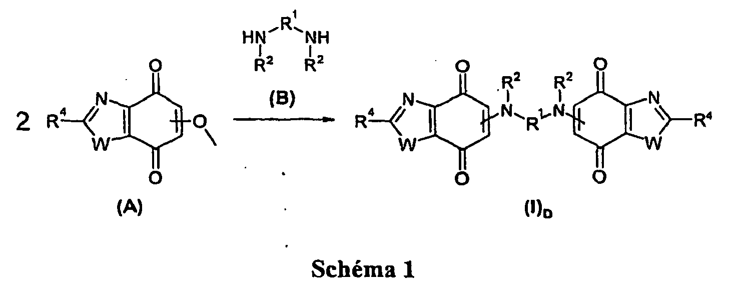

Selon cette méthode, les composés de formule générale **(I),** dans lesquels R¹, R², R⁴ et W sont tels que décrits ci-dessus, sont obtenus par traitement des composés de formule générale **(A),** avec des amines de formule générale R²HNR¹NHR² (ci-après les amines de formule générale **(B))** dans un solvant protique tel que le méthanol ou l'éthanol, à une température de préférence comprise entre 20 °C et 80 °C et éventuellement en présence d'une base telle que, par exemple, la diisopropyléthylamine (Yasuyuki Kita et coll., J. Org. Chem. (1996), 61,223-227).

### ii) Composés de formule générale (I)_{T}:

Les composés de formule générale **(I)_{T}** dans lesquels R³ représente un atome d'hydrogène et W, R¹, R² et R⁴ sont tels que définis précédemment peuvent être préparés selon la procédure résumée dans le schéma 1 *bis* ci-après. Les conditions réactionnelles sont analogues à celles utilisées pour la synthèse représentée dans le schéma 1.

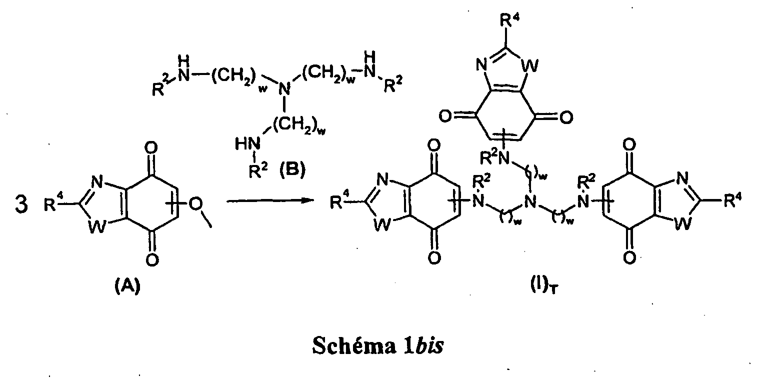

### iii) Composés de formule générale (I)_{D} où (I)_{T} dans lesquels R³ est un atome halogène :

D'une façon générale, les composés de formule générale **(I)** dans lesquels R³ est un atome halogène (Hal) peuvent être obtenus, schéma 1 *ter* (dans lequel seul la préparation des composés halogénés de formule générale **(I)_{D}** est représentée), à partir des composés de formule générale **(I)** dans lesquels R³ représente un atome d'hydrogène, par exemple par action de N-chlorosuccinimide ou N-bromosuccinimide dans un solvant aprotique tel que le dichlorométhane ou le tétrahydrofuranne (Paquette et Farley, J. Org. Chem. (1967), 32, 2725-2731), par action d'une solution aqueuse d'hypochlorite de sodium (eau de Javel) dans un solvant tel que l'acide acétique (Jagadeesh et coll., Synth Commun. (1998), 28, 3827-3833), par action de Cu(II) (dans un mélange CuCl₂/HgCl₂) en présence d'une quantité catalytique d'iode dans un solvant tel que l'acide acétique à chaud (Thapliyal, Synth. Commun. (1998), 28, 1123-1126), par action d'un agent tel que le dichloroiodate de benzyltriméthylammonium en présence de NaHCO₃ dans un solvant tel qu'un mélange dichlorométhane / méthanol (Kordik et Reitz, J. Org. Chem. (1996), 61, 5644-5645), ou encore par utilisation de chlore, de brome ou d'iode dans un solvant tel que le dichlorométhane (J. Renault, S. Giorgi-Renault et coll., J. Med. Chem. (1983), 26, 1715-1719).

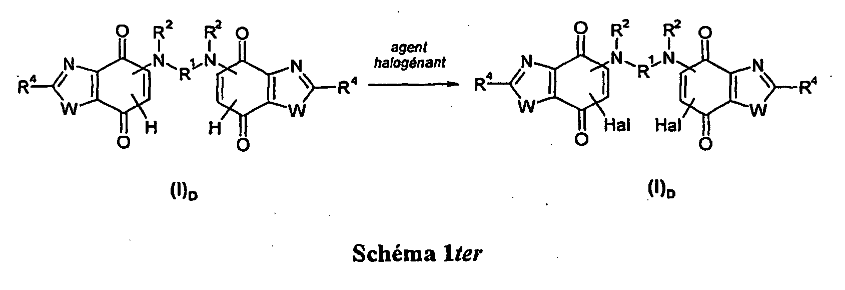

### iv) Composés de formule générale (I)_{DM} ou composés de formule générale (I)_{D} où R5 représente un radical arylalkyl :

iv.a) Composés de formule générale (I)_{DM} où R1 ne représente pas -(CH₂)ₛ-CO-NR⁸-(CH₂)ₜ- et si R¹ représente -(CH₂)ₘ-X-(CH₂)ₙ- , alors m = n et R⁵ ne représente pas un radical arylalkyl :

Les composés de formule générale (I)_{DM} dans lesquels R², R³, R⁴, R'⁴, W et W' sont tels que décrits ci-dessus et R¹ est tel que défini ci-dessus mais ne représente pas -(CH₂)ₛ-CO-N⁸-(CH₂)ₜ- et si R1 représente -(CH₂)ₘ-X-(CH₂)ₙ-, alors m = n et R⁵ ne représente pas un radical arylalkyl, peuvent être obtenus selon la procédure résumée dans le schéma 1*quater* ci-après.

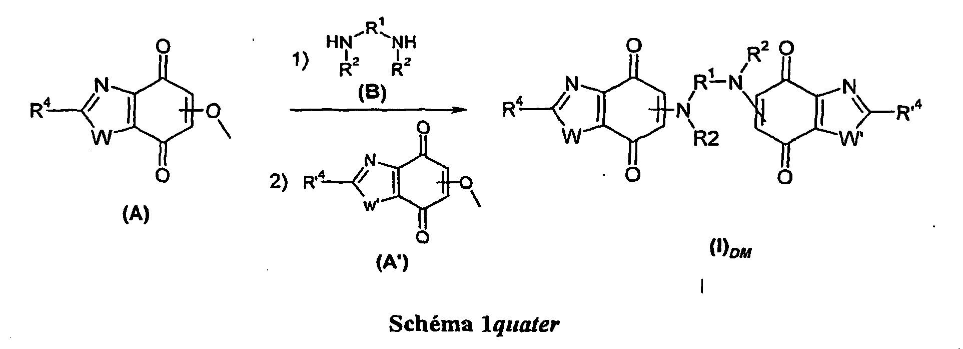

Selon cette procédure, les composés de formule générale **(I)**_{DM} sont obtenus par traitement des intermédiaires de formule générale **(A)** par une quantité stoechiométrique ou par un large excès de diamine de formule générale **(B)** dans un solvant protique tel que le méthanol ou l'éthanol, à une température de préférence comprise entre 20 °C et 80 °C, suivi par un traitement par une quantité stoechiométrique des intermédiaires de formule générale **(A')** tels que R'⁴ est différent de R⁴ et/ou W' est différent de W dans les mêmes conditions.
iv.b) Composés de formule générale **(I)**_{DM} ou si R¹ représente -(CH₂)ₘ-X-(CH₂)ₙ-, alors m ≠ n ou R⁵ représente un radical arylalkyl, et composés de formule générale (I)_{D} où si R¹ représente -(CH₂)ₘ-X-(CH₂)ₙ-, alors R⁵ représente arylalkyl :

Les composés de formule générale **(I)**_{DM} dans lesquels R², R³, R⁴, R^{,4}, W et W' sont tels que décrits ci-dessus et dans lesquels, si R¹ représente -(CH₂)ₘ-X-(CH₂)ₙ-, alors m ≠ n ou R⁵ représente arylalkyl , peuvent être obtenus selon la procédure résumée dans le schéma 1*quinquies* ci-après. Les composés de formules générale **(I)**_{D} dans lesquels W, R² et R⁴ sont tels que définis précédemment et tels que si R¹ représente le radical - (CH₂)ₘ-X-(CH₂)ₙ- et X représente -NR⁵- alors R⁵ représente un radical arylalkyl éventuellement substitué, peuvent être obtenus selon la procédure résumée dans le schéma 1*quinquies* ci-après (étant entendu que dans ce cas, W = W' et R⁴ = R^{4'}).

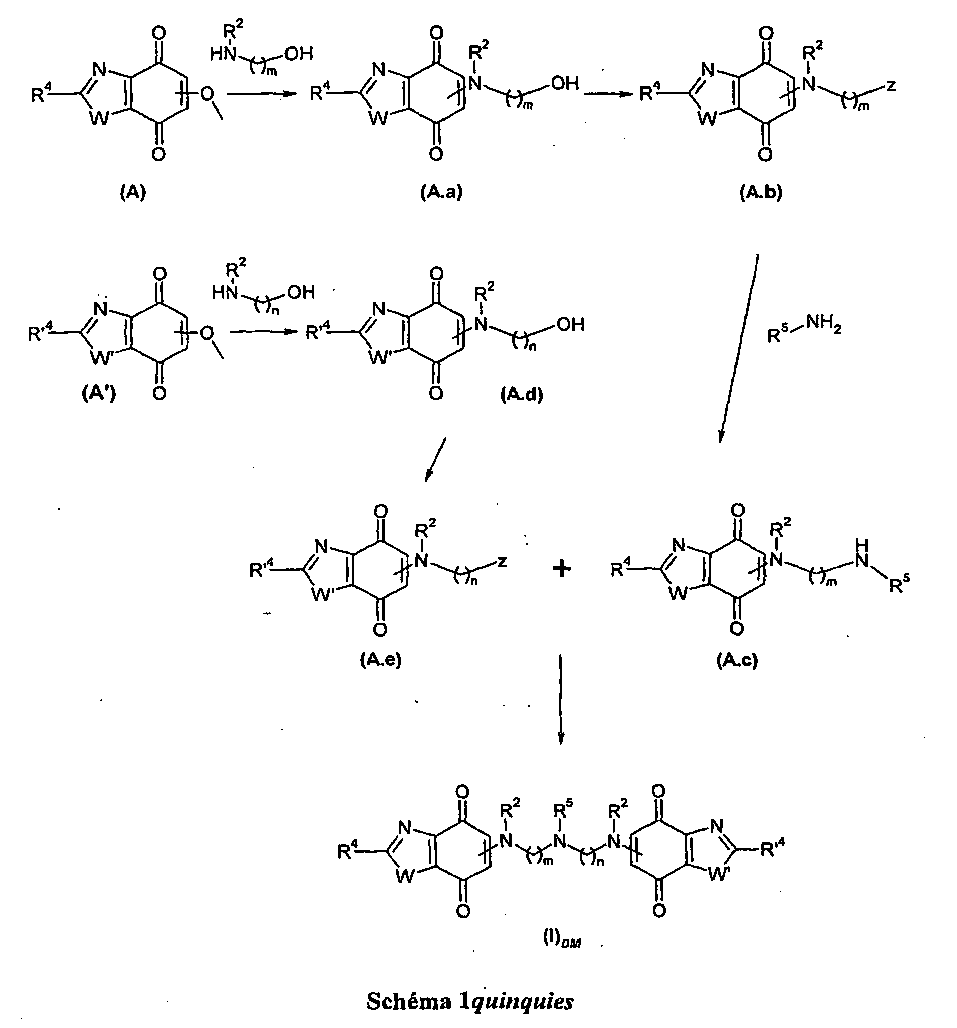

Selon cette méthode, les composés de formule générale **(I)**_{DM} ou **(I)**_{D} sont obtenus par substitution nucléophile d'un intermédiaire de formule générale **(A.e)** porteur d'un groupe partant Z tel que, par exemple, un groupement méthanesulfonate ou toluènesulfonate, ou un atome d'halogène tel qu'un atome de brome sur un intermédiaire de formule générale (**A.c**) en présence d'une base telle que, par exemple la diisopropyléthylamine dans un solvant polaire tel que, par exemple le diméthylformamide. Les intermédiaires de formule générale **(A.c)** sont obtenus dans les mêmes conditions par substitution nucléophile des intermédiaires de formule générale **(A.b)** par des amines primaires de formule générale R⁵NH₂. Les intermédiaires de formule générale **(A.b)** et **(A.e)** sont obtenus à partir des intermiédiaires de formule générale **(A)** et **(A')** respectivement par réaction avec des amines de formule générale NHR²-(CH₂)ₘ-Br et NHR²-(CH₂)ₙ-Br respectivement, dans un solvant protique polaire tel que le méthanol en présence d'une base telle que la triéthylamine à une température de préférence comprise entre 20°C et 80°C. Les intermédiaires de formule générale **(A.b)** et **(A.e)** peuvent également être obtenus à partir des intermédiaires de formule générale **(A.a)** et **(A.d)** respectivement par réaction avec du chlorure de *para*toluènesulfonyle, par exemple dans la pyridine ou dans le dichlorométhane en présence de triéthylamine. Les intermédiaires de formule générale **(A.a)** et **(A.d)** sont obtenus par traitement des intermédiaires de formule générale **(A)** et **(A')** respectivement, par des amines de formule générale NHR²-(CH₂)ₘ-OH et NHR²-(CH₂)ₙ-OH respectivement, dans un solvant protique tel que le méthanol ou l'éthanol, à une température de préférence comprise entre 20 °C et 80 °C.
iv.c) Composés de formule générale **(I)**_{DM} où R¹ représente -(CH₂)ₛ-CO-NR⁸-(CH₂)ₜ- :

Les composés de formule générale **(I)**_{DM} dans lesquels R², R³, R⁴, R'⁴, W et W' sont tels que décrits ci-dessus et R¹ représente -(CH₂)ₛ-CO-NR⁸-(CH₂)ₜ-, peuvent être obtenus selon la procédure résumée dans le schéma 1*sexies* ci-après.

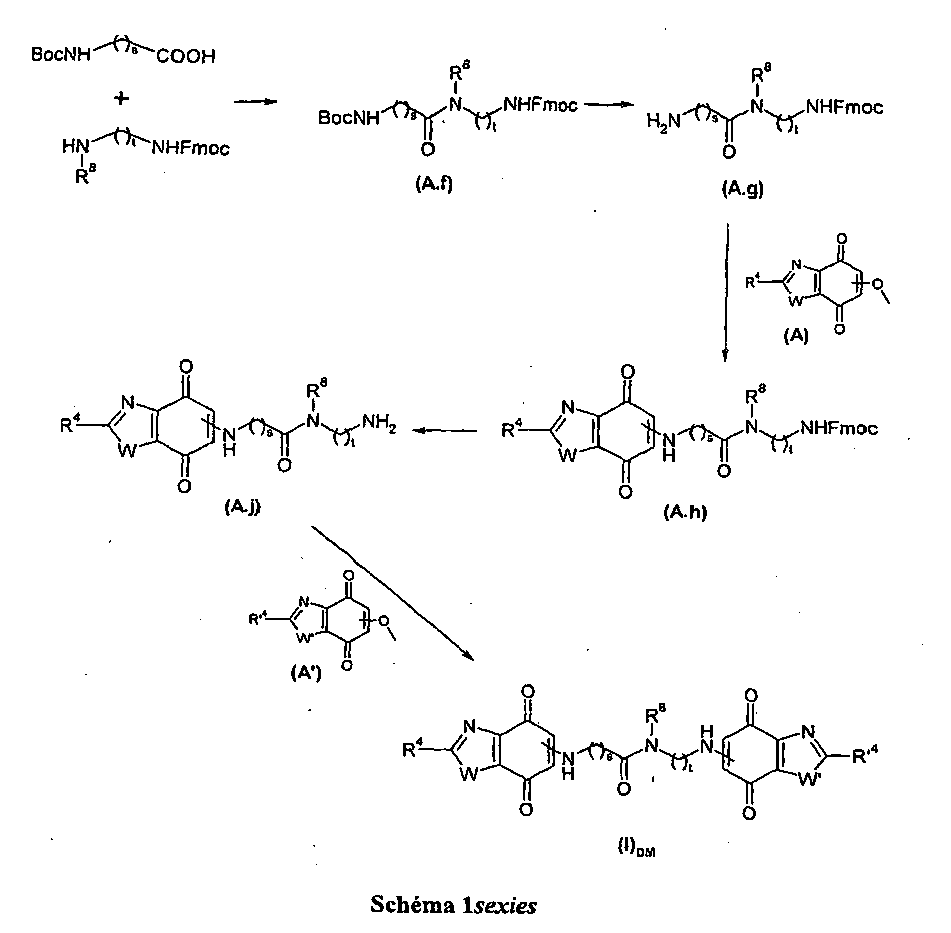

Selon cette procédure, les composés de formule générale **(I)**_{DM} sont obtenus par traitement des composés de formule générale **(A')** avec des amines de formule générale **(A.j)** dans un solvant protique tel que le méthanol ou l'éthanol à une température de préférence comprise entre 20 °C et 80 °C. Les intermédiaires de formule générale **(A.j)** sont eux-mêmes obtenus, après déprotection du groupement amine terminal selon des méthodes connues de l'homme du métier, à partir des intermédiaires de formule générale **(A)** sur lesquels on a fait réagir des amines de formule générale **(A.g)** dans les conditions décrites ci-dessus. Les intermédiaires de formule générale **(A.g)** sont obtenus par les méthodes classiques de couplage peptidique suivies d'une déprotection sélective du groupement carbamate également classique pour l'homme du métier.

### Préparation des intermédiaires de formule générale (A) et (A') :

Étant donné que les composés de formule générale **(A')** sont les mêmes que ceux de formule générale **(A),** seule la préparation des composés de formule générale **(A)** sera évoquée ci-après.

### i) W représente un atome de soufre :

Lorsque W représente un atome de soufre, les composés de formule générale **(A),** dans lesquels R⁴ a la même signification que dans la formule générale **(I),** peuvent être obtenus, schéma 2, par oxydation des composés de formule générale **(A.ii)** dans lesquels l'un de Q et Q' représente un radical amino et l'autre représente un atome d'hydrogène. Les composés de formule générale **(A.ii)** sont obtenus à partir des composés de formule générale **(A.i),** dans lesquels l'un de V et V' représente un radical nitro et l'autre représente un atome d'hydrogène, après réduction du groupement nitro par action d'hydrogène en présence de palladium sur charbon ou par action de chlorure d'étain.

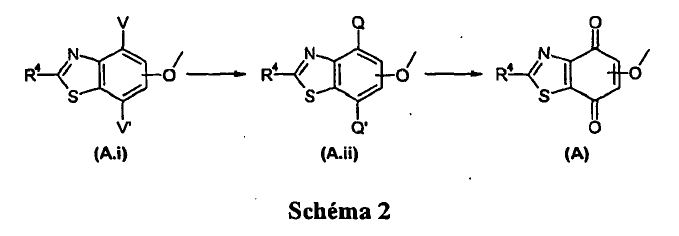

Les composés de formule générale **(A.i)** peuvent être synthétisés selon des méthodes déjà connues de l'homme du métier (voir par exemple la demande de brevet PCT WO 03/055868) ou bien peuvent être préparés selon les méthodes résumées dans les schémas ci-après.

Lorsque les composés de formule générale **(A.i)** sont tels que leur groupe méthoxy est en position 5 du noyau benzothiazole, leur préparation peut être effectuée selon la méthode résumée dans le schéma 3 ci-après. Selon ladite méthode, la 5-méthoxy-1,3-benzothiazol-2-amine (commerciale) est transformée, selon la méthode de Sandmeyer, connue de l'homme du métier, en 2-bromo-5-méthoxy-13-benzothiazole, lui-même nitré selon les méthodes connues de l'homme du métier pour obtenir le 2-bromo-5-méthoxy-4-nitro-13-benzothiazole. L'intermédiaire de formule générale **(A.i)** est alors obtenu par condensation avec des acides boroniques, selon la méthode de Suzuki, connue de l'homme du métier.

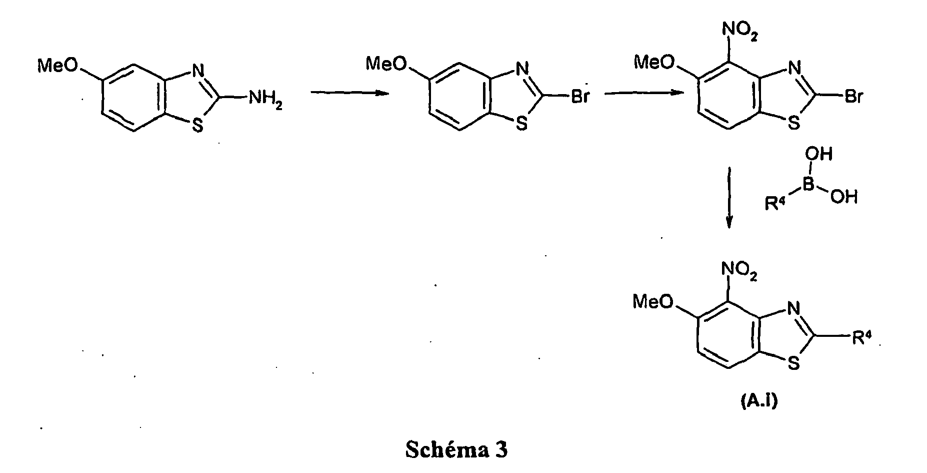

Lorsque les composés de formule générale **(A.i)** sont tels que leur groupe méthoxy est en position 6 du noyau benzothiazole, leur préparation peut être effectuée selon la méthode résumée dans le schéma 3*bis* ci-après. Selon ladite méthode, la 6-méthoxy-1,3-benzothiazol-2-amine (commerciale) est transformée, selon la méthode de Sandmeyer, connue de l'homme du métier, en 2-bromo-6-méthoxy-1,3-benzothiazole, lui-même nitré selon les méthodes connues de l'homme du métier pour obtenir le 2-bromo-6-méthoxy-7-nitro-1,3-benzothiazole. L'intermédiaire de formule générale **(A.i)** est alors obtenu par condensation avec des acides boroniques, selon la méthode de Suzuki, connue de l'homme du métier.

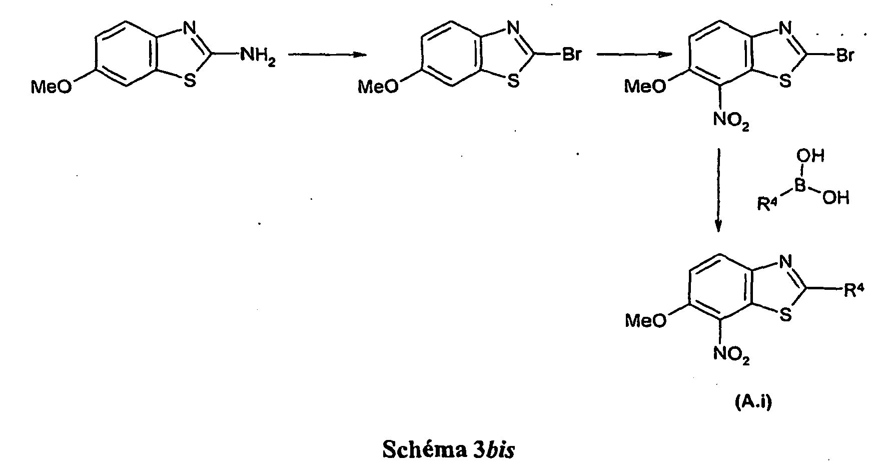

Dans le cas particulier où R⁴ représente un radical -CO-NR¹⁴R¹⁵, les intermédiaires de formule générale **(A.i)** peuvent être obtenus, schéma 4, à partir des composés de formule générale **(A.iii),** dans lequels V et V' sont tels que définis ci-dessus, et des amines de formule générale R¹⁴R¹⁵NH, en utilisant les conditions classiques de la synthèse peptidique (M. Bodansky, The Practice of Peptide Synthesis, 145 (Springer-Verlag, 1984)), par exemple dans le dichlorométhane en présence d'un réactif de couplage tel que l'hexafluorophosphate de bromo-tris-pyrrolidino-phosphonium (PyBroP) en présence de diméthylaminopyridine (DMAP) (Coste et coll., Tetrahedron Lett. (1990), 31, 669), ou dans un mélange (diméthylformamide/dichlorométhane/dioxane : 1/1/1) en présence de chlorhydrate de 1-(3-diméthylaminopropyl)-3-éthylcarbodiimide, de diisopropyléthylamine et d'une quantité catalytique de diméthylaminopyridine, ou encore par formation d'un chlorure d'acide intermédiaire obtenu par addition de chlorure d'oxalyle en solution dans du dichlorométhane. Les composés de formule générale **(A.üi)** peuvent, quant à eux, être obtenus par oxydation des carboxaldéhydes de formule générale **(A.iv)** par action d'un oxydant tel que, par exemple du chlorite de sodium dans une solution tamponnée d'hydrogénophosphate de sodium (pH 3,5) et dans une solution aqueuse de tert-butanol en présence de 2-méthyl-2-butène ; ces aldéhydes de formule générale **(A.iv)** étant eux-même obtenus par oxydation des composés de formule générale **(A.v)** par action, par exemple, d'oxyde de sélénium dans du 1,4-dioxane à 80 °C (Bu et coll., J. Med. Chem. (2001), 44, 2004-2014). Enfin, les composés de formule générale **(A.v)** dans lesquels V et V' sont tels que définis ci-dessus, peuvent être obtenus selon des méthodes déjà connues de l'homme du métier (voir par exemple la demande de brevet PCT WO 03/055868).

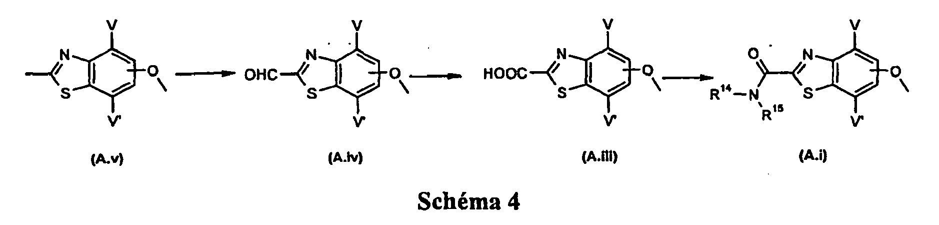

Dans le cas particulier où R⁴ représente H, les intermédiaires de formule générale **(A)** peuvent être obtenus par décarboxylation des intermédiaires de formule générale **(A.iii),** après chauffage de ceux-ci pendant 2 heures à une température comprise entre 40°C et 80°C dans un solvant organique tel que le dichlorométhane ou l'acétonitrile.

Dans le cas particulier où R⁴ représente un radical -CH₂-NR⁹R¹⁰, les intermédiaires de formule générale **(A.i)** peuvent être obtenus, schéma 5, à partir des composés de formule générale **(A.vi),** dans lequels V et V' sont tels que définis ci-dessus, et des amines de formule générale R⁹R¹⁰NH, dans un solvant polaire en présence d'une base telle que la diisopropyléthylamine et d'une quantité catalytique d'iodure de sodium. Les composés de formule générale **(A.vi)** sont obtenus par nitration, selon les méthodes classiques connues de l'homme du métier, des intermédiaires de formule générale **(A.vii),** eux-mêmes obtenus à partir des composés de formule générale **(A.viii)** que l'on soumet à une réaction de bromation radicalaire à l'aide de *N*-bromosuccinimide en présence d'un initiateur tel que le 2,2'-azobis(2-méthylpropionitrile) ou le dibenzoylperoxyde dans un solvant aprotique tel que le tétrachlorure de carbone (CCl₄) à une température de préférence comprise entre la température ambiante (i.e. environ 25 °C) et 80 °C et sous irradiation par une lampe UV (Mylari et coll., J. Med. Chem. (1991), 34; 108-122).

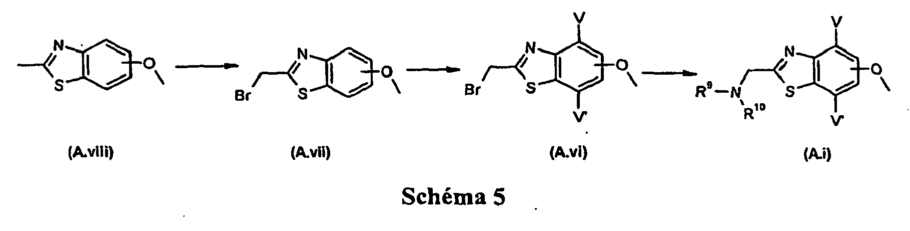

### ii) W représente un atome d'oxygène :

Lorsque W représente un atome d' oxygène, les composés de formule générale **(A),** dans lesquels R⁴ a la même signification que dans la formule générale **(I)**, peuvent être obtenus, schéma 6, par oxydation des composés de formule générale **(A.ix)** dans lesquels l'un de Q et Q' représente un radical amino et l'autre représente un atome d'hydrogène, ou bien l'un de Q et Q' représente un radical hydroxyle et l'autre un atome d'hydrogène, selon les méthodes connues de l'homme du métier (voir par exemple la demande de brevet PCT WO 03/055868). Les composés de formule générale **(A.ix)** sont eux-mêmes obtenus par condensation des composés de formule générale **(A.x)** dans lesquels Q et Q' ont la même signification que ci-dessus avec, par exemple, des thioimidates de formule générale (C) dans un solvant protique tel que l'éthanol à une température comprise entre 25 °C et la température d'ébullition du solvant (selon la méthode décrite notamment par S. Rostamizadeh et coll. J. Chem Res, Synop, 6, (2001), 227-228).

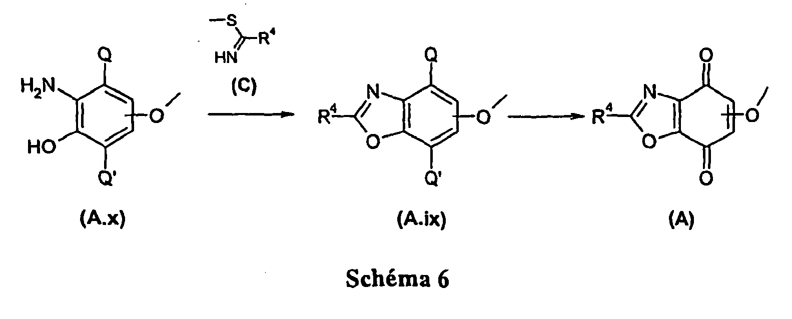

Les composés de formule générale **(A.x)** peuvent être obtenus selon des méthodes connues de l'homme du métier résumées dans les schémas 6*bis* et 6*ter* ci-après

Lorsque les composés de formule générale **(A.ix)** sont tels que leur groupe méthoxy est en position 5 du noyau benzoxazole, la préparation des composés de formule générale **(A.x)** peut être effectuée selon la méthode résumée dans le schéma 6*bis* ci-après. Selon ladite méthode, le 4-méthoxy-2,6-dinitrophénol (décrit notamment par P. Cotelle et J.-P. Catteau, Synth. Commun., 26, (1996), 4105-4112) est réduit, par exemple par action d'hydrogène en présence de palladium sur charbon, pour donner le composé de formule générale **(A.x)** correspondant.

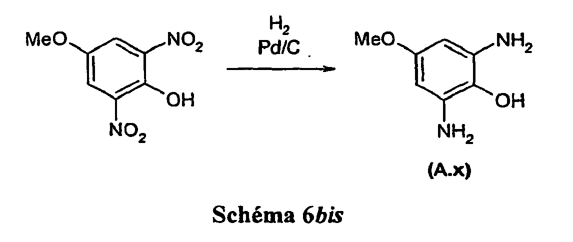

Lorsque les composés de formule générale **(A.ix)** sont tels que leur groupe méthoxy est en position 6 du noyau benzoxazole, la préparation des composés de formule générale **(A.x)** peut être effectuée selon la méthode résumée dans le schéma *6ter* ci-après. Selon ladite méthode, le 5-méthoxy-2-nitro-résorcinol (décrit notamment par J.F. Grove et coll. J. Chem. Soc. (1956), 1956-1963) est réduit, par exemple par action d'hydrogène en présence de palladium sur charbon, pour donner le composé de formule générale (A.x) correspondant.

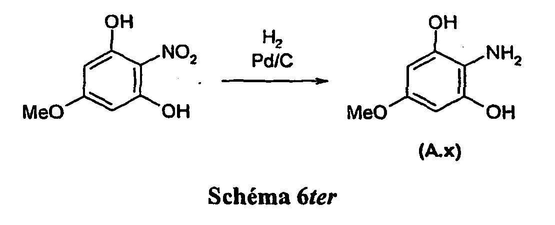

### Préparation des amines de formule générale (B) :

Les amines de formule générale **(B)** sont commerciales ou peuvent être facilement préparées selon des méthodes courantes pour l'homme du métier

### Exemples

En ce qui concerne les températures auxquelles il est fait référence dans le présent texte, le terme « environ *XX*°C » indique que la température en question correspond à un intervalle de plus ou moins 10 °C autour de la température de *XX*°C, et de préférence à un intervalle de plus ou moins 5 °C autour de la température de *XX*°C.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

Les points de fusion ont été mesurés à l'aide d'un appareil à capillaire Büchi 535.

Les spectres de RMN ont été enregistrés à l'aide d'un spectromètre Brücker ARX 400. Les déplacements chimiques sont exprimés en partie par million (ppm) par rapport au tétraméthylsilane (TMS) et la multiplicité des signaux est donnée sous forme de s (singulet), d (doublet), t (triplet), q (quadruplet), m (multiplet).

### Méthode employée pour la mesure du temps de rétention (t.r.) et du pic moléculaire (MH+)

Les composés sont caractérisés par leur temps de rétention (t.r.), exprimé en minutes, déterminé par chromatographie liquide (CL), et leur pic moléculaire (MH+) déterminé par spectrométrie de masse (SM), un spectromètre de masse simple quadripôle (Micromass, modèle Platform) équipé d'une source électrospray est utilisé avec une résolution de 0,8 da à 50% de vallée.
Pour les exemples 1 à 56 ci-après, les conditions d'élution correspondant aux résultats indiqués sont les suivantes : élution avec le mélange acétonitrile-eau-acide trifluoroacétique 50-950-0,2 (A) pendant 1 minute puis passage du mélange (A) à un mélange acétonitrile-eau 950-50 (B) par un gradient linéaire sur une période de 7,5 minutes, puis élution avec le mélange B pur pendant 2 minutes.

### Exemple 1 : 5,5'-[(méthylimino)bis(propane-3,1-diylimino]bis(2-méthyl-1,3-benzothiazole-4,7-dione) :

83,4 mg (0,57 mmol ; 0,6 équivalents) de *N-*(3-aminopropyl)-*N-*méthylpropane-1,3-diamine sont ajoutés à 200 mg (0,95 mmol) de 5-méthoxy-2-méthyl-4,7-dioxobenzothiazole en solution dans 15 ml d'éthanol anhydre. Le mélange réactionnel est agité à 60 °C pendant 2 heures, puis le solvant est évaporé sous pression réduite. Le résidu est purifié sur colonne de silice (éluant : méthanol à 3% dans du dichlorométhane) et 30 mg (rendement = 36%) de produit attendu sont obtenus sous forme d'une poudre rouge. Point de fusion : 94-96 °C.
RMN ¹H (DMSO d6, 400 MHz, δ) : 7,93 (t, 2H, 2NH) ; 5,40 (s, 2H, 2CH) ; 3,20-3,15 (m, 4H) ; 2,72 (s, 6H, 2CH₃) ; 2,40-2,37 (m, 4H) ; 2,17 (s, 3H, CH₃) ; 1,76-1,73 (m, 4H).
SM-CL : MH+ = 500,15 ; t.r. = 7,49 min.

*Les composés des exemples 2 à 4 sont obtenus de façon analogue à celle employée pour l'exemple 1, l'amine adéquate remplaçant la N-(3-aminopropyl)-N-méthylpropane-1,3-diamine.*

### Exemple 2 : 5,5'-[(lnéthylimino)bis(éthane-2,1-diylimino)]bis(2-méthyl-1,3-benzothiazole-4,7-dione) :

Poudre rouge. Point de fusion : 188-190 °C.
RMN ¹H (DMSO d6, 400 MHz, δ) : 7,32 (t, 2H, 2NH) ; 5,43 (s, 2H, 2CH) ; 3,23-3,19 (m, 4H) ; 2,72 (s, 6H, 2CH₃) ; 2,66-2,62 (m, 4H) ; 2,31 (s, 3H, CH₃). SM-CL : MH+ = 472,18 ; t.r. = 7,31 min.

### Exemple 3 : 5,5'-[oxybis(éthane-2,1-diylimino)]bis(2-méthyl-1,3-benzothiazole-4,7-dione) :

Poudre rouge.
RMN ¹H (DMSO d6, 400 MHz, δ) : 7,53 (t, 2H, 2NH) ; 5,50 (s, 2H, 2CH); 3,66-3,64 (m, 4H, 2CH₂); 3,30-3,29 (m, 4H, 2CH₂); 2,74 (s, 6H, 2CH₃).
SM-CL : MH+ = 459,06 ; t.r. = 8,20 min.

### Exemple 4 : 5,5'-(pentane-1,5-diyldiimino)bis(2-méthyl-1,3-benzothiazole-4,7-dione) :

Poudre rouge.
RMN ¹H (DMSO d6, 400 MHz, δ) : 7,74 (t, 2H, 2NH) ; 5,45 (s, 2H, 2CH) ; 3,17-3,12 (m, 4H, 2CH₂) ; 2,74 (s, 6H, 2CH₃) ; 1,61-1,56 (m, 4H, 2CH₂) ; 1,36-1,33 (m, 2H, CH₂).
SM-CL : MH+ = 457,11 ; t.r. = 8,97 min.

*Les composés des exemples 5 et 6 sont obtenus de façon analogue à celle employée pour l'exemple 1, le 6-méthoxy-2-méthyl-4,7-dioxobenzothiazole remplaçant le 5-méthoxy-2-méthyl-4,7-dioxobenzothiazole et l'amine adéquate remplaçant en outre la N-(3-aminopropyl)-N-méthylpropane-1,3-diamine dans le cas de l'exemple 6.*

### Exemple 5 : 6,6'-[(méthylimino)bis(propane-3,1-diylimino)]bis(2-méthyl-1,3-benzothiazole-4,7-dione) :

Poudre rouge. Point de fusion: 91-93 °C.
RMN ¹H (DMSO d6, 400 MHz, δ) : 7,80 (t, 2H, 2NH) ; 5,34 (s, 2H, 2CH); 3,20-3,15 (m, 4H); 2,76 (s, 6H, 2CH₃) ; 2,39-2,37 (m, 4H) ; 2,17 (s, 3H, CH₃) ; 1,76-1,73 (m, 4H).
SM-CL : MH+ = 500,35 ; t.r. = 7,43 min.

### Exemple 6 : 6,6'-[(méthylimino)bis(éthane-2,1-diylimino)]bis(2-méthyl-1,3-benzothiazole-4,7-dione) :

Poudre rouge.
RMN ¹H (DMSO d6, 400 MHz, δ) : 7,13 (t, 2H, 2NH) ; 5,37 (s, 2H, 2CH) ; 3,23-3,19 (m, 4H) ; 2,77 (s, 6H, 2CH₃) ; 2,66-2,61 (m, 4H); 2,32 (s, 3H, CH₃).
SM-CL : MH+ = 472,30 ; t.r. = 7,17 min.

*Les composés des exemples 7 à 16 sont obtenus de façon analogue à celle employée pour l'exemple 1*, *la quinone adéquate remplaçant le 5-méthoxy-2-méthyl-4,7-dioxobenzothiazole.*

### Exemple 7 : 5,5'-[(méthylimino)bis(propane-3,1-diylimino)]bis{4,7-dioxo-N-[3-(2-oxopyrrolidin-1-yl)propyl]-4,7-dihydro-1,3-benzothiazole-2-carboxamide} :

Poudre rouge. Point de fusion : 185-186 °C.
RMN ¹H (DMSO d6, 400 MHz, δ) : 9,11 (t, 2H, 2NH); 8,18 (t, 2H, 2NH); 5,54 (s, 2H, 2CH); 3,37-3,18 (m, 16H); 2,40 (m, 4H); 2,22 (m, 4H); 2,18 (s, 3H, CH₃) ; 1,92 (m, 4H); 1,74 (m, 8H).
SM-CL : MH+ = 808,51 ; t.r. = 7,61 min.

### Exemple 8: 6,6'-[(méthylimino)bis(propane-3,1-diylimino)]-bis[2-(2,5-difluorophényl)-1,3-benzoxazole-4,7-dione] :

Poudre rouge. Point de fusion : 209-210 °C.

RMN ¹H (DMSO d6, 400 MHz, δ) : 8,00 (m, 2H, H arom.) ; 7,82 (m, 2H, 2NH) ; 7,55-7,50 (4H, H arom.) ; 5,31 (s, 2H, 2CH) ; 3,22-3,19 (m, 4H) ; 2,48-2,44 (m, 4H) ; 2,22 (s, 3H, CH₃) ; 1,80-1,77 (m, 4H).
SM-CL : MH+ = 664,40 ; t.r. = 8,81 min.

### Exemple 9 : 6,6'-[(méthylimino)bis(propane-3,1-diylimino)]-bis[2-(3,5-dibromophényl)-1,3-benxoxazole-4,7-dione] :

Poudre rouge. Point de fusion : 227-228 °C.
RMN ¹H (CF₃COOD, 400 MHz, δ) : 7,94 (m, 4H, H arom.) ; 7,69 (m, 2H, H arom.) ; 4,94 (s, 2H, 2CH) ; 3,34-3,27 (m, 6H) ; 3,20-3,13 (m, 2H) ; 2,84 (s, 3H, CH₃) ; 2,17-2,06 (m, 4H).
SM-CL : MH+ = 903,90 ; t.r. = 10,11 min.

### Exemple 10 : 6,6'-[(méthylimino)bis(propane-3,1-diylimino)]bis[2-(2-chloro-6-fluorobenzyl)-1,3-benzoxazole-4,7-dione] :

Poudre rouge. Point de fusion : 165-167 °C.
RMN ¹H (DMSO d6, 400 MHz, δ) - 7,87 (t, 2H, 2NH) ; 7,48-7,39 (m, 4H, H arom.); 7,34-7,29 (m, 2H, H arom.) ; 5,25 (s, 2H, 2CH) ; 4,46 (s, 4H, 2CH₂); 3,18-3,13 (m, 4H) ; 2,38-2,36 (m, 4H) ; 2,15 (s, 3H, CH₃) ; 1,73-1,70 (m, 4H).
SM-CL : MH+ = 724,44 ; t.r. = 9,13 min.

### Exemple 11 : 6,6'-[(méthylimino)bis(propane-3,1-diylimino)]-bis[2-(3-bromophényl)-1,3-benzoxazole-4,7-dione] :

Poudre rouge. Point de fusion : 199-200 °C.
RMN ¹H (DMSO d6, 400 MHz, δ) : 8,08-8,00 (m, 6H, H arom.) ; 7,81-7,79 (m, 2H, H arom.) ; 7,54-7,50 (m, 2H, 2NH) ; 5,29 (s, 2H, 2CH) ; 3,21-3,16 (m, 4H) ; 2,42 (m, 4H) ; 2,20 (s, 3H, CH₃) ; 1,80-1,77 (m, 4H).
SM-CL : MH+ = 748,21 ; t.r. = 9,37 min.

### Exemple 12 : 6,6'-[(méthylimino)bis(propane-3,1-diylimino)]-bis[2-(4-bromophényl)-1,3-benzoxazole-4,7-dione] :

Poudre rouge. Point de fusion : 233-234 °C.
RMN ¹H (CF₃COOD, 400 MHz, δ) : 7,76-7,74 (m, 4H, H arom.) ; 7,49-7,47 (m, 4H, H arom.) ; 3,34-3,26 (m, 6H) ; 3,20-3,12 (m, 2H) ; 2,83 (s, 3H, CH₃) ; 2,13-2,06 (m, 4H).
SM-CL : MH+ = 748,22 ; t.r. = 9,20 min.

### Exemple 13 : 6,6'-[(méthylimino)bis(propane-3,1-diylimino)]-bis[2-(3,5-difluorophényl)-1,3-benzoxazole-4,7-dione] :

Poudre rouge.
RMN ¹H (DMSO d6, 400 MHz, δ) : 8,20-8,17 (m, 2H) ; 7,61-7,58 (m, 4H); 7,52-7,47 (m, 2H) ; 5,31 (s, 2H, 2CH) ; 3,21-3,17 (m, 4H); 2,44-2,41 (m, 4H) ; 2,20 (s, 3H, CH₃) ; 1,82-1,76 (m, 4H).
SM-CL : MH+ = 664,30 ; t.r. = 9,06 min.

### Exemple 14: 6,6'-[(méthylimino)bis(propane-3,1-diylimino)]-bis[2-(3-chlorophényl)-1,3-benzoxazole-4,7-dione] :

Poudre rouge. Point de fusion : 196-197 °C.
RMN ¹H (DMSO d6, 400 MHz, δ) : 8,00-7,95 (m, 6H, 6 arom.) ; 7,68-7,66 (m, 2H, H arom.) ; 7,58 (t, 2H, 2NH) ; 5,29 (s, 2H, 2CH) ; 3,21-3,16 (m, 4H) ; 2,43 (m, 4H) ; 2,21 (m, 3H, CH₃) ; 1,80-1,77 (m, 4H).
SM-CL : MH+ = 660,26 ; t.r. = 9,11 min.

### Exemple 15 : 6,6'-[(méthylimino)bis(propane-3,1-diylimino)]-bis[2-(4-fluorophényl)-1,3-benzoxazole-4,7-dione] :

Poudre rouge. Point de fusion : 204-205 °C.
RMN ¹H (DMSO d6, 400 MHz, δ) : 8,10-8,06 (m, 4H, H arom.) ; 7,96 (t, 2H, 2NH) ; 7,41-7,37 (m, 4H, H arom.) ; 5,28 (s, 2H, 2CH); 3,21-3,16 (m, 4H) ; 2,44 (m, 4H) ; 2,21 (m, 3H, CH₃) ; 1,80-1,77 (m, 4H),
SM-CL : MH+= 628,32 ; t.r. = 8,70 min.

### Exemple 16 : 6,6'-[(méthylimino)bis(propane-3,1-diylimino)]bis[2-(4-bromo-3-méthylphényl)-1,3-benzoxazole-4,7-dione] :

Poudre rouge.
SM-CL : MH+ = 776,17 ; t.r. = 9,76 min.

### Exemple 17: 5,5'-[(méthylimino)bis(propane-3,1-diylimino)]bis(6-bromo-2-méthyl-1,3-benzothiazole-4,7-dione) :

70 mg (0,2 mmol) du composé de l'exemple 1 sont mis en solution dans 10 ml d'acide acétique. 55 mg (4,4 mmol ; 2,2 éq.) de *N*-bromosuccinimide sont ajoutés et le mélange réactionnel est agité pendant 1 heure à température ambiante. Après concentration sous pression réduite, le résidu est purifié par chromatographie sur colonne de silice (éluant : dichlorométhane/méthanol 95/5) et le produit attendu est obtenu, après reprise dans de l'éther éthylique et filtration, sous forme d'une poudre violette.

RMN ¹H (DMSO d6, 400 MHz, δ) : 7,99 (s, 2H, 2NH) ; 3,83-3,79 (m, 4H) ; 2,72 (s, 6H, 2CH₃) ; 2,47-2,44 (m, 4H) ; 2,19 (s, 3H, CH₃) ; 1,78 (m, 4H).

SM-LC : MH+ = 655,96 ; t.r. = 7,83 min.

*Le composé de l'exemple 18 est obtenu de façon analogue à celle employée pour l'exemple 1, la triamine adéquate remplaçant la* N*-(3-aminopropyl)-N*-*méthylpropane-1,3-diamine.*

### Exemple 18: 5,5',5"-[nitrilotris(propane-3,1-diylimino)]tris(2-méthyl-1,3-benzothiazole-4,7-dione) :

Poudre rouge. Point de fusion = 131-132°C.
RMN ¹H (DMSO d6, 400 MHz, δ) : 7,84-7,81 (m, 3H) ; 5,38 (s, 3H) ; 3,18-3,13 (m, 6H) ; 2,71 (s, 9H, 3CH₃); 2,49-2,45 (m, 6H); 1,79-1,74 (m, 6H).
SM-CL : MH+ = 720,24 ; t.r. = 7,70 min.

*Les composés des exemples 19 à 22 sont obtenus de façon analogue à celle employée pour l'exemple 1, la diamine adéquate remplaçant la* N*-(3-aminopropyl)-*N-*méthylpropane-1,3-diamine.*

### Exemple 19: 5,5'-(2,2-diméthylpropane-1,3-diyldümino)bis(2-méthyl-1,3-benzothiazole-4,7-dione) :

Poudre rouge.
SM-CL : MH+ = 457,17 ; t.r. = 8,88 min.

### Exemple 20 : 5,5'-[cyclobexane-1,4-diylbis(méthyleneimino)]bis(2-méthyl-1,3-benzothiazole-4,7-dione) :

Poudre rouge. Point de fusion > 240°C.

*Les composés des exemples 23 et 24 sont obtenus de façon analogue à celle employée pour l'exemple 1, la diamine adéquate remplaçant la* N*-(3-aminopropyl)-*N-*méthylpropane-1,3-diamine et la 2-(2,5-difluorophényl)-6-méthoxy-1,3-benzoxazole-4,7-dione remplaçant le 5-méthoxy-2-méthyl-4,7-dioxobenzothiazole.*

### Exemple 23 _{:} 6,6'-{(méthylimino)bis[propane-3,1-diyl(méthylimino)]}bis[2-(2,5-difluorophényl)-1,3-benzoxazole-4,7-dione] :

Poudre violet foncé.
RMN ¹H (DMSO d6, 400 MHz, δ) : 7,85-7,81 (m, 2H); 7,57-7,48 (m, 4H); 5,49 (s, 2H) ; 3,66-3,63 (m, 4H); 3,06 (s, 6H, 2CH₃); 2,33-2,29 (m, 4H); 2,09 (s, 3H, CH₃) ; 1,78-1,74 (m, 4H).
SM-CL : MH+ = 692,34 ; t.r. = 8,76 min.

### Exemple 24 : N³-[2-(2,5-difluorophényl)-4,7-dioxo-4,7-dihydro-1,3-benzoxazol-6-yl]-N¹-(3-{[2-(2,5-difluorophényl)-4,7-dioxo-4,7-dihydro-1,3-benzoxazol-6-yL]amino}propyl)-β-alaninamide :

Poudre rouge.
SM-CL : MH+ = 664,29 ; t.r. = 9,90 min.

### Exemple 25 : 5,5'-[(méthylimino)bis(propane-3,1-diylimino)]bis(1,3-benzothiazole-4,7-dione) :

### 25.1) 5-méthoxy-4-nitro-1,3-benzothiazole :

### 25.1.1) 5-méthoxy-4-nitro-1,3-beruothiazole-2-carbaldéhyde :

12,8 g (0,115 mol; 6 équivalents) de dioxyde de sélénium sont ajoutés à 4,34 g (19,3 mmol) de 5-méthoxy-2-méthyl-4-nitro-1,3-benzothiazole en solution dans 180 ml de dioxane anhydre. Le mélange réactionnel est agité à 80 °C pendant 18 heures puis l'insoluble est filtré et le solvant est évaporé sous pression réduite. L'aldéhyde attendu est obtenu sous forme d'une huile jaune et purifié sur colonne de silice (éluant : acétate d'éthyle / heptane : gradient de 30% à 70%). Point de fusion : 154-155 °C.
RMN ¹H (DMSO d6, 400 MHz, δ) : 10,06 (s, 1H, CHO) ; 8,52-8,49 (d, 1H, H arom) ; 7,80-7,78 (d, 2H, H arom) ; 4,04 (s, 3H, OCH₃).

### 25.1.2) Acide 5-méthoxy-4-nitro-1,3-benzothiazole-2-carboxylique :

Une solution de 18 g de chlorite de sodium et de 18 g d'hydrogénophosphate de sodium dans 180 ml d'eau est ajoutée goutte à goutte au résidu carbaldéhyde repris dans 420 ml de *tert*-butanol et 100 ml de 2-méthyl-but-2-ène. Le mélange réactionnel est maintenu sous agitation pendant 18 heures à température ambiante, puis l'insoluble est filtré, repris par de l'eau et la solution aqueuse obtenue est acidifiée par une solution 1*M* d'acide chlorhydrique. Le précipité obtenu est filtré et lavé par de l'eau. L'acide est obtenu sous forme d'une poudre beige. (m = 3,12 g ; rendement = 64%). Point de fusion : 140-142 °C.

RMN ¹H (DMSO d6, 400 MHz, δ) : 8,45-8,43 (d, 1H, H arom) ; 7,74-7,71 (d, 1H, H arom) ; 3,99 (s, 3H, CH₃).
SM-CL : MH+ = 254,99 ; t.r. = 8,20 min.

### 25.1.3) 5-méthoxy-4-nitro-1,3-benzothiazole :

3 g (11,8 mmol) d' acide 5-méthoxy-4-nitro-1,3-benzothiazole-2-carboxylique sont mis en suspension dans 200 ml de dichlorométhane et chauffés à 60°C pendant 2 heures. Le dichlorométhane est évaporé sous pression réduite. Le 5-méthoxy-4-nitro-1,3-benzothiazole est obtenu sous forme d'une poudre beige. (m = 2g ; rendement = 80%). RMN ¹H (DMSO d6, 400 MHz, δ) : 9,53 (s, 1H); 8,35 (d, 1H); 7,60 (s, 1H).
SM-CL : MH+ = 211,01 ; t.r. = 9,30 min.

### 25.2) 5-méthozy-1,3-benzothiazol-4-amine :

730 mg (3,5 mmol) de 5-méthoxy-4-nitro-1,3-benzothiazole sont mis en solution dans 50 ml de méthanol et 73 mg (10%) de palladium sur charbon sont ajoutés au mélange réactionnel qui est maintenu sous agitation sous 2,5 bars d'hydrogène pendant 18 heures. Le catalyseur est filtré, puis le solvant évaporé sous pression réduite. 248 mg (rendement = 40%) de 5-méthoxy-1,3-benzothiazol-4-amine sont obtenus et utilisés dans l'étape suivant sans autre purification. SM-CL : MH+ = 181,08 ; t.r. = 7,97 min.

### 25.3) 5-méthozy-1,3-benzothiazol-4,7-dione :

1,34 g (2,5 mmol ; 1,8 équivalent) de sel de Frémy (nitrosodisulfonate de potassium, contenant de 25 à 50% d'eau et de méthanol) en solution dans 40 ml d'une solution 0,3 *M* d'hydrogénophosphate de sodium, est ajouté à une solution de 248 mg (1,4 mmol) de 5-méthoxy-1,3-benzothiazole-4-amine dans 9 ml d'acétone. Le mélange réactionnel est maintenu sous agitation à température ambiante pendant 3 heures, puis concentré sous pression réduite. Le produit formé est extrait avec 3 fois 50 ml de dichlorométhane et la phase aqueuse est lavée avec 50 ml d'une solution d'eau saturée en chlorure de sodium. Les phases organiques sont réunies, séchées sur sulfate de sodium et le solvant est évaporé sous pression réduite. 248 mg (rendement = 91%) de 5-méthoxy-1,3-benzothiazol-4,7-dione sont obtenus et utilisés dans l'étape suivante sans autre purification.
SM-CL : MH+ = 196,05 ; t.r. = 7,65 min.

### 25.4) 5,5'-[(méthylimino)bis(proparce-3,1-diylimino)]bis(1,3-benzothiazole-4,7-dione) :

57 µl (0,37 mmol; 0,48 équivalent) de *N*-(3-arninopropyl)-*N*-méthylpropane-1,3-diamine sont ajoutés à 150 mg (0,77 mmol) de 5-méthoxy-1,3-benzothiazol-4,7-dione en solution dans 6 ml d'éthanol. Le mélange réactionnel est maintenu sous agitation à 85°C pendant 90 minutes, puis le solvant est évaporé sous pression réduite et le produit attendu est purifié par chromatographie sur colonne de silice (éluant : mélange dichlorométhane / méthanol 94 / 6) et 95 mg (rendement = 26%) de 5,5'-[(méthylimino)bis(propane-3,1-diylimino)]bis(1,3-benzothiazole-4,7-dione) sont obtenus sous forme d'une poudre rouge. Point de fusion : 223-224°C.
RMN ¹H (DMSO d6, 400 MHz, δ) : 9,28 (s, 2H); 8,02 (t, 2H, 2NH); 5,48 (s, 2H) ; 3,24-3,19 (m, 4H) ; 2,42-2,38 (m, 4H) ; 2,18 (s, 3H, CH₃); 1,78-1,74 (m, 4H).
SM-CL : MH+ = 472,19 ; t.r. = 7,32 min.

### Exemple 26: 5,5'-[(méthylimino)bis(propane-3,1-diylimino)]bis[2-(morpholin-4-ylcarbonyl)-1,3-benzothiazole-4,7-dione] :

### 26.1) Acide 5-méthozy-4-nitro-1,3-benzothiazole-2-carboxylique :

### 26.1.1) 5-méthoxy-4-nitro-1,3-benzothiazole-2-carbaldéhyde :

Le protocole expérimental est décrit dans l'étape 25.1.1 de l'exemple 25.

### 26.1.2) Acide 5-méthoxy-4-nitro-1,3-benzothiazole-2-carboxylique :

Le protocole expérimental est décrit dans l'étape 25.1.2 de l'exemple 25.

### 26.2) 5-méthoxy-2-(morpholin-4-ylcarbonyl)-1,3-benzothiazole-4,7-dione :

### 26.2.1) 5-méthoxy-2-(morpholin-4-ylcarbonyl)-4-nitro-1,3-benzothiazole :

A 500 mg (1,97 mmol) d'acide 5-méthoxy-4-nitro-1,3-benzothiazole-2-carboxylique et 344 µl (1,97 mmol; 1 éq.) de diisopropyléthylamine en solution dans 40 ml de dichlorométhane, sont ajoutés 980 mg (2,1 mmol ; 1,1 éq.) d'hexafluorophosphate de bromo-tris-pyrrolidino-phosphonium (PyBroP). Le mélange réactionnel est maintenu pendant 15 minutes sous agitation à température ambiante, puis 202 µl (2,3 mmol ; 1,2 éq.) de morpholine et une pointe de spatule de diméthylaminopyridine sont ajoutés au milieu qui est maintenu sous agitation pendant 18 heures à température ambiante. Un insoluble est ensuite filtré et le solvant évaporé sous pression réduite. Le résidu est ensuite purifié sur colonne de silice (éluant : acétate d'éthyle / heptane : gradient de 30 à 70% sur 40 minutes puis pendant 5 minutes à 70% d'acétate d'éthyle dans l'heptane) et 210 mg (rendement = 33%) de produit attendu sont obtenus sous forme de poudre beige.
SM-CL : MH+ = 324,01 ; t.r. = 9,63 min.

### 26.2.2) 5-méthoxy-2-(morpholin-4-ylcarbonyl)-1,3-benzothiazol-4-amine :

A 210 mg (0,65 mmol) de 5-méthoxy-2-(morpholin-4-ylcarbonyl)-4-nitro-1,3-benzothiazole en solution dans 10 ml de méthanol sont ajoutés 20 mg de palladium à 10% sur charbon activé. Le milieu réactionnel est ensuite placé sous agitation sous atmosphère d'hydrogène pendant 18 heures. Le catalyseur est ensuite filtré et le solvant évaporé. 173 mg du produit attendu (rendement brut = 91 %) sont obtenus sous forme d'une huile jaune et sont utilisés dans l'étape suivante sans autre purification.
SM-CL : MH+ = 294,04 ; t.r. = 9,09 min.

### 26.2.3) 5-méthoxy-2-(morpholin-4-ylcarbonyl)-1,3-benzothiazole-4,7-dione :

Une solution de 570 mg (1,063 mmol ; 1,8 éq.) de sel de Fremy (nitrosodisulfonate de potassium, contenant de 25 à 50% d'eau et de méthanol) dans une solution aqueuse à 0,3M d'hydrogénophosphate de sodium (12 ml) est ajoutée goutte à goutte à 173 mg (0,6 mmol) de 5-méthoxy-2-(morpholin-4-ylcarbonyl)-1,3-benzothiazol-4-amine en solution dans 5 ml d'acétone. Le mélange réactionnel est maintenu sous agitation à température ambiante pendant 4 heures, puis l'acétone est évaporée et le milieu est repris par 10 ml de dichlorométhane et lavé par 2 fois 7 ml d'une solution aqueuse saturée en chlorure de sodium. Les phases organiques sont réunies, séchées sur sulfate de magnésium et le solvant évaporé sous pression réduite. 175 mg (rendement brut = 99%) de produit attendu est obtenu sous forme d'une poudre jaune et est utilisé dans l'étape suivante sans autre purification.
SM-CL : MH+ = 308,99 ; t.r. = 8,40 min.

### 26.3) 5,5'-[(méthylimino)bis(propane-3,1-diylimino)]bis[2-(morpholin-4-ylcarbonyl)-1,3-benzothiazole-4,7-dione] :

51 µl (0,32 mmol ; 0,5 équivalent) de *N*-(3-aminopropyl)-N-méthylpropane-1,3-diamine sont ajoutés à 200 mg (0,65 mmol) de 5-méthoxy-2-(morpholin-4-ylcarbonyl)-1,3-benzothiazole-4,7-dione en solution dans 15 ml d'éthanol. Le mélange réactionnel est maintenu sous agitation à 85°C pendant 2 heures, puis le solvant est évaporé sous pression réduite et le produit attendu est purifié par chromatographie sur colonne de silice (éluant : mélange dichlorométhane / méthanol 95 / 5) puis recristallisé dans un mélange d'acétone et d'éther éthylique et 110 mg (rendement = 24%) de 5,5'-[(méthylimino)bis(propane-3,1-diylimino)]bis[2-(morpholin-4-ylcarbonyl)-1,3-benzothiazole-4,7-dione] sont obtenus sous forme d'une poudre rouge. Point de fusion = 207-208°C.
RMN ¹H (DMSO d6, 400 MHz, δ) : 8,17 (m, 2H, 2NH) ; 5,52 (s, 2H, 2CH) ; 4,25 (m, 4H); 3,69 (m, 12H) ; 3,25-3,20 (m, 4H) ; 2,42 (m, 4H) ; 2,20 (s, 3H, CH₃); 1,79-1,76 (m, 4H).
SM-CL : MH+ = 698,30 ; t.r. = 7,66 min.

*Les composés des exemples 27 à 37 sont obtenus de façon analogue à celle employée pour l'exemple 26, l'amine adéquate remplaçant la morpholine dans la troisième étape et le 6-méthoxy-2-métlryl-7-nitro-1,3-benzothiazole remplaçant le 5-méthoxy-2-méthyl-4-nitro-1,3-benzothiazole dans la première étape pour l'exemple 31.*

### Exemple 27 : 5,5'-[(méthylimino)bis(propane-3,1-diylimino)]bis(2-{[4-(morpholin-4-ylcarbonyl)pipérazin-1-yl]carbonyl}-1,3-benzothiazole-4,7-dione) :

Poudre rouge. Point de fusion = 157-158°C.
RMN ¹H (DMSO d6, 400 MHz, δ) : 8,19 (t, 2H, 2NH) ; 5,52 (s, 2H, 2CH) ; 4,24 (m, 4H); 3,68 (m, 4H) ; 3,57 (m, 10H) ; 3,25-3,22 (m, 4H) ; 3,21-3,20 (m, 4H) ; 3,18-3,16 (m, 10H); 3,44-3,41 (m, 4H) ; 2,20 (s, 3H, CH₃) ; 1,79-1,76 (m, 4H).
SM-CL : MH+ = 922,43 ; t.r. = 7,60 min.

### Exemple 28: 5,5'-[(méthylimino)bis(propane-3,1-diylimino)]bis(2-{[4-(tétrahydrofuran-2-ylcarbonyl)pipérazin-1-yl]carbonyl}-1,3-benzothiazole-4,7-dione) :

Poudre rouge. Point de fusion = 152-153°C.
RMN ¹H (DMSO d6, 400 MHz, δ) : 8,20 (t, 2H, 2NH) ; 5,52 (s, 2H, 2CH) ; 4,70 (t, 2H); 4,40-4,10 (m, 4H) ; 3,80-3,50 (m, 16H) ; 3,28-3,22 (m, 4H) ; 2,43-2,40 (m, 4H) ; 2,20 (s, 3H, CH₃) ; 2,03-2,01 (m, 4H) ; 1,84-1,76 (m, 8H).
SM-CL : MH+= 892,56 ; t.r. = 7,58 min.

### Exemple 29: 5,5'-[(méthylimino)bis(propane-3,1-diylimino)]bis(2-{[4-(méthylsulfonyl)pipérazin-1-yl]carbonyl}-1,3-benzothiazole-4,7-dione) :

Poudre rouge.
SM-CL : MH+ = 852,42 ; t.r. = 7,89 min.

### Exemple 30: 5,5'-[(méthylimino)bis(propane-3,1-diylimino)]bis[N-(4-méthoxyphenyl)-4,7-dioxo-4,7-dihydro-1,3-benzothiazole-2-carboxamide] : Poudre rouge.

SM-CL : MH+= 770,38 ; t.r. = 8,87 min.

### Exemple 31: 6,6'-[(méthylimino)bis(propane-3,1-diylimino)]bis[2-(pyrrolidin-1-ylcarbonyl)-1,3-benzothiazole-4,7-dione] :

Poudre rouge. Point de fusion = 237-238°C.
RMN ¹H (DMSO d6, 400 MHz, δ) : 7,91 (t, 2H, 2NH) ; 5,45 (s, 2H, 2CH) ; 3,98 (t, 4H); 3,54 (t, 4H) ; 3,20-3,18 (m, 4H) ; 2,40 (t, 4H) ; 2,18 (s, 3H, CH₃) ; 1,97-1,94 (m, 4H) ; 1,87-1,84 (m, 4H); 1,78-1,74 (m, 4H).
SM-CL : MH+= 666,32 ; t.r. = 7,91 min.

### Exemple 32: 5,5'-[(méthylimino)bis(propane-3,1-diylimino)]bis[N,(4-fluorophényl)-4,7-dioxo-4,7-dihydro-1,3-benzothiazole-2-carboxamide] :

Poudre rouge. Point de fusion = 262-263°C.
RMN ¹H (DMSO d6, 400 MHz, δ) : 11,00 (s, 2H, 2NH); 8,26 (t, 2H, 2NH) ; 7,85-7,82 (m, 4H); 7,20-7,15 (m, 4H); 5,56 (s, 2H, 2CH) ; 3,25-3,23 (m, 4H) ; 2,44-2,41 (m, 4H); 2,20 (s, 3H, CH₃) ; 1,81-1,78 (m, 4H).
SM-CL : MH+ = 746,27 ; t.r. = 9,15 min.

### Exemple 33: 5,5'-[(méthylimino)bis(propane-3,1-diylimino)]bis[N-(4-méthoxybenzyl)-4,7-dioxo-4,7-dihydro-1,3-benzothiazole-2-carboxamide]:

Poudre rouge. Point de fusion = 174-175°C.
RMN ¹H (DMSO d6, 400 MHz, δ) : 9,63 (t, 2H, 2NH); 8,18 (t, 2H, 2NH) ; 7,27-7,25 (d, 4H); 6,88-6,85 (d, 4H); 5,54 (s, 2H, 2CH) ; 4,37-4,35 (d, 4H) ; 3,71 (s, 6H, 2CH₃) ; 3,24-3,20 (m, 4H) ; 2,41-2,38 (m, 4H); 2,17 (s, 3H, CH₃) ; 1,77-1,74 (m, 4H).
SM-CL : MH+ = 798,46 ; t.r. = 8,89 min.

### Exemple 34 : 5,5'-[(méthylimino)bis(propane-3,1-diylimino)]bis{2-[(6-méthoxy-3,4-dihydroquinolin-1(2H-yl)carbonyl]-1,3-benzothiazole-4,7-dione} :

Poudre rouge. Point de fusion = 159-160°C.
RMN ¹H (DMSO d6, 400 MHz, δ) : 8,15 (m, 2H, 2NH) ; 6,79-6,68 (m, 6H); 5,52 (s, 2H, 2CH) ; 4,15-4,12 (m, 4H); 3,72 (s, 6H, 2CH₃); 3,24-3,20 (m, 4H) ; 2,81-2,77 (m, 4H); 2,42-2,39 (m, 4H); 2,19 (s, 3H, CH₃) ; 2,00-1,91 (m, 4H); 1,78-1,75 (m, 4H).
SM-CL : MH+ = 650,48 ; t.r. = 9,12 min.

### Exemple 35 : 5,5'-[(méthylimino)bis(propane-3,1-diylimino)]bis(N-cyclohexyl-4,7-dioxo-4,7-dihydro-1,3-benzothiazole-2-carboxamide)

Poudre rouge. Point de fusion = 145-146°C.
RMN ¹H (DMSO d6, 400 MHz, δ) : 8,90-8,88 (d, 2H, 2NH); 8,19 (t, 2H, 2NH) ; 5,54 (s, 2H, 2CH) ; 3,75-3,72 (m, 2H) ; 3,25-3,20 (m, 4H); 2,42-2,39 (m, 4H) ; 2,18 (s, 3H, CH₃) ; 1,78-1,71 (m, 12H) ; 1,61-1,07 (m, 12H).
SM-CL : MH+ = 722,51 ; t.r. = 9,14 min.

### Exemple 36: 5,5'-[(méthylimino)bis(propane-3,1-diylimino)]bis(2-{[4-(4-méthoxyberizoyl)pipérazin-1-yl]carbonyl}-1,3-benzothiazole-4,7-dione) :

Poudre rouge. Point de fusion = 202-203°C.
RMN ¹H (DMSO d6, 400 MHz, δ) : 8,18 (t, 2H, 2NH) ; 7,43-7,41 (m, 4H); 7,00-6,98 (m, 4H); 5,51 (s, 2H, 2CH) ; 4,29-4,47 (m, 4H) ; 3,79 (s, 6H, 2CH₃) ; 3,73-3,72 (m, 4H); 3,64-3,62 (m, 8H); 3,24-3,19 (m, 4H); 2,43-2,40 (m, 4H); 2,19 (s, 3H, CH₃) ; 1,79-1,75 (m, 4H).
SM-CL : MH+= 964,50 ; t.r. = 8,36 min.

### Exemple 37: 5,5'-[(méthylimino)bis(propane-3,1-diylimino)]bis(2-{[4-(2-furoyl)pipérazin-1-yl]carbonyl}-1,3-benzothiazole-4,7-dione) :

Poudre rouge. Point de fusion = 173-174°C.
RMN ¹H (DMSO d6, 400 MHz, δ) : 8,20 (t, 2H, 2NH) ; 7,85 (s, 2H); 7,06 (s, 2H); 6,64 (s, 2H); 5,53 (s, 2H, 2CH) ; 4,33-4,32 (m, 4H) ; 3,82-3,77 (m, 12H); 3,25-3,21 (m, 4H); 2,44-2,41 (m, 4H); 2,20 (s, 3H, CH₃) ; 1,79-1,76 (m, 4H).
SM-CL : MH+ = 884,37 ; t.r. = 8,04 min.

*Le composé de l'exemples 38 est obtenu de façon analogue à celle employée pour l'exemple 26, la 6-méthoxy-2-(2-naphtyl)-1,3-benzothiazol-4,7-dione remplaçant la 5-méthoxy-2-(morpholin-4-ylcarbonyl}-1,3-benzothiazol-4,7-dione dans la dernière étape.*

### Exemple 38: 6,6'-[(méthylimino)bis(propane-3,1-diylimino)]bis[2-(2-naphthyl)-1,3-benzothiazole-4,7-dione] :

Poudre rouge.
SM-CL : MH+ = 724,40 ; t.r. = 9,57 min.

### Exemple 39: 6,6'-[(méthylimino)bis(propane-3,1-diylimino)]bis[2-(1,3-benzodioxol-5-yl)-1,3-benzothiazole-4,7-dione) :

### 39.1) 2-(1,3-benzodioxol-5 yl)-6-méthoxy-1,3-benzothiazole-4,7-dione :

### 39.1.1) 2-bromo-6-méthoxy-1,3-benzothiazol-2-amine :

20 g (111 mmol) de 6-méthoxy-1,3-benzothiazol-2-amine sont mis en solution dans 400 ml d'acétonitrile, puis 13,2 ml (111 mmol ; 1 équivalent) de nitrite de tert-butyle et 29 g (130 mmol ; 1,2 équivalent) de CuBr₂ sont ajoutés au milieu réactionnel qui est ensuite maintenu sous agitation à 80 °C pendant 2 heures. Le solvant est évaporé sous pression réduite, puis le résidu est repris par 250 ml d'acétate d'éthyle et lavé 2 fois avec 200 ml d'eau. La phase organique est séchée sur sulfate de sodium, puis le solvant est évaporé sous pression réduite et 24 g (rendement = 89%) de 2-bromo-6-méthoxy-1,3-benzothiazol-2-amine sont obtenus et sont utilisés sans autre purification dans l'étape suivante.
SM-CL : MH+ = 243,98 ; t.r. = 10,89 min.

### 39.1.2) 2-bromo-6-méthoxy-7-nitro-1,3-benzothiazole :

24 g (100 mmol) de 2-bromo-6-méthoxy-1,3-benzothiazol-2-amine sont dissous dans 30 ml d'acide sulfurique à 0 °C, puis 30 ml d'acide nitrique (densité 1,41) sont ajoutés goutte à goutte. L'agitation est maintenue pendant 30 minutes à 0 °C puis 1 heure à température ambiante. Après neutralisation du mélange réactionnel par une solution de soude à 35% (13,5M), le produit formé est extrait par 3 fois 100 ml de dichlorométhane. La phase organique est séchée sur sulfate de sodium puis le solvant évaporé sous pression réduite et le solide ainsi obtenu est repris dans du dichlorométhane, filtré et lavé par un mélange dichlorométhane/heptane 1:1. Les eaux-mères sont purifiées par chromatographie sur colonne de silice (éluant : mélange acétate d'éthyle/heptane 1:1). 9,9 g (rendement = 35%) de 2-bromo-6-méthoxy-7-nitro-1,3-benzothiazole sont obtenus sous forme de poudre orangée.
SM-CL: MH+ = 288,75 ; t.r. = 10,70 min.

### 39.1.3) 2-(1,3-benzodioxol-5-yl)-6-méthoxy-7-nitro-1,3-benzothiazole :

A une suspension de 1,1 g (3,82 mmol) de 2-bromo-6-méthoxy-7-nitro-1,3-benzothiazole et 133 mg (0,115 mmol ; 0,03 équivalent) de tétrakis-triphénylphosphine de palladium dans 30 ml de 1,2-diméthoxyéthane sont ajoutés 0,697 g (4,2 mmol ; 1,1 équivalent) d'acide 1,3-benzodioxol-5-yl boronique ainsi qu'une solution de 1,2 g (11,35 mmol ; 3 équivalents) de carbonate de sodium dans 15 ml d'eau. Le mélange réactionnel est maintenu sous agitation à 90 °C pendant 4 heures, puis après concentration sous pression réduite, 100 ml d'acétate d'éthyle sont ajoutés au milieu qui est ensuite lavé avec 2 fois 75 ml d'une solution d'eau saturée en chlorure de sodium. La phase organique est séchée sur sulfate de sodium puis les solvants sont évaporés sous pression réduite et le résidu est purifié par chromatographie sur colonne de silice (éluant : mélange acétate d'éthyle/heptane 1:2). 0,960 g (rendement de 76%) de 2-(1,3-benzodioxol-5-yl)-6-méthoxy-7-nitro-1,3-benzothiazole sont obtenus sous forme de poudre beige.
SM-CL : MH+ = 331,06 ; t.r. = 11,38 min.

### 39.1.4) 2-(1,3-benzodioxol-5-yl)- 6-méthoxy-1,3-benzothiazole-7-amine:

0,96 g (2,9 mmol) de 2-(1,3-benzodioxol-5-yl)-6-méthoxy-7-nitro-1,3-benzothiazole sont mis en suspension dans 100 ml de méthanol. 96 mg (10%) de palladium sur charbon sont ajoutés au mélange réactionnel qui est maintenu sous agitation sous 2,5 bars d'hydrogène pendant 18 heures. Le catalyseur est filtré, puis les solvants évaporés sous pression réduite. 0,45 g (rendement = 52%) de 2-(1,3-benzodioxol-5-yl)-6-méthoxy-1,3-benzothiazol-7-amine est obtenu et utilisé dans l'étape suivante sans autre purification.
SM-CL: MH+ = 301,10 ; t.r. = 10,48 min.

### 39.1.5) 2-(1,3-benzodioxol-5-yl)-6-méthozy-1,3-benzothiazol-4, 7-dione :

1,45 g (2,7 mmol ; 1,8 équivalent) de sel de Frémy dissous dans 45 ml d'une solution 0,3 M d'hydrogénophosphate de sodium est ajouté à 0,45 g (1,5 mmol) de 2-(1,3-benzodioxol-5-yl)-6-méthoxy-1,3-benzothiazol-7-amine en solution dans 15 ml d'acétone. Le mélange réactionnel est maintenu sous agitation à température ambiante pendant 5 heures puis concentré sous pression réduite. Le produit formé est extrait avec 3 fois 50 ml de dichlorométhane et la phase aqueuse est lavée avec 50 ml d'une solution d'eau saturée en chlorure de sodium. Les phases organiques sont réunies, séchées sur sulfate de sodium et le solvant évaporé sous pression réduite.Le produit attendu est purifié par chromatographie sur colonne de silice (éluant : mélange dichlorométhane/méthanol 95:5).0,2 g (rendement = 43%) de 2-(1,3-benzodioxol-5-yl)-6-méthoxy-1,3-benzothiazol-4,7-dione est obtenu sous forme d'une poudre jaune.
SM-CL : MH+ = 316,07 ; t.r. = 10,14 min.

### 39.2) 6,6'-[(méthylimino)bis(propane-3,1-diylimino)]bis[2-(1,3-benzodioxol-5-yl)-1,3-benzothiazole-4,7-dione] :

Le protocole expérimental est le même que celui décrit pour l'exemple 26, la 2-(1,3-benzodioxol-5-yl)-6-méthoxy-1,3-benzothiazol-4,7-dione remplaçant la 5-méthoxy-2-(morpholin-4-ylcarbonyl)-1,3-benzothiazol-4,7-dione dans la dernière étape.

Poudre rouge.
SM-CL : MH+ = 712,45 ; t.r. = 8,57 min.

*Le composé de l'exemple 40 est obtenu de façon analogue à celle employée pour l'exemple 1, la 2-(4-éthylphényl)-6-méthoxy-1,3-benzoxazole-4,7-dione remplaçant le 5-méthoxy-2-méthyl-4,7-dioxobenzothiazole.*

### Exemple 40 : 6,6'-[(méthylimino)bis(propane-3,1-diylimino)]bis[2-(4-éthylphényl)-1,3-benzoxazole-4,7-dione] :

Poudre rouge. Point de fusion = 216-217°C.
RMN ¹H (CF₃COOD, 400 MHz, δ) : 7,88-7,86 (d, 4H); 7,24-7,22 (d, 4H); 3,33=3,31 (m, 6H); 3,19-3,14 (m, 2H); 2,83 (s, 3H, CH₃); 2,57-2,51 (q, 4H, 2CH₂); 2,13-2,09 (m, 4H); 1,06-1,02 (t, 6H, 2CH₃).
SM-CL : MH+ = 648,35 ; t.r. = 8,81 min.

### Exemple 41 : N-(4-méthoxYphényl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide :

### 41.1) 5-({3-[(3-aminopropyl)(méthyl)amino]propyl}amino)-2-méthyl-1,3-benzothiazole-4,7-dione :

1,6 g (7,65 mmol) de 5-méthoxy-2-méthyl-1,3-benzothiazole-4,7-dione sont mis en solution dans 250 ml de dichlorométhane. 6,17 ml (38,3 mmol ; 5 équivalents) de *N*-(3-aminopropyl)-*N*-méthylpropane-1,3-diamine sont ajoutés et le mélange réactionnel est maintenu pendant 2 heures sous agitation à température ambiante, puis lavé par 3 fois 100 ml d'eau. Les phases organiques sont regroupées, séchées sur sulfate de sodium et le solvant est évaporé sous pression réduite.
Le produit souhaité est obtenu sous forme d'une huile rouge et est utilisé dans l'étape suivante sans autre purification.

RMN ¹H (DMSO d6, 400 MHz, δ) : 5,45 (s, 1H); 3,21-3,17 (t, 2H); 2,75 (s, 3H, CH₃); 2,72-2,61 (m, 2H); 2,37-2,30 (m, 4H); 2,13 (s, 3H, CH₃); 1,73-1,69 (m, 2H); 1,55-1,52 (m, 2H).
SM-CL : MH+ = 323,21 ; t.r. = 6,96 min.

### 41.2) 5-méthowy-N-(4-méthoxyphényl)-4,7-dioxo-4,7-dihydro-1,3-benzothiazole-2-carboxamide :

Le protocole expérimental est le même que celui décrit pour l'étape 26.2.1, la 4-(méthoxyphényl)amine remplaçant la morpholine.
RMN ¹H (DMSO d6, 400 MHz, δ) : 11,03 (s, 1H, NH); 7,79-7,75 (d, 2H); 6,97-6,93 (d, 2H); 6,37 (s, 1H); 3,90 (s, 3H, CH₃); 3,75 (s, 3H, CH₃).
SM-CL : MH+ = 345,08 ; t.r. = 10,07 min.

### 41.3) N-(4-méthozyphényl)-5-{(3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide :

La 5-({3-[(3-aminopropyl)(méthyl)amino]propyl}amino)-2-méthyl-1,3-benzothiazole-4,7-dione est mise en solution dans 250 ml d'éthanol et 2,12 g (6,16 mmol; 0,8 équivalent) de 5-méthoxy-*N*-(4-méthoxyphényl)-4,7-dioxo-4,7-dihydro-1,3-benzothiazole-2-carboxamide sont ajoutés. Le mélange réactionnel est maintenu sous agitation à 60°C pendant 4 heures, puis le solvant est évaporé sous pression réduite. Le résidu est ensuite purifié par chromatographie sur colonne de silice (éluant: dichlorométhane / méthanol: 95 / 5) et 450 mg (rendement = 9%) de *N*-(4-méthoxyphényl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide sont obtenus sous forme de poudre rouge.
Point de fusion = 141-142°C.
RMN ¹H (DMSO d6, 400 MHz, δ) : 10,87 (s, 1H, NH) ; 8,26 (t, 1H, NH); 7,92 (t, 1H, NH); 7,78-7,77 (d, 2H); 6,97-6,95 (d, 2H); 5,56 (s, 1H); 5,39 (s, 1H) ; 3,76 (s, 3H, CH₃); 3,25-3,15 (2m, 4H) ; 2,67 (s, 3H, CH₃) ; 2,43-2,38 (m, 4H) ; 2,18 (s, 3H, CH₃); 1,79-1,75 (m, 4H).
SM-CL : MH+ = 635,21 ; t.r. = 8,31 min.

### Passage au méthanesulfonate :

Le protocole expérimental fait appel à des méthodes connues de l'homme de l'art.
Le composé salifié précipite sous forme d'une poudre rouge.

*Les composés des exemples 42 à 48 sont obtenus de façon analogue à celle employée pour l'exemple 41, la 6-méthoxy-2-méthyl-1,3-benzothiazole-4,7-dione remplaçant la 5-méthoxy-2-méthyl-1,3-benzothiazole-4,7-dione dans la première étape pour l'exemple 42 et l'amine adéquate remplaçant la 4-(méthoxyphényl)amine dans la deuxième étape.*

### Exemple 42 : 5-{[3-(méthyl{3-[(2-méthyl-4,7-dioxo-4,7-dihydro-1,3-benzothiazol-6-yl)amino]propyl}amino)propyl]amino}-2-{[4-(tétrahydrofuran-2-ylcarbonyl)pipérazin-1-yl]carbonyl}-1,3-benzothiazole-4,7-dione :

Poudre rouge. Point de fusion = 121-122°C.

RMN ¹H (DMSO d6, 400 MHz, δ) : 8,24 (m, 1H, NH); 7,78 (t, 1H, NH); 5,53 (s, 1H); 5,31 (s, 1H) ; 4,70 (t, 1H) ; 4,18-4,35 (m, 2H) ; 3,55-3,80 (m, 8H) ; 3,24-3,16 (2m, 4H) ; 3,03-2,99 (m, 4H) ; 2,75 (s, 3H, CH₃) ; 2,42-2,40 (m, 4H) ; 2,18 (s, 3H, CH₃); 2,08-1,98 (m, 2H) ; 1,78-1,76 (m, 2H).
SM-CL : MH+ = 696,40 ; t.r. = 7,49 min.

### Exemple 43 : N-éthyl-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide :

Poudre rouge. Point de fusion = 107-108°C. ;

RMN ¹H (DMSO d6, 400 MHz, δ) : 9,16 (t, 1H, NH); 8,17 (t, 1H, NH) ; 7,93 (t, 1H, NH); 5,53 (s, 1H); 5,40 (s, 1H) ; 3,22-3,19 (m, 4H) ; 3,01-2,99 (m, 2H) ; 2,71 (s, 3H, CH₃) ; 2,42-2,40 (m, 2H) ; 2,18 (s, 3H, CH₃); 1,77-1,72 (m, 6H) ; 1,13 (t, 3H, CH₃). SM-CL : MH+ = 557,15 ; t.r. = 7,68 min.

### Exemple 44: 5-({3-[(3-{[4,7-dioxo-2-(pyrrolidin-1-ylcarbonyl)-4,7-dihydro-1,3-benzothiazol-6-yl]amino}propyl)(méthyl)amino]propyl}amino)-2-méthyl-1,3-benzothiazole-4,7-dione :

Poudre rouge.

RMN ¹H (DMSO d6, 400 MHz, δ): 7,92 (t, 1H, NH) ; 7,75 (t, 1H, NH); 5,48 (s, 1H); 5,39 (s, 1H) ; 4,00 (t, 2H) ; 3,55 (t, 2H) ; 3,21-3,15 (m, 4H) ; 2,71 (s, 3H, CH₃) ; 2,42-2,40 (m, 4H) ; 2,18 (s, 3H, CH₃); 1,98-1,93 (m, 2H) ; 1,90-1,84 (m, 2H) ; 1,78-1,75 (m, 4H).
SM-CL : MH+ = 583,27 ; t.r. = 7,74 min.

### Exemple 45 : N-(4-méthoxybenzyl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide :

Poudre rouge.

RMN ¹H (DMSO d6, 400 MHz, δ) : 9,65 (t, 1H, NH); 8,17 (t, 1H, NH) ; 7,94 (t, 1H, NH); 7,28-7,26 (d, 2H) ; 6,89-6,87 (d, 2H) ; 5,53 (s, 1H); 5,40 (s, 1H) ; 4,38-4,37 (d, 2H, CH₂); 3,72 (s, 3H, CH₃) ; 3,22-3,18 (m, 4H) ; 3,01-2,99 (m, 2H) ; 2,69 (s, 3H, CH₃) ; 2,42-2,40 (m, 2H) ; 2,18 (s, 3H, CH₃); 1,77-1,72 (m, 6H).
SM-CL : MH+ = 649,31 ; t.r. = 8,28 min.

### Exemple 46 : N-1,3-benzodioxol-5-yl-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide :

Poudre rouge.
SM-CL : MH+= 649,36 ; t.r. = 8,42 min.

### Exemple 47 : 2-[(6-méthoxy-3,4-dihydroquinolin-1(2H)-yl)carbonyl]-5-{[3-(méthyl{3-[(2-méthyl-4,7-dioxo-4,7-dihydro-1,3-benzothiazol-5-yl)amino]propyl}amino)propyl]amino}-1,3-benzothiazole-4,7-dione :

Poudre rouge.
SM-CL : MH+= 675,33 ; t.r. = 8,40 min.

### Exemple 48: 2-{[4-(4-méthoxybenzoyl)pipérazin-1-yl]carbonyl}-5-{[3-(méthyl{3-[(2-méthyl-4,7-dioxo-4,7-dihydro-1,3-benzothiazol-5-yl)amino]propyl}amino)propyl]amino}-1,3-benzothiazole-4,7-dione :

Poudre rouge.

RMN ¹H (DMSO d6, 400 MHz, δ) : 8,26 (t, 1H, NH) ; 7,89 (t, 1H, NH); 7,44-7,42 (d, 2H) ; 7,01-6,99 (d, 2H) ; 5,52 (s, 1H); 5,36 (s, 1H) ; 4,28-4,27 (m, 2H) ; 3,80 (s, 3H, CH₃) ; 3,74-3,64 (m, 4H) ; 3,22-3,14 (m, 6H) ; 2,69 (s, 3H, CH₃); 2,44-2,40 (m, 4H) ; 2,19 (s, 3H, CH₃) ; 1,78-1,75 (m, 4H).
SM-CL : MH+ = 732,32 ; t.r. = 8,01 min.

### Exemple 49 : 5-({3-[(3-{[2-(4-méthoxyphenyl)-4,7-dioxo-4,7-dihydro-1,3-benzothiazol-6-yl]amino}propyl)(méthyl)amino]propyl}amino)-2-méthyl-1,3-benzothiazole-4,7-dione :

### 49.1) 6-méthoxy-2-(4-méthoxyphényl)-1,3-benzothiazole-4,7-dione:

Le protocole expérimental est le même que celui décrit pour l'intermédiaire 39.1, l'acide 4-méthoxyphényl boronique remplaçant l'acide 1,3-benzodioxol-5-yl boronique dans la troisième étape.
SM-CL : MH+ = 302,10 ; t.r. = 10,29 min.

### 49.2) 5-({3-[(3-{(2-(4-méthoxyphenyl)-4,7-dioxo-4,7-dihydro-1,3-benzothiazol-6-yl]amino}propyl)(méthyl)amino]propyl}amino)-2-méthyl-1,3-benzothiazole-4,7-dione :

Le protocole expérimental est le même que celui décrit pour l'exemple 41, la 6-méthoxy-2-(4-méthoxyphényl)-1,3-benzothiazole-4,7-dione remplaçant le 5-méthoxy-*N*-(4-méthoxyphényl)-4,7-dioxo-4,7-dihydro-1,3-benzothiazole-2-carboxamide dans la dernière étape.
Poudre rouge.
SM-CL : MH+ = 592,35 ; t.r. = 8,25 min.

### Exemple 50 : 5-({3-[{3-[(4,7-dioxo-4,7-dihydro-1,3-benzothiazol-5-yl)amino]propyl}(méthyl)amino]propyl}amino)-2-méthyl-1,3-benzothiazole-4,7-dione :

Le protocole expérimental est le même que celui décrit pour l'exemple 41, la 5-méthoxy-1,3-benzothiazol-4,7-dione remplaçant le 5-méthoxy-*N*-(4-méthoxyphényl)-4,7-dioxo-4,7-dihydro-1,3-benzothiazole-2-carboxamide dans la dernière étape.
Poudre rouge.

RMN ¹H (DMSO d6, 400 MHz, δ) : 9,28 (s, 1H); 8,02 (t, 1H, NH); 7,95 (t, 1H, NH); 5,46 (s, 1H) ; 5,41 (s, 1H) ; 3,21-3,18 (m, 4H) ; 2,72 (s, 3H, CH₃); 2,41-2,38 (m, 4H) ; 2,18 (s, 3H, CH₃); 1,77-1,74 (m, 4H).
SM-CL : MH+ = 486,23 ; t.r. = 7,45 min.

### Exemple 51: 2-(2,5-difluorophényl)-6-{[3-(méthyl{3-[(2-méthyl-4,7-dioxo-4,7-dihydro-1,3-benzothiazol-5-yl)amino]propyl}amino)propyl]amino}-1,3-benzoxazole-4,7-dione:

Le protocole expérimental est le même que celui décrit pour l'exemple 41, la 2-(2,5-difluorophényl)-6-méthoxy-1,3-benzoxazole-4,7-dione remplaçant le 5-méthoxy-N-(4-méthoxyphényl)-4,7-dioxo-4,7-dihydro-1,3-benzothiazole-2-carboxamide dans la dernière étape.
Point de fusion = 149-150°C.

RMN ¹H (DMSO d6, 400 MHz, δ) : 8,03 (t, 1H, NH); 7,93-7,88 (m, 2H); 7,61-7,58 (m, 2H); 5,39 (s, 1H) ; 5,33 (s, 1H); 3,21-3,15 (m, 4H); 2,68 (s, 3H, CH₃); 2,43-2,38 (m, 4H) ; 2,18 (s, 3H, CH₃); 1,78-1,75 (m, 4H).
SM-CL : MH+ = 582,22 ; t.r. = 8,14 min.

### Passage au méthanesulfonate :

0,378 g (0,65 mmol) de 2-(2,5-difluorophényl)-6-{[3-(méthyl{3-[(2-méthyl-4,7-dioxo-4,7-dihydro-1,3-benzothiazol-5-yl)amino]propyl amino)propyl]amino} -1,3-benzoxazole-4,7-dione sont mis en solution dans 50 ml d'acétone et 46,4 µl (0,7 mmol ; 1,1 équivalent) d'acide méthanesulfonique sont ajoutés goutte à goutte. Le méthanesulfonate précipite sous forme d'une poudre rouge. Point de fusion = 99-100°C.

RMN ¹H (D₂O, 400 MHz, δ) : 7,58 (m, 1H); 7,47 (m, 1H); 7,37-7,31 (m, 1H); 5,21 (s, 1H) ; 5,08 (s, 1H); 3,35-3,21 (m, 8H); 2,91 (s, 3H, CH₃); 2,74 (s, 3H, CH₃); 2,51 (s, 3H, CH₃); 2,09-2,06 (m, 4H).
SM-CL : MH+ = 582,34 ; t.r. = 8,45 min.

### Exemple 52: 2-(4-éthylphényl)-6-{[3-(méthyl{3-[(2-méthyl-4,7-dioxo-4,7-dihydro-1,3-benzothiazol-5-yl)amino]propyl}amino)propyl]amino}-1,3-benzoxazole-4,7-dione :

Le protocole expérimental est le même que celui décrit pour l'exemple 41, la 2-(4-éthylphényl)-6-méthoxy-1,3-benzoxazole-4,7-dione remplaçant le 5-méthoxy-N-(4-méthoxyphényl)-4,7-dioxo-4,7-dihydro-1,3-benzothiazole-2-carboxamide dans la dernière étape.

RMN ¹H (DMSO d6, 400 MHz, δ) : 8,03-8,01 (d, 2H); 7,90-8,00 (2m, 2H, 2NH); 7,48-7,46 (d, 2H); 5,40 (s, 1H) ; 5,28 (s, 1H); 3,20-3,17 (m, 4H); 2,73-2,68 (q, 2H); 2,65 (s, 3H, CH₃); 2,42-2,40 (m, 4H); 2,19 (s, 3H, CH₃); 1,78-1,75 (m, 4H); 1,24 (t, 3H, CH₃).
SM-CL : MH+ = 574,24 ; t.r. = 8,40 min.

### Exemple 53: 2-(2,5-difluorophényl)-6-{[3-(méthyl{3-[(2-méthyl-4,7-dioxo-4,7-dihydro-1,3-benzothiazol-6-yl)amino]propyl}amino)propyl]amino)-1,3-benzoxazole-4,7-dione :

Le protocole expérimental est le même que celui décrit pour l'exemple 41, la 6-méthoxy-2-méthyl-1,3-benzothiazole-4,7-dione remplaçant la 5-méthoxy-2-méthyl-1,3-benzothiazole-4,7-dione dans la première étape et la 2-(2,5-difluorophényl)-6-méthoxy-1,3-benzoxazole-4,7-dione remplaçant le 5-méthoxy-N-(4-méthoxyphényl)-4,7-dioxo-4,7-dihydro-1,3-benzothiazole-2-carboxamide dans la dernière étape.
Poudre rouge.

RMN ¹H (DMSO d6, 400 MHz, δ) : 8,03 (t, 1H, NH) ; 7,91 (m, 1H); 7,80 (t, 1H, NH); 7,61-7,59 (m, 2H); 5,34 (s, 1H); 5,33 (s, 1H) ; 3,21-3,17 (m, 4H) ; 2,72 (s, 3H, CH₃) ; 2,42-2,38 (m, 4H) ; 2,18 (s, 3H, CH₃); 1,78-1,74 (m, 4H).
SM-CL : MH+ = 582,31 ; t.r. = 8,07 min.

### Exemple 54: 5,5'-[[(4-méthoxybenzyl)imino]bis(propane-3,1-diylimino)]bis(2-méthyl-1,3-benzothiazole-4,7-dione):

### 54.1) 5-[(3-bromopropyl)amino]-2-méthyl-1,3-benzothiazole-4,7-dione:

A 1 g (4,78 mmol) de 5-méthoxy-2-méthyl-1,3-benzothiazole-4,7-dione en solution dans 100 ml de méthanol, sont ajoutés 1,57 g (7,17 mmol ; 1,5 équivalent) de bromhydrate de 3-bromopropylamine et 1,02 ml (7,17 mmol ; 1,5 équivalent) de triéthylamine. Le mélange réactionnel est maintenu pendant 2 heures sous agitation à 60°C, puis le solvant est évaporé sous pression réduite et le résidu est purifié par chromatographie sur colonne de silice (éluant : dichlorométhane / méthanol : 97 / 3). La 5-[(3-bromopropyl)amino]-2-méthyl-1,3-benzothiazole-4,7-dione est obtenue sous forme d'une huile rouge.
SM-CL : MH+ = 314,99 ; t.r. = 9,13 min.

### 54.2) 5-({3-[(4-méthoxybenzyl)amino]propyl}amino)-2-méthyl-1,3-benzothiazole-4,7-dione:

A 0,35 g (1,11 mmol) de 5-[(3-bromopropyl)amino]-2-méthyl-1,3-benzothiazole-4,7-dione en solution dans 15 ml de diméthylformamide, sont ajoutés 160 µl (1,22 mmol ; 1,1 équivalent) de 4-méthoxybenzylamine, 213 µl (1,22 mmol ; 1,1 équivalent) de diisopropyléthylamine et une pointe de spatule d'iodure de sodium. Le mélange réactionnel est maintenu sous agitation sous micro-ondes pendant 5 minutes à 180°C. Puis le solvant est évaporé sous pression réduite et le résidu réactionnel est repris par du dichlorométhane, lavé par 3 fois 50 ml d'eau. Les phases organiques sont regroupées, séchées sur sulfate de magnésium et le solvant est évaporé sous pression réduite. Le produit attendu est purifié par chromatographie sur colonne de silice (éluant : dichlorométhane / méthanol : 95 / 5) et obtenu sous forme d'une huile rouge.
SM-CL : MH+ = 372,15 ; t.r. = 7,64 min.

### 54.3) 5,5'-[[(4-méthoxybenzyl)imino]bis(propane-3,1-diylimino)] bis(2-méthyl-1, 3-benzothiazole-4,7-dione):

A 23,6 mg (64 µmol) de 5-({3-[(4-méthoxybenzyl)amino]propyl}amino)-2-méthyl-1,3-benzothiazole-4,7-dione dissous dans 5 ml d'acétonitrile, sont ajoutés 22 µl (128 µmol ; 2 équivalents) de diisopropyléthylamine, 21 mg (64 µmol; 1 équivalent) de 5-[(3-bromopropyl)amino]-2-méthyl-1,3-benzothiazole-4,7-dione et une pointe de spatule d'iodure de sodium. Le mélange réactionnel est maintenu sous agitation sous microondes à 165°C pendant 15 minutes, puis le solvant est évaporé sous pression réduite et le résidu réactionnel est repris par du dichlorométhane, lavé par 3 fois 50 ml d'eau. Les phases organiques sont regroupées, séchées sur sulfate de magnésium et le solvant est évaporé sous pression réduite. Le produit attendu est purifié par chromatographie sur colonne de silice (éluant : dichlorométhane / méthanol : 93 / 7) et obtenu sous forme d'une huile rouge.
SM-CL : MH+ = 606,23 ; t.r. = 7,95 min.

### Exemple 55 : 5,5'-[(méthylimino)bis(butane-4,1-diylimino)]bis(2-méthyl-1,3-benzothiazole-4,7-dione) :

### 55.1) tert-buyl[4-(1,3-dioxo-1, 3-dihydro-2H-isoindol-2 yl)butyl]méthylcarbamate :

2 g (9,9 mmol) de *tert*-butyl(4-aminobutyl)méthylcarbamate et 1,46 g (9,9 mmol ; 1 équivalent) de 2-benzofuran-1,3-dione sont mis en solution dans 50 ml de toluène et le mélange réactionnel est maintenu au reflux pendant 24 heures dans un Dean-Stark. Le solvant est évaporé sous pression réduite et le produit attendu est purifié sur colonne de silice (éluant : dichlorométhane / méthanol : 90 / 10).
SM-CL : MH+ = 333,26 ; t.r. = 10,90 min.

### 55.2) 2-[4-(méthylamino)butyl)-1H-isoindole-1,3(2H)-dione :

0,6 g (1,8 mmol) de *tert*-butyl[4-(1,3-dioxo-1,3-dihydro-2*H*-isoindol-2-yl)butyl]méthylcarbamate sont mis en solution dans 865 ml (10,8 mmol ; 6 équivalents) d'acide trifluorométhanesulfonique et le mélange réactionnel est maintenu sous agitation 2 heures à température ambiante. Les solvants sont évaporés, puis l'excès d'acide est coévaporé avec 3 fois 50 ml de dichlorométhane. Le produit attendu est utilisé sans autre purification dans l'étape suivante.
SM-CL : MH+ = 233,19 ; t.r. = 7,38 min.

### 55.3) 2,2'-[(méthylimino)dibutane-4,1-diyl]bis (1H-isoindole-1,3(2H)-dione) :

209 mg (0,9 mmol) de 2-[4-(méthylamino)butyl]-1*H*-isoindole-1,3(2*H*)-dione sont mis en solution dans 5 ml de tétrahydrofurane, puis 385 µl (2,7 mmol ; 3 équivalents) de triéthylamine et 254 mg (0,9 mmol ; 1 équivalent) de 2-(4-bromobutyl)-1*H*-isoindole-1,3(2*H*)-dione sont ajoutés au mélange réactionnel qui est maintenu pendant 25 minutes sous microondes à 165°C. Le précipité obtenu est filté et le filtrat concentré sous pression réduite. Le produit attendu est purifié par chromatographie sur colonne de silice (éluant : dichlorométhane / méthanol : 95 / 5).
SM-CL : MH+ = 434,25 ; t.r. = 8,37 min.

### 55.4) N-(4-aminobutyl)-N-méthylbutane-1,4-diamine :

144 mg (0,33 mmol) de 2,2'-[(méthylimino)dibutane-4,1-diyl]bis (1*H*-isoindole-1,3(2*H*)-dione) sont mis en solution dans 2 ml d'éthanol et 65 µl (0,73 mmol ; 2,2 équivalents) d'hydrate d'hydrazine 35% sont ajoutés au mélange réactionnel qui est maintenu sous agitation à 150°C sous micro-ondes pendant 30 minutes. Les solvants sont évaporés sous pression réduite et l'excès d'hydrate d'hydrazine est éliminé par co-évaporation avec 4 fois 15 ml d'éthanol. 100 ml d'éthanol sont ajoutés au mélange réactionnel qui est acidifié jusqu'à pH 1 par une solution 1N d'acide chlorhydrique dans l'éther éthylique. Le milieu réactionnel est concentré sous pression réduite, puis repris avec 50 ml d'éthanol et le précipité blanc formé est filtré, lavé par 3 fois 20 ml d'eau et éliminé. Le filtrat est basifié jusqu'à pH 12 avec une solution de soude 2M, puis concentré sous pression réduite et utilisé dans l'étape suivant sans autre purification.

RMN ¹H (DMSO d6, 400 MHz, δ) :3,13-3,10 (m, 4H); 2,24-2,20 (m, 4H); 2,08 (s, 3H, CH₃); 1,38-1,34 (m, 4H).
SM-CL : MH+ = 174,29 ; t.r. = 8,99 min.

### 55.5) 5,5'-[(méthylimino)bis(butane-4,1-diylimino)]bis(2-méthyl-1,3-benzothiazole-4,7-dione) :

6 mg (34 µmol) de *N*-(4-aminobutyl)-*N*-méthylbutane-1,4-diamine sont ajoutés à une solution de 15 mg (71 mmol; 2,1 équivalents) de 5-méthoxy-2-méthyl-1,3-benzothiazole-4,7-dione dans 1,5 ml d'éthanol, puis le mélange réactionnel est agité à 60°C pendant 1 heure. Le solvant est ensuite évaporé sous pression réduite et le produit attendu est obtenu par purification par chromatographie sur colonne de silice (éluant : dichlorométhane / méthanol : 95 / 5) sous forme d'une huile rouge.
SM-CL : MH+ = 527,69 ; t.r. = 10,13 min.

### Exemple 56: 2-méthyl-5-{[3-(méthyl{4-[(2-méthyl-4,7-dioxo-4,7-dihydro-1,3-benzothiazol-5-yl)amino]butyl}amino)propyl]amino}-1,3-benzothiazole-4,7-dione :

### 56.1) 2-{3-[[4-(1,3-dioxo-1,3-dihydro-2H-isoindol-2-yl)butyl](méthyl)amino]propyl}-1H-isoindole-1,3(2H)-dione :

Le protocole expérimental est le même que celui décrit pour l'exemple 55.3, la 2-(3-bromopropyl)-1*H*-isoindole-1,3(2*H*-dione remplaçant la 2-(4-bromobutyl)-1H-isoindole-1,3(2*H*)-dione.
SM-CL : MH+ = 420,24 ; t.r. = 8,19 min.

### 56.2) N-(3-aminopropyl)-N-méthylbutane-1,4-diamine :

Le protocole expérimental est le même que celui décrit pour l'exemple 55.4.

### 56.3) 2-méthyl-5-{[3-(méthyl{4-[(2-méthyl-4,7-dioxo-4,7-dihydro-1,3-benzothiazol-5-yl)amino]butyl}amino)propyl]amino}-1,3-benzothiazole-4,7-dione :

Le protocole expérimental est le même que celui décrit pour l'exemple 55.5, la N-(3-aminopropyl)-*N*-méthylbutane-1,4-diamine remplaçant la *N*-(4-aminobutyl)-*N-*méthylbutane-1,4-diamine.

### Etude pharmacologique des composés de l'invention

### Protocoles des tests

### i) Mesure de l'activité phosphatase de l'enzyme recombinante Cdc25C purifiée

L'activité phosphatase de la protéine MBP-Cdc25C est évaluée par la déphosphorylation du 3-O-méthylfluorescéine-phosphate (OMFP) en 3-O-méthylfluorescéine (OMF) avec une détermination de la fluorescence à 475 nm du produit de la réaction. Cet essai permet d'identifier des inhibiteurs de l'enzyme recombinante cdc25. La préparation de la protéine de fusion MBP-Cdc25C est décrite dans la demande de brevet WO 01/44467.

La réaction est réalisée en format de plaque 384 puits sous un volume final de 50 µl. La protéine MBP-Cdc25C (préparée comme décrit ci-dessus) est conservée dans le tampon d'élution suivant : 20 mM Tris-HCl pH 7,4 ; 250 mM NaCl ; 1mM EDTA ; 1 mM de dithiothréitol (DTT) ; 10 mM maltose. Elle est diluée à la concentration de 60 µM dans le tampon de réaction suivant : 50 mM Tris-HCl pH 8,2 ; 50 mM NaCl ; 1 mM DTT ; 20% glycérol. La mesure du bruit de fond est effectuée avec le tampon sans addition de l'enzyme. Les produits sont testés à des concentrations décroissantes à partir de 40 µM. La réaction est initiée par l'ajout d'une solution OMFP à 500 µM finale (préparée extemporanément à partir d'une solution stock 12,5 mM dans du DMSO 100% (Sigma #M2629)). Après 4 heures à 30 °C dans une plaque 384 puits à usage unique, la fluorescence mesurée à DO 475 nm est lue à l'aide d'un lecteur de plaque Victor² (EGG-Wallac). La détermination de la concentration inhibant de 50% la réaction enzymatique est calculée à partir de trois expériences indépendantes. Seules les valeurs contenues dans la partie linéaire de la sigmoïde sont retenues pour l'analyse de régression linéaire.

### ii) Caractérisation de l'activité anti-proliférative :

A titre d'exemple, on étudiera l'effet d'un traitement sur deux lignées de cellules humaines Mia-Paca2 et DU145 par les composés des exemples décrits précédemment. Les lignées cellulaires DU145 (cellules humaines de cancer de la prostate) et Mia-PaCa2 (cellules humaines de cancer du pancréas) ont été acquises auprès de American Tissue Culture Collection (Rockville, Maryland, USA). Les cellules placées dans 80 µl de milieu Eagle modifié de Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 10 µM. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅₀. Les produits sont solubilisés dans le diméthylsulfoxide (DMSO) à 10⁻² M et utilisés en culture avec 0,1% DMSO en final.

### Résultats des tests

a) Les composés des exemples 1 à 5, 7, 8, 10, 14, 15, 17, 18, 20, 22, 25 à 28, 31 à 37, 41 à 45, 47, 48, 50, 51 et 53 présentent une CI₅₀ inférieure ou égale à 1 µM sur l'activité phosphatase de l'enzyme recombinante Cdc25-C purifiée. Les composés des exemples 6, 11 et 23 présentent quant à eux une CI₅₀ inférieure ou égale à 5 µM sur l'activité phosphatase de l'enzyme recombinante Cdc25-C purifiée
b) Les composés des exemples 1 à 8, 10,11,14,15,17, 18, 20, 22, 25 à 28, 31 à 37, 41 à 45,48, 50, 51 et 53 présentent une CI₅₀ inférieure ou égale à 10 µM sur la prolifération cellulaire des lignées. Mia-Paca2.
c) Les composés des exemples 1 à 8, 10, 11, 14, 15, 17, 18, 20, 22, 25, 26, 31 à 37, 41 à 45, 48, 50 et 53 présentent une CI₅₀ inférieure ou égale à 10 µM sur la prolifération cellulaire des lignées DU-145.

## Revendications

1. Composé répondant à la formule générale **(I)** 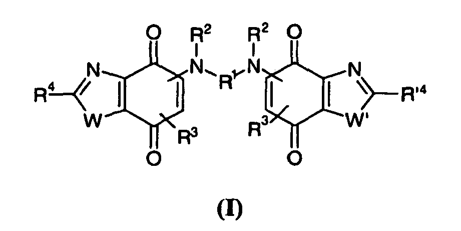 sous forme racémique, d'énantiomère ou toute combinaison de ces formes, dans laquelle :
chacun de W et W' représente indépendamment O ou S ;
R¹ représente l'un des radicaux -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ- et -(CH₂)ₛ-CO-NR⁸-(CH₂)ₜ- dans lesquels m et n sont chacun indépendamment un entier de 2 à 6 (de préférence un entier de 2 à 4 et plus préférentiellement un entier de 2 à 3), p et t sont chacun indépendamment un entier de 2 à 12 (de préférence un entier de 2 à 8 et plus préférentiellement un entier de 2 à 6), q est un entier de 2 à 4 (de préférence un entier de 2 à 3), r est un entier de 0 à 4 (de préférence un entier de 0 à 2), s est un entier de 1 à 12 (de préférence un entier de 1 à 8 et plus préférentiellement un entier de 1 à 6), X est choisi parmi les radicaux NR⁵-, -S, -CO-, -CR⁶R⁷-, cycloalkyle et aryle, étant entendu que lorsque X représente -S-, -CO-, - CR⁶R⁷-, cycloalkyle ou aryle, m et n sont égaux,
R⁵ représentant un atome d'hydrogène ou un radical alkyle ou arylalkyl éventuellement substitué de 1 à 3 fois par des substituants choisis indépendamment parmi un atome halogène et un radical alkyle ou alkoxy, R⁶ et R⁷ représentant chacun indépendamment un atome d'hydrogène ou un radical alkyle et R⁸ représentant un atome d'hydrogène ou un radical alkyle ou arylalkyl ,
ou R¹ représente, 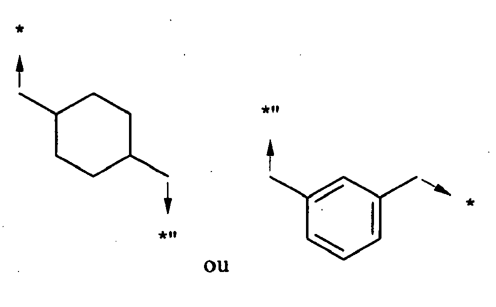 étant entendu que 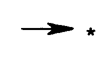 signifie le point d'attachement à la formule générale (I) ; ou encore R¹ représente le radical -(CH₂)_{w}-N(Y)-(CH₂)_{w}- dans lequel Y est le radical 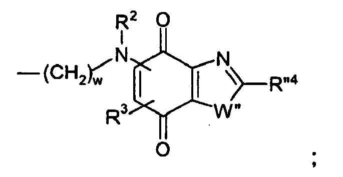
W" représente O ou S et w est un entier de 2 à 3 ;
R² représente un atome d'hydrogène ou un radical alkyle ou arylalkyl ;
R³ représente un atome d'hydrogène ou un atome halogène ;
chacun de R⁴, R'⁴ et R"⁴ représente indépendamment un atome d'hydrogène, un radical alkyle, un radical alkoxyalkyle, un radical aryloxyalkyle, un radical phényle possédant deux substituants qui forment ensemble un radical méthylènedioxy ou éthylène dioxy, un radical -CH₂-NR⁹R¹⁰, un radical -CO-NR¹⁴R¹⁵, ou encore un radical aryle carbocyclique ou arylalkyl carbocyclique éventuellement substitué de 1 à 4 fois par des substituants choisis indépendamment parmi un atome halogène et un radical alkyle, haloalkyle, alkoxy, haloalkoxy ou aryle,
ou R⁴ représente le radical 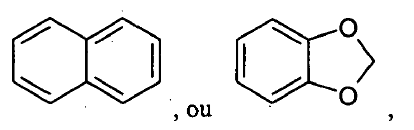
R⁹ représentant indépendamment à chaque fois qu'il intervient un radical alkyle et R¹⁰ représentant indépendamment à chaque fois qu'il intervient un atome d'hydrogène ou un radical alkyle, ou encore R⁹ et R¹⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹¹R¹²-, -O-, -S-, et -NR¹³-, R¹¹ et R¹² représentant indépendamment à chaque fois qu'ils interviennent un atome d'hydrogène ou un radical alkyle et R¹³ représentant un radical alkyle ou arylalkyl , ou encore R¹³ représentant un radical phényle éventuellement substitué de 1 à 3 fois par des substituants choisis indépendamment parmi un atome halogène et un radical alkyle ou alkoxy,
R¹⁴ représentant indépendamment à chaque fois qu'il intervient un radical alkyle, un radical haloalkyle, un radical cycloalkyle, un radical cycloalkylalkyle, un radical alkoxyalkyle, l'un des radicaux aryle carbocyclique ou hétérocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂,
ou R¹⁴ représente un radical 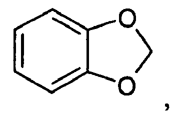
ou encore R¹⁴ représentant l'un des radicaux -(CH₂)ₐ-[O-(CH₂)_{b}]_{c}-O-Alk, -(CH₂)_{d}-[O-(CH₂)ₑ]_{f}-NR¹⁶R¹⁷ ou -(CH₂)_{g}-A dans lesquels a, b et e sont chacun indépendamment un entier de 2 à 4, c est un entier de 1 à 4, f est un entier de 0 à 4, d est un entier de 2 à 12 (et de préférence un entier de 2 à 8 et notamment un entier de 2 à 6) et g est un entier de 1 à 12 (et de préférence un entier de 1 à 8 et notamment un entier de 1 à 6), Alk est un radical alkyle, R¹⁶ est un atome d'hydrogène ou un radical alkyle, alkoxycarbonyle ou aralkoxycarbonyle, R¹⁷ est un atome d'hydrogène ou un radical alkyle et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical alkyle et R¹⁹ représentant un atome d'hydrogène, un radical alkyle ou un groupement alkoxycarbonyle ou aralkoxycarbonyle,
et R¹⁵ représentant indépendamment à chaque fois qu'il intervient un atome d'hydrogène ou un radical alkyle ou arylalkyl , R¹⁵ pouvant également représenter un radical identique à R¹⁴ lorsque R¹⁴ représente un radical alkyle, haloalkyle, alkoxyalkyle
ou arylalkyl carbocyclique ou hétérocyclique dont le noyau aryle est éventuellement substitué de 1 à 3 fois par des substituants choisis indépendamment parmi le groupe constitué par un atome halogène, un radical alkyle, un radical alkoxy, un radical haloalkyle et un radical -SO₂-NH₂
ou encore R¹⁴ et R¹⁵ formant ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et -NR²²-, R²⁰ et R²¹ représentant un atome d'hydrogène ou un radical alkyle ou arylalkyl et R²² représentant -COR²³ ou -SO₂R²⁴,
R²³ représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore R²³ représentant un radical aryle hétérocyclique ou un hétérocycle saturé comptant de 5 à 7 chaînons et de 1 à 2 hétéroatomes choisis indépendamment parmi O, N et S (et notamment l'un des radicaux pipéridino, pipérazino, morpholino, thiomorpholino ou 2-tétrahydrofuryle),
R²⁴ représentant un atome d'hydrogène ou un radical alkyle (et de préférence un radical alkyle),
ou enfin R¹⁴ et R¹⁵ formant ensemble avec l'atome d'azote qui les porte un radical aryle hétérocyclique choisi parmi les radicaux 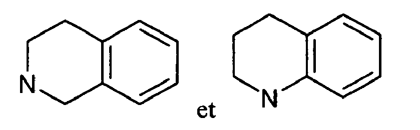 dont le noyau aromatique peut être substitué de 1 à 3 fois par des substituants choisis indépendamment parmi le groupe constitué par un radical alkyle et un radical alkoxy
étant entendu que dans le cas où R¹ représente le radical -(CH₂)_{w}-N(Y)-(CH₂)_{w}-, W, W' et W" sont identiques (autrement dit représentent soit tous O soit tous S), R⁴, R'⁴ et R"⁴ sont identiques et les atomes d'azote adjacents aux noyaux 1,3-benzothiazole-4,7-dione ou 1,3-benzoxazole-4,7-dione sont soit chacun attaché à la position 5 du noyau 1,3-benzothiazole-4,7-dione ou 1,3-benzoxazole-4,7-dione correspondant soit chacun attaché à la position 6 du noyau 1,3-benzothiazole-4,7-dione ou 1,3-benzoxazole-4,7-dione correspondant ;
ou sel d'un tel composé.

2. Composé selon la revendication 1, **caractérisé en ce que** R¹ ne représente pas un radical -(CH₂)_{w}-N(Y)-(CH₂)_{w}-, W et W' sont identiques et R⁴ et R'⁴ sont identiques ;
ou sel d'un tel composé.

3. Composé selon la revendication 1, **caractérisé en ce que** R¹ ne représente pas un radical -(CH₂)_{w}-N(Y)-(CH₂)_{w}- et l'un de W et W' représente O tandis que l'autre représente S et/ou R⁴ et R'⁴ sont différents.

4. Composé selon la revendication 1, **caractérisé en ce qu'**il possède au moins l'une des caractéristiques suivantes :
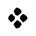 R¹ représentant l'un des radicaux -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ-, -(CH₂)ₛ-CO-NR⁸-(CH₂)ₜ- ou bien R¹ représentant le radical -(CH₂)_{w}-N(Y)-(CH₂)_{w}- ;
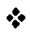 R² représentant un atome d'hydrogène ou le radical méthyle, éthyle ou benzyle ;
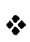 R³ représentant un atome d'hydrogène ou un atome halogène ;
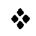 chacun de R⁴, R'⁴ et R"⁴ représentant indépendamment un atome d'hydrogène, un radical alkyle, -CO-NR¹⁴R¹⁵ ou encore un radical aryle carbocyclique ou arylalkyl carbocyclique éventuellement substitué de 1 à 4 fois par des substituants choisis indépendamment parmi un atome halogène et un radical alkyle, haloalkyle, alkoxy, haloalkoxy ou aryle ;
ou sel d'un tel composé.

5. Composé selon l'une des revendications 1 à 4, **caractérisé en ce qu'**il possède au moins l'une des caractéristiques suivantes :
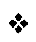 R¹ représentant -(CH₂)ₘ-X-(CH₂)ₙ- ;
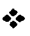 R² représentant un atome d'hydrogène;
ou sel d'un tel composé.

6. Composé selon l'une des revendications 1 à 4, **caractérisé en ce que** R¹ représente l'un des radicaux -CH₂-CR⁶R⁷-CH2-, -(CH₂)ₘ-X-(CH₂)ₙ-, -(CH₂)ₚ-[O-(CH₂)_{q}]ᵣO-(CH₂)ₚ- et -(CH₂)ₛCO-NR⁸-(CH₂)ₜ ;
ou sel d'un tel composé.

7. Composé selon l'une des revendications 1 à 6, **caractérisé en ce que** R¹ représente -(CH₂)ₘX-(CH₂)ₙ- ;
ou sel d'un tel composé.

8. Composé selon l'une des revendications 1 à 7, **caractérisé en ce qu'**il possède au moins l'une des caractéristiques suivantes :
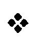 X représente -NR⁵- dans lequel R⁵ représente un radical méthyle ;
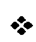 R¹⁴ représente indépendamment à chaque fois qu'il intervient un radical alkyle, un radical cycloalkyle, un radical cycloalkylalkyle, un radical alkoxyalkyle, l'un des radicaux aryle carbocyclique ou arylalkyl carbocyclique ou hétérocyclique dont le noyau aryle est éventuellement substitué de 1 à 3 fois par des substituants choisis indépendamment parmi le groupe constitué par un atome halogène, un radical alkyle et un radical alkoxy,
ou encore R¹⁴ représente l'un des radicaux -(CH₂)_{d}-[O-(CH₂)ₑ]_{f}-NR¹⁶R¹⁷ ou -(CH₂)_{g}-A dans lesquels e est 2 ou 3, f est un entier de 0 à 3, d est un entier de 2 à 6 et g est un entier de 1 à 6, R¹⁶ est un atome d'hydrogène ou un radical alkoxycarbonyle (et notamment *tert*-butoxycarbonyle), R¹⁷ est un atome d'hydrogène et A est un hétérocycle saturé comptant de 1 à 2 hétéroatomes choisis indépendamment parmi O, N et S et attaché au groupe -(CH₂)_{g}- par un chaînon N ou CH, ledit hétérocycle saturé comptant par ailleurs de 2 à 6 chaînons supplémentaires choisis indépendamment parmi -CHR¹⁸-, -CO-, -NR¹⁹-, -O- et -S-, R¹⁸ représentant un atome d'hydrogène ou un radical méthyle et R¹⁹ représentant un atome d'hydrogène, un radical alkyle ou un groupement alkoxycarbonyle (et notamment *tert*-butoxycarbonyle),
ou encore R¹⁴ représente un radical 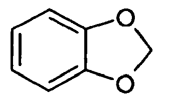
et R¹⁵ représente indépendamment à chaque fois qu'il intervient un atome d'hydrogène,
ou encore R¹⁴ et R¹⁵ forment ensemble avec l'atome d'azote qui les porte un hétérocycle saturé de 4 à 7 chaînons comportant de 1 à 2 hétéroatomes en tout, les chaînons nécessaires pour compléter l'hétérocycle étant choisis indépendamment parmi les radicaux -CR²⁰R²¹-, -O-, -S- et -NR²²-, R²⁰ et R²¹ représentant un atome d'hydrogène ou un radical méthyle et R²² représentant -COR²³ ou -SO₂R²⁴,
R²³ représentant un radical alkyle, un radical aryle carbocyclique éventuellement substitué de 1 à 3 fois par des substituants choisis indépendamment parmi le groupe constitué par un atome halogène, un radical alkyle et un radical alkoxy, ou encore
R²³ représentant un radical aryle hétérocyclique ou un hétérocycle saturé choisi parmi les radicaux pipéridino, pipérazino, morpholino et 2-tétrahydrofuryle,
R²⁴ représentant un atome d'hydrogène ou un radical alkyle (et en particulier un radical alkyle, notamment méthyle) ;
ou encore R¹⁴ et R¹⁵ formant ensemble avec l'atome d'azote qui les porte un radical aryle hétérocyclique du type 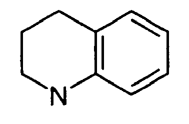 dont le noyau aromatique peut être substitué un radical methoxy;
ou sel d'un tel composé.

9. Composé selon la revendication 1, **caractérisé en ce qu'**il est choisi parmi les composés suivants :
- 5,5'-[(méthylimino)bis(propane-3,1-diylimino)]*bis*(2-méthyl-1,3-benzothiazole-4,7-dione) ;
- 5,5'-[(méthylimino)*bis*(éthane-2,1-diylimino)]bis(2-méthyl-1,3-benzothiazole-4,7-dione) ;
- 5,5'-[oxybis(éthane-2,1-diylimino)]*bis*(2-méthyl-1,3-benzothiazole-4,7-dione) ;
- 5,5'-(pentane-1,5-diyldiimino)*bis*(2-méthyl-1,3-benzothiazole-4,7-dione) ;
- 6,6'-[(méthylimino)bis(propane-3,1-diylimino)]bis(2-méthyl-1,3-benzothiazole-4,7-dione) ;
- 6,6'-[(méthylimino)bis(éthane-2,1-diylimino)]bis(2-méthyl-1,3-benzothiazole-4,7-dione) ;
- 5,5'-[(méthylimino)bis(propane-3,1-diylimino)]bis{4,7-dioxo-*N*-[3-(2-oxopyrrolidin-1-yl)propyl]-4,7-dihydro-1,3-benzothiazole-2-carboxamide} ;
- 6,6'-[(méthylimino)bis(propane-3,1-diylimino)]bis[2-(2,5-difluorophényl)-1,3-benzoxazole-4,7-dione] ;
- 6,6'-[(méthylimino)bis(propane-3,1-diylimino)]bis[2-(3,5-dibromophényl)-1,3-benzoxazole-4,7-dione] ;
- 6,6'-[(méthylimino)bis(propane-3,1-diylimino)]bis[2-(2-chloro-6-fluorobenzyl)-1,3-benzoxazole-4,7-dione] ;
- 6,6'-[(méthylimino)bis(propane-3,1-diylimino)]bis[2-(3-bromophényl)-1,3-benzoxazole-4,7-dione] ;
- 6,6'-[(méthylimino)bis(propane-3,1-diylimino)]bis[2-(4-bromophényl)-1,3-benzoxazole-4,7-dione] ;
- 6,6'-[(méthylimino)bis(propane-3,1-diylimino)]bis[2-(3,5-difluorophényl)-1,3-benzoxazole-4,7-dione] ;
- 6,6'-[(méthylimino)bis(propane-3,1-diylimino)]bis[2-(3-chlorophényl)-1,3-benzoxazole-4,7-dione] ;
- 6,6'-[(méthylimino)bis(propane-3,1-diylimino)]bis[2-(4-fluorophényl)-1,3-benzoxazole-4,7-dione] ;
- 6,6'-[(méthylimino)bis(propane-3,1-diylimino)]bis[2-(4-bromo-3-méthylphényl)-1,3-benzoxazole-4,7-dione] ;
- 5,5'-[(méthylimino)bis(propane-3,1-diylimino)]bis(6-bromo-2-méthyl-1,3-benzothiazole-4,7-dione) ;
- 5,5',5"-[nitrilotris(propane-3,1-diylimino)]tris(2-méthyl-1,3-benzothiazole-4,7-dione) ;
- 5,5'-(2,2-diméthylpropane-1,3-diyldiimino)bis(2-méthyl-1,3-benzothiazole-4,7-dione) ;
- 5,5'-[cyclohexane-1,4-diylbis(méthyleneimino)]bis(2-méthyl-1,3-benzothiazole-4,7-dione) ;
- 5,5'-[1,3-phénylènebis(méthylèneimino)]bis(2-méthyl-1,3-benzothiazole-4,7-dione) ;
- 5,5'-[éthane-1,2-diylbis(oxypropane-3,1-diylimino)]bis(2-méthyl-1,3-benzothiazole-4,7-dione) ;
- 6,6'-{(méthylimino)bis[propane-3,1-diyl(méthylimino)]}bis[2-(2,5-difluorophényl)-1,3-benzoxazole-4,7-dione] ;
- *N*³-[2-(2,5-difluorophényl)-4,7-dioxo-4,7-dihydro-1,3-benzoxazol-6-yl]-*N*¹-(3-{[2-(2,5-difluorophényl)-4,7-dioxo-4,7-dihydro-1,3-benzoxazol-6-yl]amino}propyl)-β-alaninamide ;
- 5,5'-[(méthylimino)bis(propane-3,1-diylimino)]bis(1,3-benzothiazole-4,7-dione) ;
- 5,5'-[(méthylimino)bis(propane-3,1-diylimino)]bis[2-(morpholin-4-ylcarbonyl)-1,3-benzothiazole-4,7-dione] ;
- 5,5'-[(méthylimino)bis(propane-3,1-diylimino)]bis(2-{[4-(morpholin-4-ylcarbonyl)pipérazin-1-yl]carbonyl}-1,3-benzothiazole-4,7-dione) ;
- 5,5'-[(méthylimino)bis(propane-3,1-diylimino)]bis(2-{[4-(tétrahydrofuran-2-ylcarbonyl)pipérazin-1-yl]carbonyl}-1,3-benzothiazole-4,7-dione) ;
- 5,5'-[(méthylimino)bis(propane-3,1-diylimino)]bis(2-{[4-(méthylsulfonyl)pipérazin-1-yl]carbonyl}-1,3-benzothiazole-4,7-dione) ;
- 5,5'-[(méthylimino)bis(propane-3,1-diylimino)]bis[*N*-(4-méthoxyphenyl)-4,7-dioxo-4,7-dihydro-1,3-benzothiazole-2-carboxamide] ;
- 6,6'-[(méthylimino)bis(propane-3,1-diylimino)]bis[2-(pyrrolidin-1-ylcarbonyl)-1 ,3-benzothiazole-4,7-dione] ;
- 5,5'-[(méthylimino)bis(propane-3,1-diylimino)]bis[*N*-(4-fluorophényl)-4,7-dioxo-4,7-dihydro-1,3-benzothiazole-2-carboxamide] ;
- 5,5'-[(méthylimino)bis(propane-3,1-diylimino)]bis[*N*-(4-méthoxybenzyl)-4,7-dioxo-4,7-dihydro-1,3-benzothiazole-2-carboxamide] ;
- 5,5'-[(méthylimino)bis(propane-3,1-diylimino)]bis{2-[(6-méthoxy-3,4-dihydroquinolin-1(2*H*)-yl)carbonyl]-1,3-benzothiazole-4,7-dione} ;
- 5,5'-[(méthylimino)bis(propane-3,1-diylimino)]bis(*N-*cyclohexyl-4,7-dioxo-4,7-dihydro-1,3-benzothiazole-2-carboxamide) ;
- 5,5'-[(méthylimino)bis(propane-3,1-diylimino)]bis(2-{[4-(4-méthoxybenzoyl)pipérazin-1-yl]carbonyl}-1,3-benzothiazole-4,7-dione) ;
- 5,5'-[(méthylimino)bis(propane-3,1-diylimino)]bis(2-{[4-(2-furoyl)pipérazin-1-yl]carbonyl}-1,3-benzothiazole-4,7-dione) ;
- 6,6'-[(méthylimino)bis(propane-3,1-diylimino)]bis[2-(2-naphthyl)-1,3-benzothiazole-4,7-dione] ;
- 6,6'-[(méthylimino)bis(propane-3,1-diylimino)]bis[2-(1,3-benzodioxol-5-yl)-1,3-benzothiazole-4,7-dione] ;
- 6,6'-[(méthylimino)bis(propane-3,1-diylimino)]bis[2-(4-éthylphényl)-1,3-benzoxazole-4,7-dione] ;
- *N*-(4-méthoxyphényl)-5- {[3-(méthyl {3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- 5-{[3-(méthyl{3-[(2-méthyl-4,7-dioxo-4,7-dihydro-1,3-benzothiazol-6-yl)amino]propyl}amino)propyl]amino}-2-{[4-(tétrahydrofuran-2-ylcarbonyl)pipérazin-1-yl]carbonyl}-1,3-benzothiazole-4,7-dione ;
- *N*-éthyl-5-{[3-(méthyl{3-[(2-méthyl-4,7-dioxo-4,7-dihydro-1,3-benzothiazol-5-yl)amino]propyl} amino)propyl]amino}-4,7-dioxo-4,7-dihydro-1,3-benzothiazole-2-carboxamide ;
- 5-({3-[(3-{[4,7-dioxo-2-(pyrrolidin-1-ylcarbonyl)-4,7-dihydro-1,3-benzothiazol-6-yl]amino}propyl)(méthyl)amino]propyl}amino)-2-méthyl-1,3-benzothiazole-4,7-dione ;
- *N*-(4-méthoxybenzyl)-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- *N*-1,3-benzodioxol-5-yl-5-{[3-(méthyl{3-[(2-méthyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide ;
- 2-[(6-méthoxy-3,4-dihydroquinolin-1(2*H*)-yl)carbonyl]-5-{[3-(méthyl{3-[(2-méthyl-4,7-dioxo-4,7-dihydro-1,3-benzothiazol-5-yl)amino]propyl}amino)propyl]amino}-1,3-benzothiazole-4,7-dione ;
- 2-{[4-(4-méthoxybenzoyl)pipérazin-1-yl]carbonyl}-5-{[3-(méthyl{3-[(2-méthyl-4,7-dioxo-4,7-dihydro-1,3-benzothiazol-5-yl)amino]propyl} amino)propyl]amino}-1,3-benzothiazole-4,7-dione ;
- 5-({3-[(3-{[2-(4-méthoxyphenyl)-4,7-dioxo-4,7-dihydro-1,3-benzothiazol-6-yl]amino}propyl)(méthyl)amino]propyl}amino)-2-méthyl-1,3-benzothiazole-4,7-dione ;
- 5-({3-[{3-[(4,7-dioxo-4,7-dihydro-1,3-benzothiazol-5-yl)amino]propyl}(méthyl)amino]propyl}amino)-2-méthyl-1,3-benzothiazole-4,7-dione ;
- 2-(2,5-difluorophényl)-6-{[3-(méthyl {3-[(2-méthyl-4,7-dioxo-4,7-dihydro-1,3-benzothiazol-5-yl)amino]propyl}amino)propyl]amino}-1,3-benzoxazole-4,7-dione ;
- 2-(4-éthylphényl)-6- {[3-(méthyl {3-[(2-méthyl-4,7-dioxo-4,7-dihydro-1,3-benzothiazol-5-yl)amino]propyl}amino)propyl]amino}-1,3-benzoxazole-4,7-dione ;
- 2-(2,5-difluorophényl)-6-{[3-(méthyl{3-[(2-méthyl-4,7-dioxo-4,7-dihydro-1,3-benzothiazol-6-yl)amino]propyl}amino)propyl]amino}-1,3-benzoxazole-4,7-dione ;
- 5,5'-[[(4-méthoxybenzyl)imino]bis(propane-3,1-diylimino)]bis(2-méthyl-1,3-benzothiazole-4,7-dione) ;
- 5,5'-[(méthylimino)bis(butane-4,1-diylimino)]bis(2-méthyl-1,3-benzothiazole-4,7-dione) ;
- 2-méthyl-5-{[3-(méthyl{4-[(2-méthyl-4,7-dioxo-4,7-dihydro-1,3-benzothiazol-5-yl)amino]butyl}amino)propyl]amino}-1,3-benzothiazole-4,7-dione ;
ou sel d'un tel composé.

10. Composé répondant à la formule générale (I), **caractérisé en ce qu'**il s'agit du 2-(2,5-difluorophényl)-6-{[3-(méthyl {3-[(2-méthyl-4,7-dioxo-4,7-dihydro-1,3-benzothiazol-5-yl)amino]propyl}amino)propyl]amino}-1,3-benzoxazole-4,7-dione ou d'un sel de ce composé.

11. A titre de médicament, un composé de formule générale **(I)** selon l'une des revendications 1 à 10 ou un sel pharmaceutiquement acceptable d'un tel composé.

12. Composition pharmaceutique comprenant, à titre de principe actif, un composé de formule générale **(I)** selon l'une des revendications 1 à 10 ou un sel pharmaceutiquement acceptable d'un tel composé, avec au moins un excipient pharmaceutiquement acceptable.

13. Utilisation d'un composé de formule générale **(I)** selon l'une des revendications 1 à 10, ou d'un sel pharmaceutiquement acceptable d'un tel composé, pour préparer un médicament destiné à traiter une maladie / un désordre choisi parmi les maladies suivantes / les désordres suivants : les maladies prolifératives tumorales, les maladies prolifératives non tumorales, les maladies neurodégénératives, les maladies parasitaires, les infections virales, l'alopécie spontanée, l'alopécie induite par des produits exogènes, l'alopécie radio-induite, les maladies auto-immunes, les rejets de greffes, les maladies inflammatoires et les allergies.

14. Utilisation selon la revendication 13, **caractérisée en ce que** le médicament préparé est destiné à traiter le cancer.

15. Utilisation selon la revendication 14, **caractérisée en ce que** le cancer destiné à être traité est choisi parmi le cancer du sein, les lymphomes, les cancers du cou et de la tête, le cancer du poumon, le cancer du colon, le cancer de la prostate et le cancer du pancréas.

## Claims

1. Compound corresponding to the general formula **(I)** 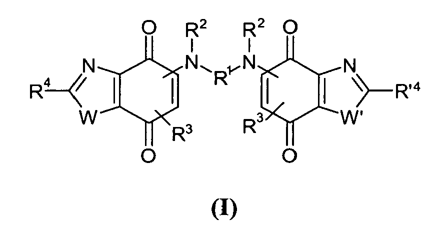 in racemic, enantiomeric form or any combination of these forms, in which:
each of W and W' represents independently O or S;
R¹ represents one of the -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ- and -(CH₂)ₛ-CO-NR⁸-(CH₂)ₜ- radicals in which m and n are each independently an integer from 2 to 6 (preferably an integer from 2 to 4 and more preferentially an integer from 2 to 3), p and t are each independently an integer from 2 to 12 (preferably an integer from 2 to 8 and more preferentially an integer from 2 to 6), q is an integer from 2 to 4 (preferably an integer from 2 to 3), r is an integer from 0 to 4 (preferably an integer from 0 to 2), s is an integer from 1 to 12 (preferably an integer from 1 to 8 and more preferentially an integer from 1 to 6), X is chosen from the-NR⁵-, -S, -CO-, -CR⁶R⁷-, cycloalkyl and aryl radicals, it being understood that when X represents -S-, -CO-, -CR⁶R⁷-, cycloalkyl or aryl, m and n are equal,
R⁵ representing a hydrogen atom or an alkyl or arylalkyl radical optionally substituted 1 to 3 times by substituents chosen independently from a halogen atom and an alkyl or alkoxy radical, R⁶ and R⁷ each independently representing a hydrogen atom or an alkyl radical and R⁸ representing a hydrogen atom or an alkyl or arylalkyl radical,
or R¹ represents 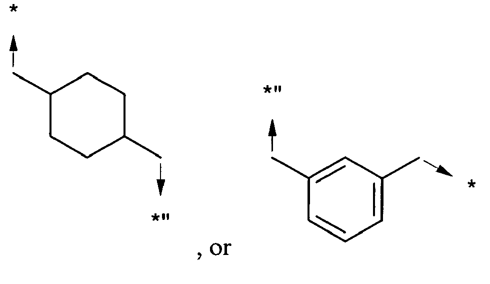 it being understood that 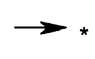 signifies the attachment point to the general formula (I);
or also R¹ represents the -(CH₂)_{w}-N(Y)-(CH₂)_{w}- radical in which Y is the radical 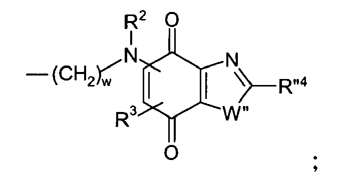
W" represents O or S and w is an integer from 2 to 3;
R² represents a hydrogen atom or an alkyl or arylalkyl radical;
R³ represents a hydrogen atom or a halogen atom;
each of R⁴, R'⁴ and R"⁴ represents independently a hydrogen atom, an alkyl radical, an alkoxylalkyl radical, an aryloxyalkyl radical, a phenyl radical possessing two substituents which form together a methylenedioxy or ethylene dioxy radical, a -CH₂-NR⁹R¹⁰ radical, a-CO-NR¹⁴R¹⁵ radical, or also a carbocyclic aryl or carbocyclic arylalkyl radical optionally substituted 1 to 4 times by substituents chosen independently from a halogen atom and an alkyl, haloalkyl, alkoxy, haloalkoxy or aryl, radical
or R⁴ represents the radical 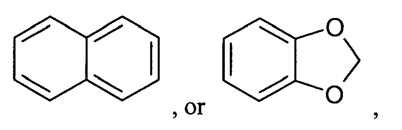
R⁹ representing independently each time that it occurs an alkyl radical and R¹⁰ representing independently each time that it occurs a hydrogen atom or an alkyl radical, or also R⁹ and R¹⁰ forming together with the nitrogen atom a heterocycle with 4 to 7 members comprising 1 to 2 heteroatoms, the members necessary for completing the heterocycle being chosen independently from the -CR¹¹R¹², -O-, -S-, and -NR¹³-radicals, R¹¹ and R¹² representing independently each time that they occur a hydrogen atom or an alkyl radical and R¹³ representing an alkyl or arylalkyl radical, or also R¹³ representing a phenyl radical optionally substituted 1 to 3 times by substituents chosen independently from a halogen atom and an alkyl or alkoxy radical,
R¹⁴ representing independently each time that it occurs an alkyl radical, a haloalkyl radical, a cycloalkyl radical, a cycloalkylalkyl radical, an alkoxylalkyl radical, one of the carbocyclic or heterocyclic aryl or carbocyclic or heterocyclic arylalkyl radicals the aryl ring of which is optionally substituted 1 to 3 times by substituents chosen independently from the group constituted by a halogen atom, an alkyl radical, an alkyl radical, an alkoxy radical, a haloalkyl radical and an -SO₂-NH₂ radical,
or R¹⁴ represents a radical 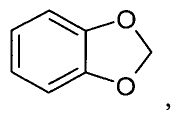
or also R¹⁴ representing one of the -(CH₂)ₐ-[O-(CH₂)_{b}]_{c}-O-Alk, -(CH₂)_{d}-[O-(CH₂)ₑ]_{f}-NR¹⁶R¹⁷ or -(CH₂)_{g}-A radicals in which a, b and e are each independently an integer from 2 to 4, c is an integer from 1 to 4, f is an integer from 0 to 4, d is an integer from 2 to 12 (and preferably an integer from 2 to 8 and in particular an integer from 2 to 6) and g is an integer from 1 to 12 (and preferably an integer from 1 to 8 and in particular an integer from 1 to 6), Alk is an alkyl radical, R¹⁶ is a hydrogen atom or an alkyl, alkoxycarbonyl or aralkoxycarbonyl radical, R¹⁷ is a hydrogen atom or an alkyl radical and A is a saturated heterocycle containing 1 to 2 heteroatoms chosen independently from O, N and S and attached to the group -(CH₂)_{g}- by an N or CH member, said saturated heterocycle containing moreover from 2 to 6 additional members chosen independently from -CHR¹⁸-, -CO-, -NR¹⁹-, -O- and -S-, R¹⁸ representing a hydrogen atom or an alkyl radical and R¹⁹ representing a hydrogen atom, an alkyl radical or an alkoxycarbonyl or aralkoxycarbonyl group,
and R¹⁵ representing independently each time that it occurs a hydrogen atom or an alkyl or arylalkyl radical, R¹⁵ also being able to represent a radical identical to R¹⁴ when R¹⁴ represents a carbocyclic or heterocyclic alkyl, haloalkyl, alkoxyalkyl or arylalkyl radical the aryl ring of which is optionally substituted 1 to 3 times by substituents chosen independently from the group constituted by a halogen atom, an alkyl radical, an alkoxy radical, a haloalkyl radical and an -SO₂-NH₂ radical
or also R¹⁴ and R¹⁵ forming together with the nitrogen atom which carries them a saturated heterocycle with 4 to 7 members comprising 1 to 2 heteroatoms in total, the members necessary for completing the heterocycle being chosen independently from the -CR²⁰R²¹-, -O-, -S- and -NR²²- radicals, R²⁰ and R²¹ representing a hydrogen atom or an alkyl or arylalkyl radical and R²² representing -COR²³ or -SO₂R²⁴,
R²³ representing an alkyl radical, a carbocyclic aryl radical optionally substituted 1 to 3 times by substituents chosen independently from the group constituted by a halogen atom, an alkyl radical and an alkoxy radical, or also R²³ representing a heterocyclic aryl radical or a saturated heterocycle containing 5 to 7 members and 1 to 2 heteroatoms chosen independently from O, N and S (and in particular one of the piperidino, piperazino, morpholino, thiomorpholino or 2-tetrahydrofuryl radicals),
R²⁴ representing a hydrogen atom or an alkyl radical (and preferably an alkyl radical), or finally R¹⁴ and R¹⁵ forming together with the nitrogen atom which carries them a heterocyclic aryl radical chosen from the radicals 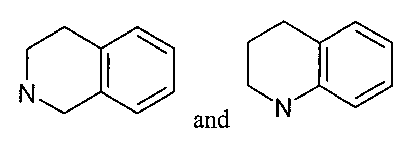
the aromatic ring of which can be substituted 1 to 3 times by substituents chosen independently from the group constituted by an alkyl radical and an alkoxy radical; it being understood that in the case where R¹ represents the-(CH₂)_{w}-N(Y)-(CH₂)_{w}-radical, W, W' and W" are identical (in other words represent either all O or all S), R⁴, R'⁴ and R"⁴ are identical and the nitrogen atoms adjacent to the 1,3-benzothiazole-4,7-dione or 1,3-benzoxazole-4,7-dione rings are either each attached in position 5 of the corresponding 1,3-benzothiazole-4,7-dione or 1,3-benzoxazole-4,7-dione ring or are each attached in position 6 of the corresponding 1,3-benzothiazole-4,7-dione or 1,3-benzoxazole-4,7-dione ring;
or salt of such a compound.

2. Compound according to claim 1, **characterized in that** R¹ does not represent a -(CH₂)_{w}-N(Y)-(CH₂)_{w}- radical, W and W' are identical and R⁴ and R'⁴ are identical;
or salt of such a compound.

3. Compound according to claim 1, **characterized in that** R¹ does not represent a -(CH₂)_{w}-N(Y)-(CH₂)_{w}- radical and one of W and W' represents O whilst the other represents S and/or R⁴ and R'⁴ are different.

4. Compound according to claim 1, **characterized in that** it has at least one of the following characteristics:
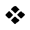 R¹ representing one of the -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ-, -(CH₂)ₛ-CO-NR⁸-(CH₂)ₜ- radicals or R¹ representing the -(CH₂)_{w}-N(Y)-(CH₂)_{w}- radical;
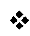 R² representing a hydrogen atom or the methyl, ethyl or benzyl radical;
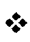 R³ representing a hydrogen atom or a halogen atom;
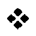 each of R⁴, R'⁴ and R"⁴ representing independently a hydrogen atom, an alkyl, -CO-NR¹⁴R¹⁵ radical or also a carbocyclic aryl or carbocyclic arylalkyl radical optionally substituted 1 to 4 times by substituents chosen independently from a halogen atom and an alkyl, haloalkyl, alkoxy, haloalkoxy or aryl radical
or salt of such a compound.

5. Compound according to one of claims 1 to 4, **characterized in that** it has at least one of the following characteristics :
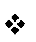 R¹ representing -(CH₂)ₘ-X-(CH₂)ₙ-;
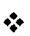 R² representing a hydrogen atom;
or salt of such a compound.

6. Compound according to one of claims 1 to 4, **characterized in that** R¹ represents one of the -CH₂-CR⁶R⁷-CH2-, -(CH₂)ₘ-X-(CH₂)ₙ-, -(CH₂)ₚ-[O-(CH₂)_{q}]ᵣ-O-(CH₂)ₚ- and -(CH₂)ₛ-CO-NR⁸-(CH₂)ₜ- radicals; or salt of such a compound.

7. Compound according to one of claims 1 to 6, **characterized in that** R¹ represents -(CH₂)ₘ-X-(CH₂)ₙ-;
or salt of such a compound.

8. Compound according to one of claims 1 to 7, **characterized in that** it has at least one of the following characteristics :
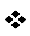 X represents -NR⁵- in which R⁵ represents a methyl radical;
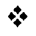 R¹⁴ represents independently each time that it occurs an alkyl radical, a cycloalkyl radical, a cycloalkylalkyl radical, an alkoxylalkyl radical, one of the carbocyclic aryl or carbocyclic or heterocyclic arylalkyl radicals the aryl ring of which is optionally substituted 1 to 3 times by substituents chosen independently from the group constituted by a halogen atom, an alkyl radical and an alkoxy radical,
or also R¹⁴ represents one of the -(CH₂)_{d}-[O-(CH₂)ₑ]_{f}-NR¹⁶R¹⁷ or -(CH₂)_{g}-A radicals in which e is 2 or 3, f is an integer from 0 to 3, d is an integer from 2 to 6 and g is an integer from 1 to 6, R¹⁶ is a hydrogen atom or an alkoxycarbonyl radical (and in particular *tert*-butoxycarbonyl), R¹⁷ is a hydrogen atom and A is a saturated heterocycle containing 1 to 2 heteroatoms chosen independently from O, N and S and attached to the group -(CH₂)_{g}- by an N or CH member, said saturated heterocycle containing moreover from 2 to 6 additional members chosen independently from -CHR¹⁸-, -CO-, -NR¹⁹-, -O- and -S-, R¹⁸ representing a hydrogen atom or a methyl radical and R¹⁹ representing a hydrogen atom, an alkyl radical or an alkoxycarbonyl group (and in particular *tert*-butoxycarbonyl),
or also R¹⁴ represents a radical 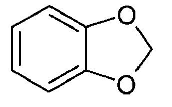
and R¹⁵ represents independently each time that it occurs a hydrogen atom,
or also R¹⁴ and R¹⁵ form together with the nitrogen atom which carries them a saturated heterocycle with 4 to 7 members comprising 1 to 2 heteroatoms in total, the members necessary for completing the heterocycle being chosen independently from the-CR²⁰R²¹-, -O-, -S- and -NR²²- radicals, R²⁰ and R²¹ representing a hydrogen atom or a methyl radical and R²² representing -COR²³ or -SO₂R²⁴,
R²³ representing an alkyl radical, a carbocyclic aryl radical optionally substituted 1 to 3 times by substituents chosen independently from the group constituted by a halogen atom, an alkyl radical and an alkoxy radical, or also R²³ representing a heterocyclic aryl radical or a saturated heterocycle chosen from the piperidino, piperazino, morpholino and 2-tetrahydrofuryl radicals,
R²⁴ representing a hydrogen atom or an alkyl radical (and in particular an alkyl radical, in particular methyl);
or also R¹⁴ and R¹⁵ forming together with the nitrogen atom which carries them a heterocyclic aryl radical of the type 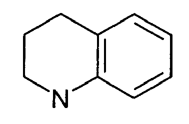 the aromatic ring of which can be substituted by a methoxy radical;
or salt of such a compound.

9. Compound according to claim 1, **characterized in that** it is chosen from the following compounds:
- 5.5'-[(methylimino)bis(propane-3,1-diylimino)]bis(2-methyl-1,3-benzothiazole-4,7-dione);
- 5.5'-[(methylimino)*bis*(ethane-2,1-diylimino)]bis(2-methyl-1,3-benzothiazole-4,7-dione);
- 5.5'-[oxybis(ethane-2,1-diylimino)]*bis*(2-methyl-1,3-benzothiazole-4,7-dione);
- 5.5'-(pentane-1,5-diyldiimino)*bis*(2-methyl-1,3-benzothiazole-4,7-dione);
- 6.6'-[(methylimino)bis(propane-3,1-diylimino)]bis(2-methyl-1,3-benzothiazole-4,7-dione);
- 6.6'-[(methylimino)bis(ethane-2,1-diylimino)]bis(2-methyl-1,3-benzothiazole-4,7-dione);
- 5.5'-[(methylimino)bis(propane-3,1-diylimino)]bis{4,7-dioxo-*N*-[3-(2-oxopyrrolidin-1-yl)propyl]-4,7-dihydro-1,3-benzothiazole-2-carboxamide};
- 6.6'-[(methylimino)bis(propane-3,1-diylimino)]bis[2-(2,5-difluorophenyl)-1,3-benzoxazole-4,7-dione];
- 6.6'-[(methylimino)bis(propane-3,1-diylimino)]bis[2-(3,5-dibromophenyl)-1,3-benzoxazole-4,7-dione];
- 6.6'-[(methylimino)bis(propane-3,1-diylimino)]bis[2-(2-chloro-6-fluorobenzyl)-1,3-benzoxazole-4,7-dione];
- 6.6'-[(methylimino)bis(propane-3,1-diylimino)]bis[2-(3-bromophenyl)-1,3-benzoxazole-4,7-dione];
- 6.6'-[(methylimino)bis(propane-3,1-diylimino)]bis[2-(4-bromophenyl)-1,3-benzoxazole-4,7-dione];
- 6.6'-[(methylimino)bis(propane-3,1-diylimino)]bis[2-(3,5-difluorophenyl)-1,3-benzoxazole-4,7-dione];
- 6.6'-[(methylimino)bis(propane-3,1-diylimino)]bis[2-(3-chlorophenyl)-1,3-benzoxazole-4,7-dione];
- 6.6'-[(methylimino)bis(propane-3,1-diylimino)]bis[2-(4-fluorophenyl)-1,3-benzoxazole-4,7-dione];
- 6.6'-[(methylimino)bis(propane-3,1-diylimino)]bis[2-(4-bromo-3-methylphenyl)-1,3-benzoxazole-4,7-dione];
- 5.5'-[(methylimino)bis(propane-3,1-diylimino)]bis(6-bromo-2-methyl-1,3-benzothiazole-4,7-dione);
- 5.5',5"-[nitrilotris(propane-3,1-diylimino)]tris(2-methyl-1,3-benzothiazole-4,7-dione);
- 5.5'-(2,2-dimethylpropane-1,3-diyldiimino)bis(2-methyl-1,3-benzothiazole-4,7-dione);
- 5.5'-[cyclohexane-1,4-diylbis(methyleneimino)]bis(2-methyl-1,3-benzothiazole-4,7-dione);
- 5.5'-[1,3-phenylenebis(methyleneimino)]bis(2-methyl-1,3-benzothiazole-4,7-dione);
- 5.5'-[ethane-1,2-diylbis(oxypropane-3,1-diylimino)]bis(2-methyl-1,3-benzothiazole-4,7-dione);
- 6.6'-{(methylimino)bis[propane-3,1-diyl(methylimino)]}bis[2-(2,5-difluorophenyl)-1,3-benzoxazole-4,7-dione];
- *N*³-[2-(2,5-difluorophenyl)-4,7-dioxo-4,7-dihydro-1,3-benzoxazol-6-yl]-*N*¹-(3-{[2-(2,5-difluorophenyl)-4,7-dioxo-4,7-dihydro-1,3-benzoxazol-6-yl]amino}propyl)-β-alaninamide;
- 5.5'-[(methylimino)bis(propane-3,1-diylimino)]bis(1,3-benzothiazole-4,7-dione);
- 5.5'-[(methylimino)bis(propane-3,1-diylimino)]bis[2-(morpholin-4-ylcarbonyl)-1,3-benzothiazole-4,7-dione];
- 5.5'-[(methylimino)bis(propane-3,1-diylimino)]bis(2-{[4-(morpholin-4-ylcarbonyl)piperazin-1-yl]carbonyl}-1,3-benzothiazole-4,7-dione);
- 5.5'-[(methylimino)bis(propane-3,1-diylimino)]bis(2-{[4-(tetrahydrofuran-2-ylcarbonyl)piperazin-1-yl]carbonyl}-1,3-benzothiazole-4,7-dione);
- 5.5'-[(methylimino)bis(propane-3,1-diylimino)]bis(2-{[4-(methylsulphonyl)piperazin-1-yl]carbonyl}-1,3-benzothiazole-4,7-dione);
- 5.5'-[(methylimino)bis(propane-3,1-diylimino)]bis[*N*-(4-methoxyphenyl)-4,7-dioxo-4,7-dihydro-1,3-benzothiazole-2-carboxamide];
- 6.6'-[(methylimino)bis(propane-3,1-diylimino)]bis[2-(pyrrolidin-1-ylcarbonyl)-1,3-benzothiazole-4,7-dione];
- 5.5'-[(methylimino)bis(propane-3,1-diylimino)]bis[N-(4-fluorophenyl)-4,7-dioxo-4,7-dihydro-1,3-benzothiazole-2-carboxamide];
- 5.5'-[(methylimino)bis(propane-3,1-diylimino)]bis[*N*-(4-methoxybenzyl)-4,7-dioxo-4,7-dihydro-1,3-benzothiazole-2-carboxamide];
- 5.5'-[(methylimino)bis(propane-3,1-diylimino)]bis{2-[(6-methoxy-3,4-dihydroquinolin-1(2*H*)-yl)carbonyl]-1,3-benzothiazole-4,7-dione};
- 5.5'-[(methylimino)bis(propane-3,1-diylimino)]bis(*N*-cyclohexyl-4,7-dioxo-4,7-dihydro-1,3-benzothiazole-2-carboxamide);
- 5.5'-[(methylimino)bis(propane-3,1-diylimino)]bis(2-{[4-(4-methoxybenzoyl)piperazin-1-yl]carbonyl}-1,3-benzothiazole-4,7-dione);
- 5.5'-[(methylimino)bis(propane-3,1-diylimino)]bis(2-{[4-(2-furoyl)piperazin-1-yl]carbonyl}-1,3-benzothiazole-4,7-dione);
- 6.6'-[(methylimino)bis(propane-3,1-diylimino)]bis[2-(2-naphthhyl)-1,3-benzothiazole-4,7-dione];
- 6.6'-[(methylimino)bis(propane-3,1-diylimino)]bis[2-(1,3-benzodioxol-5-yl)-1,3-benzothiazole-4,7-dione];
- 6.6'-[(methylimino)bis(propane-3,1-diylimino)]bis[2-(4-ethylphenyl)-1,3-benzoxazole-4,7-dione];
- *N*-(4-methoxyphenyl)-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide;
- 5-{[3-(methyl{3-[(2-methyl-4,7-dioxo-4,7-dihydro-1,3-benzothiazol-6-yl)amino]propyl}amino)propyl]amino}-2-{[4-(tetrahydrofuran-2-ylcarbonyl)piperazin-1-yl]carbonyl}-1,3-benzothiazole-4,7-dione;
- *N*-ethyl-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide;
- 5-({3-[(3-{[4,7-dioxo-2-(pyrrolidin-1-ylcarbonyl)-4,7-dihydro-1,3-benzothiazol-6-yl]amino}propyl)(methyl)amino]propyl}amino)-2-methyl-1,3-benzothiazole-4,7-dione;
- *N*-(4-methoxybenzyl)-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide;
- *N*-1,3-benzodioxol-5-yl-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazole-2-carboxamide;
- 2-[(6-methoxy-3,4-dihydroquinolin-1(2*H*)-yl)carbonyl]-5-{[3-(methyl{3-[(2-methyl-4,7-dioxo-4,7-dihydro-1,3-benzothiazol-5-yl)amino]propyl}amino)propyl]amino}-1,3-benzothiazole-4,7-dione;
- 2-{[4-(4-methoxybenzoyl)piperazin-1-yl]carbonyl}-5-{[3-(methyl{3-[(2-methyl-4,7-dioxo-4,7-dihydro-1,3-benzothiazol-5-yl)amino]propyl}amino)propyl]amino}-1,3-benzothiazole-4,7-dione;
- 5-({3-[(3-{[2-(4-methoxyphenyl)-4,7-dioxo-4,7-dihydro-1,3-benzothiazol-6-yl]amino}propyl)(methyl)amino]propyl}amino)-2-methyl-1,3-benzothiazole-4,7-dione;
- 5-({3-[{3-[(4,7-dioxo-4,7-dihydro-1,3-benzothiazol-5-yl)amino]propyl}(methyl)amino]propyl}amino)-2-methyl-1,3-benzothiazole-4,7-dione;
- 2-(2,5-difluorophenyl)-6-{[3-(methyl{3-[(2-methyl-4,7-dioxo-4,7-dihydro-1,3-benzothiazol-5-yl)amino]propyl}amino)propyl]amino}-1,3-benzoxazole-4,7-dione;
- 2-(4-ethylphenyl)-6-{[3-(methyl{3-[(2-methyl-4,7-dioxo-4,7-dihydro-1,3-benzothiazol-5-yl)amino]propyl}amino)propyl]amino}-1,3-benzoxazole-4,7-dione;
- 2-(2,5-difluorophenyl)-6-{[3-(methyl{3-[(2-methyl-4,7-dioxo-4,7-dihydro-1,3-benzothiazol-6-yl)amino]propyl}amino)propyl]amino}-1,3-benzoxazole-4,7-dione;
- 5.5'-[[(4-methoxybenzyl)imino]bis(propane-3,1-diylimino)]bis(2-methyl-1,3-benzothiazole-4,7-dione);
- 5.5'-[(methylimino)bis(butane-4,1-diylimino)]bis(2-methyl-1,3-benzothiazole-4,7-dione);
- 2-methyl-5-{[3-(methyl{4-[(2-methyl-4,7-dioxo-4,7-dihydro-1,3-benzothiazol-5-yl)amino]butyl}amino)propyl]amino}-1,3-benzothiazole-4,7-dione;
or salt of such a compound.

10. Compound of general formula (I), **characterized in that** it is the 2-(2,5-difluorophenyl)-6- {[3-(methyl {3-[(2-methyl-4,7-dioxo-4,7-dihydro-1,3-benzothiazol-5-yl)amino]propyl}amino)propyl]amino}-1,3-benzoxazole-4,7-dione or a salt this compound.

11. As a medicament, a compound of general formula **(I)** according to one of claims 1 to 10 or a pharmaceutically acceptable salt of such a compound.

12. Pharmaceutical composition comprising, as active ingredient, a compound of general formula **(I)** according to one of claims 1 to 10 or a pharmaceutically acceptable salt of such a compound, with at least one pharmaceutically acceptable excipient.

13. Use of a compound of general formula **(I)** according to one of claims 1 to 10, or of a pharmaceutically acceptable salt of such a compound, for preparing a medicament intended to treat a disease / a disorder chosen from the following diseases / the following disorders: tumorous proliferative diseases, non-tumorous proliferative diseases, neurodegenerative diseases, parasitic diseases, viral infections, spontaneous alopecia, alopecia induced by exogenous products, radiation-induced alopecia, autoimmune diseases, transplant rejections, inflammatory diseases or allergies.

14. Use according to claim 13, **characterized in that** the medicament prepared is intended to treat cancer.

15. Use according to claim 14, **characterized in that** the cancer intended to be treated is chosen from breast cancer, lymphomas, cancers of the neck or head, lung cancer, cancer of the colon, prostate cancer or cancer of the pancreas.

## Patentansprüche

1. Verbindung der allgemeinen Formel (I) 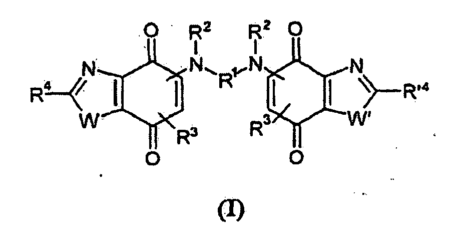 in racemischer Form, Enantiomeren-Form oder jeder Kombination dieser Formen, in der:
jedes von W und W' unabhängig O oder S darstellt;
R¹ einen der Reste -CH₂CR⁶R⁷-CH₂-, -(CH₂)ₘ-X-(CH₂)ₙ-, -(CH₂)ₚ-[O-(CH₂)_{q}]ᵣ-O-(CH₂)ₚ-und -(CH₂)ₛ-CO-NR⁸-(CH₂)ₜ- darstellt, in denen m und n jeweils unabhängig eine ganze Zahl von 2 bis 6 (vorzugsweise eine ganze Zahl von 2 bis 4 und bevorzugter eine ganze Zahl von 2 bis 3) sind, p und t jeweils unabhängig eine ganze Zahl von 2 bis 12 (vorzugsweise eine ganze Zahl von 2 bis 8 und bevorzugter eine ganze Zahl von 2 bis 6) sind, q eine ganze Zahl von 2 bis 4 (vorzugsweise eine ganze Zahl von 2 bis 3) ist, r eine ganze Zahl von 0 bis 4 (vorzugsweise eine ganze Zahl von 0 bis 2) ist, s eine ganze Zahl von 1 bis 12 (vorzugsweise eine ganze Zahl von 1 bis 8 und bevorzugter eine ganze Zahl von 1 bis 6) ist, X ausgewählt ist aus den Resten -NR⁵-, -S-, -CO-, -CR⁶R⁷-, Cycloalkyl und Aryl, vorausgesetzt, dass, wenn X -S-, -CO-, -CR⁶R⁷-, Cycloalkyl oder Aryl darstellt, m und n gleich sind,
R⁵ ein Wasserstoffatom oder einen Alkyl- oder Arylalkylrest, gegebenenfalls 1- bis 3-mal mit Substituenten substituiert, die unabhängig ausgewählt sind aus einem Halogenatom und einem Alkyl- oder Alkoxyrest, darstellt, R⁶ und R⁷ jeweils unabhängig ein Wasserstoffatom oder einen Alkylrest darstellen und R⁸ ein Wasserstoffatom oder einen Alkyl- oder Arylalkylrest darstellt,
oder R¹ 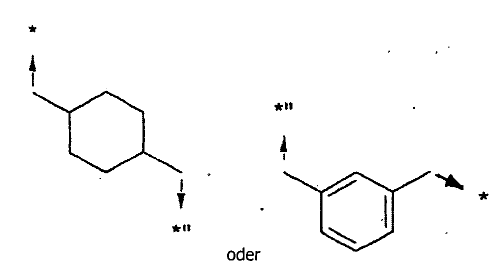 darstellt, vorausgesetzt, dass 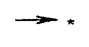 den Verknüpfungspunkt mit der allgemeinen Formel (I) bedeutet;
oder R¹ den Rest -(CH₂)_{w}-N(Y)-(CH₂)_{w}- darstellt, in dem Y der Rest 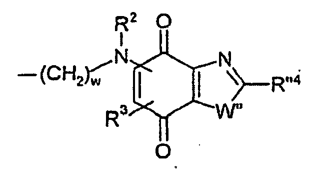 ist;
W" O oder S darstellt und w eine ganze Zahl von 2 bis 3 ist;
R² ein Wasserstoffatom oder einen Alkyl- oder Arylalkylrest darstellt;
R³ ein Wasserstoffatom oder ein Halogenatom darstellt;
jedes von R⁴, R'⁴ und R"⁴ unabhängig ein Wasserstoffatom, einen Alkylrest, einen Alkoxyalkylrest, einen Aryloxyalkylrest, einen Phenylrest, der zwei Substituenten besitzt, die zusammen einen Methylendioxy- oder Ethylendioxyrest bilden, einen Rest -CH₂NR⁹R¹⁰, einen Rest
-CO-NR¹⁴R¹⁵ oder auch einen carbocyclischen Arylrest oder carbocyclischen Arylalkylrest, gegebenenfalls 1- bis 4-mal substituiert mit Substituenten, unabhängig ausgewählt aus einem Halogenatom und einem Alkyl-, Halogenalkyl-, Alkoxy-, Halogenalkoxy- oder Arylrest, darstellt,
oder R⁴ den Rest 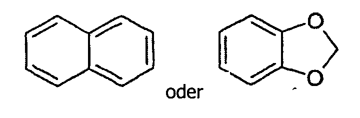 darstellt,
R⁹ unabhängig jedes Mal, wenn es vorkommt, einen Alkylrest darstellt und R¹⁰ unabhängig jedes Mal, wenn es vorkommt, ein Wasserstoffatom oder einen Alkylrest darstellt, oder R⁹ und R¹⁰ zusammen mit dem Stickstoffatom einen Heterocyclus mit 4 bis 7 Kettengliedern, der 1 bis 2 Heteroatome umfasst, bilden, wobei die Kettenglieder, die notwendig sind, um den Heterocyclus zu vervollständigen, unabhängig ausgewählt sind aus den Resten -CR¹¹R¹²-, -O-, -S- und -NR¹³-, R¹¹ und R¹² unabhängig jedes Mal, wenn sie vorkommen, ein Wasserstoffatom oder einen Alkylrest darstellen und R¹³ einen Alkyl- oder Arylalkylrest darstellt, oder R¹³ einen Phenylrest darstellt, der gegebenenfalls 1- bis 3-mal mit Substituenten substituiert ist, die unabhängig ausgewählt sind aus einem Halogenatom und einem Alkyl- oder Alkoxyrest,
R¹⁴ unabhängig jedes Mal, wenn es vorkommt, einen Alkylrest, einen Halogenalkylrest, einen Cycloalkylrest, einen Cycloalkylalkylrest, einen Alkoxyalkylrest, einen der Reste carbocyclisches oder heterocyclisches Aryl oder carbocyclisches oder heterocyclisches Arylalkyl, bei denen der Arylkern gegebenenfalls 1- bis 3-mal mit Substituenten substituiert ist, die unabhängig ausgewählt sind aus der Gruppe, bestehend aus einem Halogenatom, einem Alkylrest, einem Alkoxyrest, einem Halogenalkylrest und einem Rest -SO₂-NH₂, darstellt,
oder R¹⁴ einen Rest 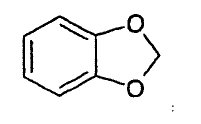 darstellt,
oder R¹⁴ einen der Reste - (CH₂)ₐ- [O- (CH₂)_{b}]_{c}-O-Alk, - (CH₂)_{d}- [O-(CH₂)ₑ]_{f}-NR¹⁶R¹⁷ oder -(CH₂)_{g} -A darstellt, in denen a, b und e jeweils unabhängig eine ganze Zahl von 2 bis 4 sind, c eine ganze Zahl von 1 bis 4 ist, f eine ganze Zahl von 0 bis 4 ist, d eine ganze Zahl von 2 bis 12 (und vorzugsweise eine ganze Zahl von 2 bis 8 und insbesondere eine ganze Zahl von 2 bis 6) ist und g eine ganze Zahl von 1 bis 12 (und vorzugsweise eine ganze Zahl von 1 bis 8 und insbesondere eine ganze Zahl von 1 bis 6) ist, Alk ein Alkylrest ist, R¹⁶ ein Wasserstoffatom oder ein Alkyl-, Alkoxycarbonyl- oder Aralkoxycarbonylrest ist, R¹⁷ ein Wasserstoffatom oder ein Alkylrest ist und A ein gesättigter Heterocyclus ist, der 1 bis 2 Heteroatome zählt, die unabhängig aus O, N und S ausgewählt sind, und der durch ein Kettenglied N oder CH an die Gruppe -(CH₂)_{g}- gebunden ist, wobei der gesättigte Heterocyclus außerdem 2 bis 6 zusätzliche Kettenglieder zählt, unabhängig ausgewählt aus -CHR¹⁸-, -CO-, -NR¹⁹-, -O- und -S-, R¹⁸ ein Wasserstoffatom oder einen Alkylrest darstellt und R¹⁹ ein Wasserstoffatom, einen Alkylrest oder eine Alkoxycarbonyl- oder Aralkoxycarbonylgruppe darstellt,
und R¹⁵ unabhängig jedes Mal, wenn es vorkommt, ein Wasserstoffatom oder einen Alkyl- oder Arylalkylrest darstellt, wobei R¹⁵ auch einen mit R¹⁴ identischen Rest darstellen kann, wenn R¹⁴ einen Alkyl-, Halogenalkyl-, Alkoxyalkylrest oder einen carbocyclischen oder heterocyclischen Arylalkylrest, dessen Arylkern gegebenenfalls 1- bis 3-mal mit Substituenten substituiert ist, die unabhängig ausgewählt sind aus der Gruppe, bestehend aus einem Halogenatom, einem Alkylrest, einem Alkoxyrest, einem Halogenalkylrest und einem Rest -SO₂NH₂, darstellt,
oder R¹⁴ und R¹⁵ zusammen mit dem Stickstoffatom, an das sie gebunden sind, einen gesättigten Heterocyclus mit 4 bis 7 Kettengliedern bilden, der insgesamt 1 bis 2 Heteroatome trägt, wobei die Kettenglieder, die erforderlich sind, um den Heterocyclus zu vervollständigen, unabhängig ausgewählt sind aus den Resten -CR²⁰R²¹-, -O-, -S- und -NR²²- , R²¹ und R²¹ ein Wasserstoffatom oder einen Alkyl- oder Arylalkylrest darstellen, und R²² -COR²³ oder -SO₂R²⁴ darstellt,
R²³ einen Alkylrest, einen carbocyclischen Arylrest, der gegebenenfalls 1- bis 3-mal mit Substituenten substituiert ist, unabhängig ausgewählt aus der Gruppe, bestehend aus einem Halogenatom, einem Alkylrest und einem Alkoxyrest, darstellt, oder R²³ einen heterocyclischen Arylrest oder einen gesättigten Heterocyclus, der 5 bis 7 Kettenglieder und 1 bis 2 Heteroatome, unabhängig ausgewählt aus O, N und S, zählt (und insbesondere einen der Piperidino-, Piperazino-, Morpholino-, Thiomorpholino- und 2-Tetrahydrofuryl-Reste) darstellt,
R²⁴ ein Wasserstoffatom oder einen Alkylrest (und vorzugsweise einen Alkylrest) darstellt, oder
R¹⁴ und R¹⁵ zusammen mit dem Stickstoffatom, an das sie gebunden sind, einen heterocyclischen Arylrest, ausgewählt aus den Resten 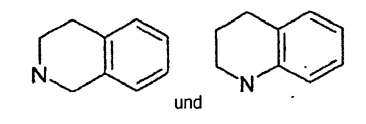 deren aromatischer Kern 1- bis 3-mal mit Substituenten, unabhängig ausgewählt aus der Gruppe, bestehend aus einem Alkylrest und einem Alkoxyrest, substituiert sein kann, bilden;
vorausgesetzt, dass, wenn R¹ den Rest - (CH₂)_{w}-N(Y)-(CH₂)_{w}- darstellt, W, W' und W" identisch sind (anders ausgedrückt, entweder alle O darstellen oder alle S darstellen), R⁴, R'⁴ und R"⁴ identisch sind und die Stickstoffatome, die den Kernen 1,3-Benzothiazol-4,7-dion oder 1,3-Benzoxazol-4,7-dion benachbart sind, jeweils an die Position 5 des entsprechenden Kerns 1,3-Benzothiazo-4,7-dion oder 1,3-Benzoxazol-4,7-dion gebunden sind oder jeweils an die Position 6 des entsprechenden Kerns 1,3-Benzothiazol-4,7-dion oder 1,3-Benzoxazol-4,7-dion gebunden sind;
oder ein Salz einer solchen Verbindung.

2. Verbindung gemäß Anspruch 1, **dadurch gekennzeichnet, dass** R¹ keinen Rest - (CH₂)_{w}-N(Y)-(CH₂)_{w}- darstellt, W und W' identisch sind und R⁴ und R'⁴ identisch sind;
oder ein Salz einer solchen Verbindung.

3. Verbindung gemäß Anspruch 1, **dadurch gekennzeichnet, dass** R¹ keinen Rest -(CH₂)_{w}-N(Y)-(CH₂)_{w}- darstellt, und eines von W und W' O darstellt, während das andere S darstellt und/oder R⁴ und R'⁴ verschieden sind.

4. Verbindung gemäß Anspruch 1, **dadurch gekennzeichnet, dass** sie wenigstens eines der folgenden Charakteristika aufweist:
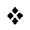 R¹ stellt einen der Reste -CH₂-CR⁶R⁷-CH₂-, -(CH₂)ₘ-X-(CH₂)ₙ-, - (CH₂)ₚ- [O- (CH₂)_{q}]ᵣ-O- (CH₂)ₚ-, -(CH₂)ₛ-CO-NR⁸- (CH₂)ₜ- dar oder R¹ stellt den Rest - -(CH₂)_{w}-N(Y)-(CH₂)_{w}- dar;
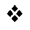 R² stellt ein Wasserstoffatom oder den Rest Methyl, Ethyl oder Benzyl dar;
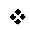 R³ stellt ein Wasserstoffatom oder ein Halogenatom dar;
jedes von R⁴, R'⁴ und R"⁴ stellt unabhängig ein Wasserstoffatom, einen Alkylrest, -CO-NR¹⁴R¹⁵ oder auch einen carbocyclischen Arylrest oder carbocyclischen Arylalkylrest, gegebenenfalls 1- bis 4-mal substituiert mit Substituenten, unabhängig ausgewählt aus einem Halogenatom und einem Alkyl-, Halogenalkyl-, Alkoxy-, Halogenalkoxy- oder Arylrest, dar;
oder ein Salz einer solchen Verbindung.

5. Verbindung gemäß einem der Ansprüche 1 bis 4, **dadurch gekennzeichnet, dass** sie wenigstens eines der folgenden Charakteristika aufweist:
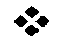 R¹ stellt - -(CH₂)ₘ-X-(CH₂)ₙ- dar;
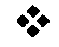 R² stellt ein Wasserstoffatom dar;
oder ein Salz einer solchen Verbindung.

6. Verbindung gemäß einem der Ansprüche 1 bis 4, **dadurch gekennzeichnet, dass** R¹ einen der Reste -CH₂-CR⁶R⁷-CH₂-, -(CH₂)ₘ-X-(CH₂)ₙ-, - (CH₂)ₚ-[O-(CH₂)_{q}]ᵣ-O-(CH₂)ₚ- und - (CH₂)ₛ-CO-NR⁸-(CH₂)ₜ- darstellt;
oder ein Salz einer solchen Verbindung.

7. Verbindung gemäß einem der Ansprüche 1 bis 6, **dadurch gekennzeichnet, dass** R¹-(CH₂)ₘ-X-(CH₂)ₙ- darstellt;
oder ein Salz einer solchen Verbindung.

8. Verbindung gemäß einem der Ansprüche 1 bis 7, **dadurch gekennzeichnet, dass** sie wenigstens eines der folgenden Charakteristika aufweist:
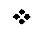 X stellt -NR⁵-, worin R⁵ einen Methylrest darstellt, dar;
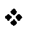 R¹⁴ stellt unabhängig jedes Mal, wenn es vorkommt, einen Alkylrest, einen Cycloalkylrest, einen Cycloalkylalkylrest, einen Alkoxyalkylrest, einen der carbocyclischen Arylreste oder carbocyclischen oder heterocyclischen Arylalkylreste, dessen Arylkern gegebenenfalls 1- bis 3-mal mit Substituenten, unabhängig ausgewählt aus der Gruppe, bestehend aus einem Halogenatom, einem Alkylrest und einem Alkoxyrest, substituiert ist, dar, oder R¹⁴ stellt einen der Reste - (CH₂)_{d}-[O-(CH₂)ₑ]_{f}-NR¹⁶R¹⁷ oder -(CH₂)g-A dar, in denen e 2 oder 3 ist, f eine ganze Zahl von 0 bis 3 ist, d eine ganze Zahl von 2 bis 6 ist und g eine ganze Zahl von 1 bis 6 ist, R¹⁶ ein Wasserstoffatom oder ein Alkoxycarbonylrest (und insbesondere tert-Butoxycarbonyl) ist, R¹⁷ ein Wasserstoffatom ist und A ein gesättigter Heterocyclus ist, der 1 bis 2 Heteroatome, unabhängig ausgewählt aus O, N und S, zählt und der an die Gruppe -(CH₂)_{g}- durch ein Kettenglied N oder CH gebunden ist, wobei der gesättigte Heterocyclus außerdem 2 bis 6 zusätzliche Kettenglieder zählt, unabhängig ausgewählt aus -CHR¹⁸-, -CO-, -NR¹⁹-, -O- und -S-, wobei R¹⁸ ein Wasserstoffatom oder einen Methylrest darstellt und R¹⁹ ein Wasserstoffatom, einen Alkylrest oder eine Alkoxycarbonylgruppe (und insbesondere tert-Butoxycarbonyl) darstellt,
oder R¹⁴ stellt einen Rest 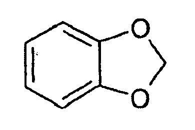 dar,
und R¹⁵ stellt unabhängig jedes Mal, wenn es auftritt, ein Wasserstoffatom dar
oder R¹⁴ und R¹⁵ bilden zusammen mit dem Stickstoffatom, an das sie gebunden sind, einen gesättigten Heterocyclus mit 4 bis 7 Kettengliedern, insgesamt umfassend 1 bis 2 Heteroatome, wobei die Kettenglieder, die notwendig sind, um den Heterocyclus zu vervollständigen, unabhängig ausgewählt sind aus den Resten -CR²⁰R²¹-, -O-, -S- und -NR²²-, wobei R²² und R²¹ ein Wasserstoffatom oder einen Methylrest darstellen und R²² -COR²³ oder -SO₂R²⁴ darstellt,
R²³ einen Alkylrest, einen carbocyclischen Arylrest, der gegebenenfalls 1- bis 3-mal mit Substituenten substituiert ist, unabhängig ausgewählt aus der Gruppe, bestehend aus einem Halogenatom, einem Alkylrest und einem Alkoxyrest, oder R²³ einen heterocyclischen Arylrest oder einen gesättigten Heterocyclus, ausgewählt aus den Resten Piperidino, Piperazino, Morpholino und 2-Tetrahydrofuryl, darstellt,
R²⁴ ein Wasserstoffatom oder einen Alkylrest (und insbesondere einen Alkylrest, insbesondere Methyl) darstellt;
oder R¹⁴ und R¹⁵ bilden zusammen mit dem Stickstoffatom, an das sie gebunden sind, einen heterocyclischen Arylrest des Typs 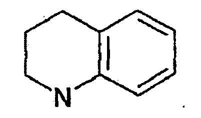 dessen aromatischer Kern mit einem Methoxyrest substituiert sein kann;
oder ein Salz einer solchen Verbindung.

9. Verbindung gemäß Anspruch 1, **dadurch gekennzeichnet, dass** sie unter den folgenden Verbindungen ausgewählt ist:
5,5'-[(Methylimino)bis(propan-3,1-diylimino)]bis(2-methyl-1,3-benzothiazol-4,7-dion);
5,5'-[(Methylimino)bis(ethan-2,1-diylimino)]bis(2-methyl-1,3-benzothiazol-4,7-dion);
5,5'-[Oxybis(ethan-2,1-diylimino)]bis(2-methyl-1,3-benzothiazol-4,7-dion);
5,5'-(Pentan-1,5-diyldiimino)bis(2-methyl-1,3-benzothiazol-4,7-dion);
6,6'-[(Methylimino)bis(propan-3,1-diylimino)]bis(2-methyl-1,3-benzothiazol-4,7-dion);
6,6'-[(Methylimino)bis(ethan-2,1-diylimino)]bis(2-methyl-1,3-benzothiazol-4,7-dion);
5,5'-[(Methylimino)bis(propan-3,1-diylimino)]bis{4,7-dioxo-N-[3-(2-oxopyrrolidin-1-yl)propyl]-4,7-dihydro-1,3-benzothiazol-2-carboxamid};
6,6'-[(Methylimino)bis(propan-3,1-diylimino)]bis[2-(2,5-difluorphenyl)-1,3-benzoxazol-4,7-dion];
6,6'-[(Methylimino)bis(propan-3,1-diylimino)]bis[2-(3,5-dibromphenyl)-1,3-benzoxazol-4,7-dion];
6,6'-[(Methylimino)bis(propan-3,1-diylimino)]bis[2-(2-chlor-6-fluorbenzyl)-1,3-benzoxazol-4,7-dion];
6,6'-[(Methylimino)bis(propan-3,1-diylimino)]bis[2-(3-bromphenyl)-1,3-benzoxazol-4,7-dion];
6,6'-[(Methylimino)bis(propan-3,1-diylimino)]bis[2-(4-bromphenyl)-1,3-benzoxazol-4,7-dion];
6,6'-[(Methylimino)bis(propan-3,l-diylimino)]bis[2-(3,5-difluorphenyl)-1,3-benzoxazol-4,7-dion];
6,6'-[(Methylimino)bis(propan-3,1-diylimino)]bis[2-(3-chlorphenyl)-1,3-benzoxazol-4,7-dion];
6,6'-[(Methylimino)bis(propan-3,1-diylimino)]bis[2-(4-fluorphenyl)-1,3-benzoxazol-4,7-dion];
6,6'-[(Methylimino)bis(propan-3,1-diylimino)]bis[2-(4-brom-3-methylphenyl)-1,3-benzoxazol-4,7-dion];
5,5'-[(Methylimino)bis(propan-3,1-diylimino)]bis(6-brom-2-methyl-1,3-benzothiazol-4,7-dion);
5,5',5"-[Nitrilotris(propan-3,1-diylimino)]tris(2-methyl-1,3-benzothiazol-4,7-dion);
5,5'-(2,2-Dimethylpropan-1,3-diyldiimino)bis(2-methyl-1,3-benzothiazol-4,7-dion);
5,5'-[Cyclohexan-1,4-diylbis(methylenimino)]bis(2-methyl-1,3-benzothiazol-4,7-dion);
5,5'-[1,3-Phenylenbis(methylenimino)]bis(2-methyl-1,3-benzothiazol-4,7-dion);
5,5'-[Ethan-1,2-diylbis(oxypropan-3,1-diylimino)]bis(2-methyl-1,3-benzo-thiazol-4,7-dion);
6,6'-{(Methylimino)bis[propan-3,1-diyl(methylimino)]}bis[2-(2,5-difluorphenyl)-1,3-benzoxazol-4,7-dion];
N³-[2-(2,5-Difluorphenyl)-4,7-dioxo-4,7-dihydro-1,3-benzoxazol-6-yl]-N¹-(3-{[2-(2,5-difluorphenyl)-4,7-dioxo-4,7-dihydro-1,3-benzoxazol-6-yl]amino}-propyl)-β-alaninamid;
5,5'-[(Methylimino)bis(propan-3,1-diylimino)]bis(1,3-benzothiazol-4,7-dion);
5,5'-[(Methylimino)bis(propan-3,1-diylimino)]bis[2-(morpholin-4-ylcarbonyl)-1,3-benzothiazol-4,7-dion];
5,5'-[(Methylimino)bis(propan-3,1-diylimino)]bis(2-{[4-(morpholin-4-ylcarbonyl)piperazin-1-yl]carbonyl}-1,3-benzothiazol-4,7,dion)
5,5'-[(Methylimino)bis(propan-3,1-diylimino)]bis(2-{[4-(tetrahydrofuran-2-ylcarbonyl)piperazin-1-yl]carbonyl}-1,3-benzothiazol-4,7-dion);
5,5'-[(Methylimino)bis(propan-3,1-diylimino)]bis(2-{[4-(methylsulfonyl)-piperazin-1-yllcarbonyl}-1,3-benzothiazol-1,4-dion);
5,5'-[(Methylimino)bis(propan-3,1-diylimino)]bis[N-(4-methoxyphenyl)-4,7-dioxo-4,7-dihydro-1,3-benzothiazol-2-carboxyamid];
6,6'-[(Methylimino)bis(propan-3,1-diylimino)]bis[2-(pyrrolidin-1-ylcarbonyl)-1,3-benzothiazol-4,7-dion];
5,5'-[(Methylimino)bis(propan-3,1-diylimino)]bis[N-(4-fluorphenyl)-4,7-dioxo-4,7-dihydro-1,3-benzothiazol-2-carboxamid];
5,5'-[(Methylimino)bis(propan-3,1-diylimino)]bis[N-(4-methoxybenzyl)-4,7-dioxo-4,7-dihydro-1,3-benzothiazol-2-carboxamid];
5,5'-[(Methylimino)bis(propan-3,1-diylimino)]bis{2-[(6-methoxy-3,4-dihydrochinolin-1(2H)-yl)carbonyl]-1,3-benzothiazo-4,7-dion};
5,5'-[(Methylimino)bis(propan-3,1-diylimino)]bis(N-cyclohexyl-4,7-dioxo-4,7-dihydro-1,3-benzothiazol-2-carboxamid);
5,5'-[(Methylimino)bis(propan-3,1-diylimino)]bis(2-{[4-(4-methoxybenzoyl)piperazin-1-yl]carbonyl}-1,3-benzothiazol-4,7-dion);
5,5'-[(Methylimino)bis(propan-3,1-diylimino)]bis(2-{[4-(2-furoyl)-piperazin-1-yl]carbonyl}-1,3-benzothiazol-4,7-dion)
6,6'-[(Methylimino)bis(propan-3,1-diylimino)]bis[2-(2-naphthyl)-1,3-benzothiazol-4,7-dion];
6,6'-[(Methylimino)bis(propan-3,1-diylimino)]bis[2-(1,3-benzodioxol-5-yl)-1,3-benzothiazol-4,7-dion];
6,6'-[(Methylimino)bis(propan-3,1-diylimino)]bis[2-(4-ethylphenyl)-1,3-benzoxazol-4,7-dion];
N-(4-Methoxyphenyl)-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazol-2-carboxamid;
5-{[3-(Methyl{3-[(2-methyl-4,7-dioxo-4,7-dihydro-1,3-benzothiazol-6-yl)amino]propyl}amino)propyl]amino}-2-{[4-(tetrahydrofuran-2-ylcarbonyl)-piperazin-1-yl]carbonyl}-1,3-benzothiazol-4,7-dion;
N-Ethyl-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazol-2-carboxamid;
5-({3-[(3-{[4,7-Dioxo-2-(pyrrolidin-1-ylcarbonyl)-4,7-dihydro-1,3-benzothiazol-6-yl]amino}propyl)(methyl)amino]propyl}amino)-2-methyl-1,3-benzothiazol-4,7-dion;
N-(4-Methoxybenzyl)-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazol-2-carboxamid;
N-1,3-Benzodioxol-5-yl-5-{[3-(methyl{3-[(2-methyl-4,7-dioxo-4,7-dihydro-1,3-benzothiazol-5-yl)amino]propyl}amino)propyl]amino}-4,7-dioxo-4,7-dihydro-1,3-benzothiazol-2-carboxamid;
2-[(6-Methoxy-3,4-dihydrochinolin-1(2H)-yl)carbonyl]-5-{[3-(methyl{3-[(2-methyl-4,7-dioxo-4,7-dihydro-1,3-benzothiazol-5-yl)-amino]propyl}amino)propyl]amino}-1,3-benzothiazol-4,7-dion;
2-{[4-(4-Methoxybenzoyl)piperazin-1-yl]carbonyl}-5-{[3-(methyl-{3-{(2-methyl-4,7-dioxo-4,7-dihydro-1,3-benzothiazol-5-yl)amino]-propyl}amino)propyl]amino}-1,3-benzothiazol-4,7-dion;
5-({3-[(3-{[2-(4-Methoxyphenyl)-4,7-dioxo-4,7-dihydro-1,3-benzo-thiazol-6-yl]amino}propyl)(methyl)amino]propyl}amino)-2-methyl-1,3-benzothiazol-4,7-dion;
5-({3-[{3-[(4,7-dioxo-4,7-dihydro-1,3-benzothiazol-5-yl)amino]-propyl}(methyl)amino]propyl}amino)-2-methyl-1,3-benzothiazol-4,7-dion;
2-(2,5-Difluorphenyl)-6-{[3-(methyl{3-[(2-methyl-4,7-dioxo-4,7-dihydro-1,3-benzothiazol-5-yl)amino]propyl}amino)propyl]amino}-1,3-benzoxazol-4,7-dion;
2-(4-Ethylphenyl)-6-{[3-(methyl{3-[(2-methyl-4,7-dioxo-4,7-dihydro-1,3-benzothiazol-5-yl)amino]propyl}amino)propyl]amino}-1,3-benzoxazol-4,7-dion;
2-(2,5-Difluorphenyl)-6-{[3-(methyl{3-[(2-methyl-4,7-dioxo-4,7-dihydro-1,3-benzothiazol-6-yl)amino]propyl}amino)propyl]amino}-1,3-benzoxazol-4,7-dion;
5,5'-[[(4-Methoxybenzyl)imino]bis(propan-3,1-diylimino)]bis(2-methyl-1,3-benzothiazol-4,7-dion);
5,5'-[(Methylimino)bis(butan-4,1-diylimino)]bis(2-methyl-1,3-benzothiazol-4,7-dion);
2-Methyl-5-{[3-(methyl{4-[(2-methyl-4,7-dioxo-4,7-dihydro-1,3-benzothiazol-5-yl)amino]butyl}amino)propyl]amino}-1,3-benzothiazol-4,7-dion;
oder ein Salz einer solchen Verbindung.

10. Verbindung der allgemeinen Formel (I), **dadurch gekennzeichnet, dass** sie 2-(2,5-Difluorphenyl)-6-{[3-(methyl{3-[(2-methyl-4,7-dioxo-4,7-dihydro-1,3-benzothiazol-5-yl)amino]propyl}amino)propyl]amino}-1,3-benzoxazol-4,7-dion oder ein Salz einer solchen Verbindung, ist.

11. Verbindung der allgemeinen Formel (I) gemäß einem der Ansprüche 1 bis 10 oder ein pharmazeutisch verträgliches Salz einer solchen Verbindung als Medikament.

12. Pharmazeutische Zusammensetzung, umfassend als Wirkstoff eine Verbindung der allgemeinen Formel (I) gemäß einem der Ansprüche 1 bis 10 oder ein pharmazeutisch verträgliches Salz einer solchen Verbindung mit wenigstens einem pharmazeutisch verträglichen Hilfsstoff.

13. Verwendung einer Verbindung der allgemeinen Formel (I) gemäß einem der Ansprüche 1 bis 10 oder eines pharmazeutisch verträglichen Salzes einer solchen Verbindung zur Herstellung eines Medikaments, das dazu bestimmt ist, eine Erkrankung/Störung, ausgewählt aus den folgenden Erkrankungen/den folgenden Störungen, zu behandeln: proliferative tumoröse Erkrankungen, proliferative nicht-tumoröse Erkrankungen, neurodegenerative Erkrankungen, parasitäre Erkrankungen, Virusinfektionen, spontane Alopezie, durch exogene Produkte induzierte Alopezie, strahleninduzierte Alopezie, Autoimmunerkrankungen, Transplantatabstoßungen, Entzündungserkrankungen und Allergien.

14. Verwendung gemäß Anspruch 13 , **dadurch gekennzeichnet, dass** das hergestellte Medikament dazu bestimmt ist, Krebs zu behandeln.

15. Verwendung gemäß Anspruch 14 , **dadurch gekennzeichnet, dass** der zu behandelnde Krebs ausgewählt ist aus Brustkrebs, Lymphomen, Krebsarten des Halses und des Kopfes, Lungenkrebs, Colonkrebs, Prostatakrebs und Pankreaskrebs.
